# EUROPEAN PATENT SPECIFICATION

(11) **EP 0 575 555 B1**
(45) Date of publication and mention of the grant of the patent: **18.07.2001**
(21) Application number: 92910260.6
(22) Date of filing: 11.03.1992
(51) Int. Cl.: A61K 38/17, A61F 2/02, C07K 14/00

(54) **PROTEIN-INDUCED MORPHOGENESIS**
PROTEIN-INDUZIERENDE MORPHOGENESE
MORPHOGENESE INDUITE PAR DES PROTEINES

(30) Priority: 11.03.1991 US 667274
(43) Date of publication of application: 29.12.1993
(73) Proprietor: Curis, Inc., Cambridge, MA 02138 (US)
(72) Inventor: COHEN, Charles, M., Medway, MA 02053 (US); KUBERASAMPATH, Thangavel, Medway, MA 02053 (US); PANG, Roy, H., L., Etna, New Hampshire 03750 (US); OPPERMANN, Hermann, Medway, MA 02053 (US); RUEGER, David, C., Hopkinton, MA 01748 (US)
(74) Representative: Price, Vincent Andrew
(86) International application number: US9201968
(87) International publication number: WO9215323

(56) References cited:
- WO-A-89/09787
- WO-A-89/09788
- WO-A-90/03733
- WO-A-91/05802
- WO-A-91/18558

### SEQUENCE LISTING

(1) GENERAL INFORMATION:
   (i)APPLICANT: COHEN, CHARLES M.
      KUBERASAMPATH, THANGAVEL
      PANG, ROY H.L.
      OPPERMANN, HERMANN
      RUEGER, DAVID C.
   (ii) TITLE OF INVENTION: PROTEIN-INDUCED MORPHOGENESIS
   (iii) NUMBER OF SEQUENCES: 23
   (iv) CORRESPONDENCE ADDRESS:
      (A) ADDRESSEE: TESTA, HURWITZ & THIBEAULT
      (B) STREET: 53 STATE STREET
      (C) CITY: BOSTON
      (D) STATE: MASSACHUSETTS
      (E) COUNTRY: U.S.A.
      (F) ZIP: 02109
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vii) PRIOR APPLICATION DATA:
      (A) APPLICATION NUMBER: US 667,274
      (B) FILING DATE: 11-MAR-1991
   (vii) PRIOR APPLICATION DATA:
      (A) APPLICATION NUMBER: US 752,764
      (B) FILING DATE: 30-AUG-1991
(2) INFORMATION FOR SEQ ID NO:1:
   (i) SEQUENCE CHARACTERISTICS:
      (A) LENGTH: 97 amino acids
      (B) TYPE: amino acids
      (C) TOPOLOGY: linear
   (ii) MOLECULE TYPE: protein
   (ix) FEATURE:
      (A) NAME: Generic Sequence 1
      (D) OTHER INFORMATION: Each Xaa indicates one of the 20 naturally-occurring L-isomer, α-amino acids or a derivative thereof.
   (xi) SEQUENCE DESCRIPTION: SEQ ID NO:1:
   Xaa Xaa Xaa Xaa Xaa Xaa
    1               5
   Xaa Xaa Xaa Xaa Xaa Xaa Xaa Xaa Xaa Xaa Xaa
                10                  15
   Xaa Xaa Xaa Xaa Xaa Xaa Xaa Cys Xaa Xaa Xaa
            20                  25
   Cys Xaa Xaa Xaa Xaa Xaa Xaa Xaa Xaa Xaa Xaa
        30                  35
   Xaa Xaa Xaa Xaa Xaa Xaa Xaa Xaa Xaa Xaa Xaa
   40                   45                  50
   Xaa Xaa Xaa Xaa Xaa Xaa Xaa Xaa Xaa Xaa Cys
                    55                  60
   Cys Xaa Xaa Xaa Xaa Xaa Xaa Xaa Xaa Xaa Xaa
                65                  70
   Xaa Xaa Xaa Xaa Xaa Xaa Xaa Xaa Xaa Xaa Xaa
            75                  80
   Xaa Xaa Xaa Xaa Xaa Xaa Xaa Xaa Xaa Xaa Cys
        85                  90
   Xaa Cys Xaa
    95
(2) INFORMATION FOR SEQ ID NO:2:
   (i) SEQUENCE CHARACTERISTICS:
      (A) LENGTH: 97 amino acids
      (B) TYPE: amino acids
      (C) TOPOLOGY: linear
   (ii) MOLECULE TYPE: protein
   (ix) FEATURE:
      (A) NAME: Generic Sequence 2
      (D) OTHER INFORMATION: Each Xaa indicates one of the 20 naturally-occurring L-isomer, α-amino acids or a derivative thereof.
   (xi) SEQUENCE DESCRIPTION: SEQ ID NO:2:
   Xaa Xaa Xaa Xaa Xaa Xaa
    1                   5
   Xaa Xaa Xaa Xaa Xaa Xaa Xaa Xaa Xaa Xaa Xaa
                   10                      15
   Xaa Xaa Xaa Xaa Xaa Xaa Xaa Cys Xaa Xaa Xaa
               20                      25
   Cys Xaa Xaa Xaa Xaa Xaa Xaa Cys Xaa Xaa Xaa
           30                      35
   Xaa Xaa Xaa Xaa Xaa Xaa Xaa Xaa Xaa Xaa Xaa
   40                       45                 50
   Xaa Xaa Xaa Xaa Xaa Xaa Xaa Xaa Xaa Xaa Cys
                       55                      60
   Cys Xaa Xaa Xaa Xaa Xaa Xaa Xaa Xaa Xaa Xaa
                   65                      70
   Xaa Xaa Xaa Xaa Xaa Xaa Xaa Xaa Xaa Xaa Xaa
               75                      80
   Xaa Xaa Xaa Xaa Xaa Xaa Xaa Xaa Xaa Xaa Cys
           85                      90
   Xaa Cys Xaa
    95
(2) INFORMATION FOR SEQ ID NO:3:
   (i) SEQUENCE CHARACTERISTICS:
      (A) LENGTH: 97 amino acids
      (B) TYPE: amino acids
      (C) TOPOLOGY: linear
   (ii) MOLECULE TYPE: protein
   (ix) FEATURE:
      (A) NAME: Generic Sequence 3
      (D) OTHER INFORMATION: wherein each Xaa is independently selected from a group of one or more specified amino acids as defined in the specification.
   (xi) SEQUENCE DESCRIPTION: SEQ ID NO:3:
   Leu Tyr Val Xaa Phe
   1               5
   Xaa Xaa Xaa Gly Trp Xaa Xaa Trp Xaa
                       10
   Xaa Ala Pro Gly Xaa Xaa Xaa Ala
    15                  20
   Xaa Tyr Cys Xaa Gly Xaa Cys Xaa
           25                  30
   Xaa Pro Xaa Xaa Xaa Xaa Xaa
                       35
   Xaa Xaa Xaa Asn His Ala Xaa Xaa
           40                  45
   Xaa Xaa Leu Xaa Xaa Xaa Xaa Xaa
                       50
   Xaa Xaa Xaa Xaa Xaa Xaa Xaa Cys
       55                  60
   Cys Xaa Pro Xaa Xaa Xaa Xaa Xaa
                   65
   Xaa Xaa Xaa Leu Xaa Xaa Xaa
    70                  75
   Xaa Xaa Xaa Xaa Val Xaa Leu Xaa
                   80
   Xaa Xaa Xaa Xaa Met Xaa Val Xaa
    85                  90
   Xaa Cys Gly Cys Xaa
           95
(2) INFORMATION FOR SEQ ID NO:4:
   (i) SEQUENCE CHARACTERISTICS:
      (A) LENGTH: 102 amino acids
      (B) TYPE: amino acids
      (C) TOPOLOGY: linear
   (ii) MOLECULE TYPE: protein
   (ix) FEATURE:
      (A) NAME: Generic Sequence 4
      (D) OTHER INFORMATION: wherein each Xaa is independently selected from a group of one or more specified amino acids as defined in the specification.

      (xi) SEQUENCE DESCRIPTION: SEQ ID NO:4:
   Cys Xaa Xaa Xaa Xaa Leu Tyr Val Xaa Phe
    1              5                   10
   Xaa Xaa Xaa Gly Trp Xaa Xaa Trp Xaa
                  15
   Xaa Ala Pro Xaa Gly Xaa Xaa Ala
    20                 25
   Xaa Tyr Cys Xaa Gly Xaa Cys Xaa
           30                  35
   Xaa Pro Xaa Xaa Xaa Xaa Xaa
                   40
   Asn Xaa Xaa Asn His Ala Xaa Xaa
           45                  50
   Xaa Xaa Leu Xaa Xaa Xaa Xaa Xaa
                   55
   Xaa Xaa Xaa Xaa Xaa Xaa Xaa Cys
       60                  65
   Cys Xaa Pro Xaa Xaa Xaa Xaa Xaa
               70
   Xaa Xaa Xaa Leu Xaa Xaa Xaa
    75                 80
   Xaa Xaa Xaa Xaa Val Xaa Leu Xaa
               85
   Xaa Xaa Xaa Xaa Met Xaa Val Xaa
    90                 95
   Xaa Cys Gly Cys Xaa
           100
(2) INFORMATION FOR SEQ ID NO:5:
   (i) SEQUENCE CHARACTERISTICS:
      (A) LENGTH: 139 amino acids
      (B) TYPE: amino acids
      (C) TOPOLOGY: linear
   (ii) MOLECULE TYPE: protein
   (ix) FEATURE:
      (A) NAME: hOP-1 (mature form)
   (xi) SEQUENCE DESCRIPTION: SEQ ID NO:5:
   Ser Thr Gly Ser Lys Gln Arg Ser Gln
   1 5
   Asn Arg Ser Lys Thr Pro Lys Asn Gln
   10 15
   Glu Ala Leu Arg Met Ala Asn Val Ala
   20 25
   Glu Asn Ser Ser Ser Asp Gln Arg Gln
   30 35
   Ala Cys Lys Lys His Glu Leu Tyr Val
   40 45
   Ser Phe Arg Asp Leu Gly Trp Gln Asp
   50
   Trp Ile Ile Ala Pro Glu Gly Tyr Ala
   55 60
   Ala Tyr Tyr Cys Glu Gly Glu Cys Ala
   65 70
   Phe Pro Leu Asn Ser Tyr Met Asn Ala
   75 80
   Thr Asn His Ala Ile Val Gln Thr Leu
   85 90
   Val His Phe Ile Asn Pro Glu Thr Val
   95
   Pro Lys Pro Cys Cys Ala Pro Thr Gln
   100 105
   Leu Asn Ala Ile Ser Val Leu Tyr Phe
   110 115
   Asp Asp Ser Ser Asn Val Ile Leu Lys
   120 125
   Lys Tyr Arg Asn Met Val Val Arg Ala
   130 135
   Cys Gly Cys His
(2) INFORMATION FOR SEQ ID NO:6:
   (i) SEQUENCE CHARACTERISTICS:
      (A) LENGTH: 139 amino acids
      (B) TYPE: amino acids
      (C) TOPOLOGY: linear
   (ii) MOLECULE TYPE: protein
   (ix) FEATURE:
      (A) NAME: mOP-1 (mature form)
   (xi) SEQUENCE DESCRIPTION: SEQ ID NO:6:
   Ser Thr Gly Gly Lys Gln Arg Ser Gln
     1                 5
   Asn Arg Ser Lys Thr Pro Lys Asn Gln
    10                  15
   Glu Ala Leu Arg Met Ala Ser Val Ala
        20                  25
   Glu Asn Ser Ser Ser Asp Gln Arg Gln
           30                  35
   Ala Cys Lys Lys His Glu Leu Tyr Val
               40                  45
   Ser Phe Arg Asp Leu Gly Trp Gln Asp
                   50
   Trp Ile Ile Ala Pro Glu Gly Tyr Ala
   55                  60
   Ala Tyr Tyr Cys Glu Gly Glu Cys Ala
       65                  70
   Phe Pro Leu Asn Ser Tyr Met Asn Ala
           75                  80
   Thr Asn His Ala Ile Val Gln Thr Leu
               85                  90
   Val His Phe Ile Asn Pro Asp Thr Val
                   95
   Pro Lys Pro Cys Cys Ala Pro Thr Gln
   100                 105
   Leu Asn Ala Ile Ser Val Leu Tyr Phe
       110                 115
   Asp Asp Ser Ser Asn Val Ile Leu Lys
           120                 125
   Lys Tyr Arg Asn Met Val Val Arg Ala
               130                 135
   Cys Gly Cys His
(2) INFORMATION FOR SEQ ID NO:7:
   (i) SEQUENCE CHARACTERISTICS:
      (A) LENGTH: 139 amino acids
      (B) TYPE: amino acids
      (C) TOPOLOGY: linear
   (ii) MOLECULE TYPE: protein
   (ix) FEATURE:
      (A) NAME: hOP-2 (mature form)
   (xi) SEQUENCE DESCRIPTION: SEQ ID NO:7:
   Ala Val Arg Pro Leu Arg Arg Arg Gln
   1 5
   Pro Lys Lys Ser Asn Glu Leu Pro Gln
   10 15
   Ala Asn Arg Leu Pro Gly Ile Phe Asp
   20 25
   Asp Val His Gly Ser His Gly Arg Gln
   30 35
   Val Cys Arg Arg His Glu Leu Tyr Val
   40 45
   Ser Phe Gln Asp Leu Gly Trp Leu Asp
   50
   Trp Val Ile Ala Pro Gln Gly Tyr Ser
   55 60
   Ala Tyr Tyr Cys Glu Gly Glu Cys Ser
   65 70
   Phe Pro Leu Asp Ser Cys Met Asn Ala
   75 80
   Thr Asn His Ala Ile Leu Gln Ser Leu
   85 90
   Val His Leu Met Lys Pro Asn Ala Val
   95
   Pro Lys Ala Cys Cys Ala Pro Thr Lys
   100 105
   Leu Ser Ala Thr Ser Val Leu Tyr Tyr
   110 115
   Asp Ser Ser Asn Asn Val Ile Leu Arg
   120 125
   Lys His Arg Asn Met Val Val Lys Ala
   130 135
   Cys Gly Cys His
(2) INFORMATION FOR SEQ ID NO:8:
   (i) SEQUENCE CHARACTERISTICS:
      (A) LENGTH: 139 amino acids
      (B) TYPE: amino acids
      (C) TOPOLOGY: linear
   (ii) MOLECULE TYPE: protein
   (ix) FEATURE:
      (A) NAME: mOP-2 (mature form)
   (xi) SEQUENCE DESCRIPTION: SEQ ID NO:8:
   Ala Ala Arg Pro Leu Lys Arg Arg Gln
   1               5
   Pro Lys Lys Thr Asn Glu Leu Pro His
   10                  15
   Pro Asn Lys Leu Pro Gly Ile Phe Asp
       20                  25
   Asp Gly His Gly Ser Arg Gly Arg Glu
           30                  35
   Val Cys Arg Arg His Glu Leu Tyr Val
               40                  45
   Arg Phe Arg Asp Leu Gly Trp Leu Asp
                   50
   Trp Val Ile Ala Pro Gln Gly Tyr Ser
   55                  60
   Ala Tyr Tyr Cys Glu Gly Glu Cys Ala
       65                  70
   Phe Pro Leu Asp Ser Cys Met Asn Ala
           75                  80
   Thr Asn His Ala Ile Leu Gln Ser Leu
               85                  90
   Val His Leu Met Lys Pro Asp Val Val
                   95
   Pro Lys Ala Cys Cys Ala Pro Thr Lys
   100                 105
   Leu Ser Ala Thr Ser Val Leu Tyr Tyr
       110                 115
   Asp Ser Ser Asn Asn Val Ile Leu Arg
           120                 125
   Lys His Arg Asn Met Val Val Lys Ala
               130                 135
   Cys Gly Cys His
(2) INFORMATION FOR SEQ ID NO:9:
   (i) SEQUENCE CHARACTERISTICS:
      (A) LENGTH: 96 amino acids
      (B) TYPE: amino acids
      (C) TOPOLOGY: linear
   (ii) MOLECULE TYPE: protein
   (ix) FEATURE:
      (A) NAME: CBMP-2A(fx)
   (xi) SEQUENCE DESCRIPTION: SEQ ID NO:9:
(2) INFORMATION FOR SEQ ID NO:10:
   (i) SEQUENCE CHARACTERISTICS:
      (A) LENGTH: 101 amino acids
      (B) TYPE: amino acids
      (C) TOPOLOGY: linear
   (ii) MOLECULE TYPE: protein
   (ix) FEATURE:
      (A) NAME: CBMP-2B(fx)
   (xi) SEQUENCE DESCRIPTION: SEQ ID NO:10:
   Cys Arg Arg His Ser
    1               5
   Leu Tyr Val Asp Phe Ser Asp Val Gly Trp Asn
                        10                  15
   Asp Trp Ile Val Ala Pro Pro Gly Tyr Gln Ala
                    20                  25
   Phe Tyr Cys His Gly Asp Cys Pro Phe Pro Leu
                30                  35
   Ala Asp His Leu Asn Ser Thr Asn His Ala Ile
            40                  45
   Val Gln Thr Leu Val Asn Ser Val Asn Ser Ser
    50                  55                  60
   Ile Pro Lys Ala Cys Cys Val Pro Thr Glu Leu
                        65                  70
   Ser Ala Ile Ser Met Leu Tyr Leu Asp Glu Tyr
                    75                  80
   Asp Lys Val Val Leu Lys Asn Tyr Gln Glu Met
                85                  90
   Val Val Glu Gly Cys Gly Cys Arg
        95                 100
(2) INFORMATION FOR SEQ ID NO:11:
   (i) SEQUENCE CHARACTERISTICS:
      (A) LENGTH: 102 amino acids
      (B) TYPE: amino acids
      (C) TOPOLOGY: linear
   (ii) MOLECULE TYPE: protein
   (ix) FEATURE:
      (A) NAME: DPP(fx)
   (xi) SEQUENCE DESCRIPTION: SEQ ID NO:11:
   Cys Arg Arg His Ser Leu Tyr Val Asp Phe Ser
    1              5                   10
   Asp Val Gly Trp Asp Asp Trp Ile Val Ala Pro
               15                  20
   Leu Gly Tyr Asp Ala Tyr Tyr Cys His Gly Lys
           25                  30
   Cys Pro Phe Pro Leu Ala Asp His Phe Asn Ser
       35                  40
   Thr Asn His Ala Val Val Gln Thr Leu Val Asn
   45                  50                  55
   Asn Asn Asn Pro Gly Lys Val Pro Lys Ala Cys
                   60                  65
   Cys Val Pro Thr Gln Leu Asp Ser Val Ala Met
               70                  75
   Leu Tyr Leu Asn Asp Gln Ser Thr Val Val Leu
           80                  85
   Lys Asn Tyr Gln Glu Met Thr Val Val Gly Cys
       90                  95
   Gly Cys Arg
   100
(2) INFORMATION FOR SEQ ID NO:12:
   (i) SEQUENCE CHARACTERISTICS:
      (A) LENGTH: 102 amino acids
      (B) TYPE: amino acids
      (C) TOPOLOGY: linear
   (ii) MOLECULE TYPE: protein
   (ix) FEATURE:
      (A) NAME: Vgl(fx)
   (xi) SEQUENCE DESCRIPTION: SEQ ID NO:12:
   Cys Lys Lys Arg His Leu Tyr Val Glu Phe Lys
     1                 5                  10
   Asp Val Gly Trp Gln Asn Trp Val Ile Ala Pro
                   15                  20
   Gln Gly Tyr Met Ala Asn Tyr Cys Tyr Gly Glu
               25                  30
   Cys Pro Tyr Pro Leu Thr Glu Ile Leu Asn Gly
       35                  40
   Ser Asn His Ala Ile Leu Gln Thr Leu Val His
    45                 50                  55
   Ser Ile Glu Pro Glu Asp Ile Pro Leu Pro Cys
                       60                  65
   Cys Val Pro Thr Lys Met Ser Pro Ile Ser Met
                   70                  75
   Leu Phe Tyr Asp Asn Asn Asp Asn Val Val Leu
               80                  85
   Arg His Tyr Glu Asn Met Ala Val Asp Glu Cys
       90                  95
   Gly Cys Arg
   100
(2) INFORMATION FOR SEQ ID NO:13:
   (i) SEQUENCE CHARACTERISTICS:
      (A) LENGTH: 102 amino acids
      (B) TYPE: amino acids
      (C) TOPOLOGY: linear
   (ii) MOLECULE TYPE: protein
   (ix) FEATURE:
      (A) NAME: Vgr-1(fx)
   (xi) SEQUENCE DESCRIPTION: SEQ ID NO:13:
   Cys Lys Lys His Glu Leu Tyr Val Ser Phe Gln
    1              5                   10
   Asp Val Gly Trp Gln Asp Trp Ile Ile Ala Pro
               15                  20
   Xaa Gly Tyr Ala Ala Asn Tyr Cys Asp Gly Glu
           25                  30
   Cys Ser Phe Pro Leu Asn Ala His Met Asn Ala
       35                  40
   Thr Asn His Ala Ile Val Gln Thr Leu Val His
    45                 50                  55
   Val Met Asn Pro Glu Tyr Val Pro Lys Pro Cys
                   60                  65
   Cys Ala Pro Thr Lys Val Asn Ala Ile Ser Val
               70                  75
   Leu Tyr Phe Asp Asp Asn Ser Asn Val Ile Leu
           80                  85
   Lys Lys Tyr Arg Asn Met Val Val Arg Ala Cys
       90                  95
   Gly Cys His
   100
(2) INFORMATION FOR SEQ ID NO:14:
   (i) SEQUENCE CHARACTERISTICS:
      (A) LENGTH: 106 amino acids
      (B) TYPE: protein
      (C) STRANDEDNESS: single
      (D) TOPOLOGY: linear
   (ii) MOLECULE TYPE: protein
   (vi) ORIGINAL SOURCE:
      (A) ORGANISM: human
      (F) TISSUE TYPE: BRAIN
   (ix) FEATURE:
      (D) OTHER INFORMATION: /product= "GDF-1 (fx)"
   (xi) SEQUENCE DESCRIPTION: SEQ ID NO:14:
   Cys Arg Ala Arg Arg Leu Tyr Val Ser Phe Arg Glu Val Gly
    1               5                   10
   Trp His Arg Trp Val Ile Ala Pro Arg Gly Phe Leu Ala Asn Tyr
    15                  20                  25
   Cys Gln Gly Gln Cys Ala Leu Pro Val Ala Leu Ser Gly Ser Gly
    30                  35                  40
   Gly Pro Pro Ala Leu Asn His Ala Val Leu Arg Ala Leu Met His
    45                  50                  55
   Ala Ala Ala Pro Gly Ala Ala Asp Leu Pro Cys Cys Val Pro Ala
    60                  65                  70
   Arg Leu Ser Pro Ile Ser Val Leu Phe Phe Asp Asn Ser Asp Asn
    75                  80                  85
   Val Val Leu Arg Gln Tyr Glu Asp Met Val Val Asp Glu Cys Gly
    90                  95                  100
   Cys Arg
   105
(2) INFORMATION FOR SEQ ID NO:15:
   (i) SEQUENCE CHARACTERISTICS:
      (A) LENGTH: 5 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:15:
   Cys Xaa Xaa Xaa Xaa
    1               5
(2) INFORMATION FOR SEQ ID NO:16:
   (i) SEQUENCE CHARACTERISTICS:
      (A) LENGTH: 1822 base pairs
      (B) TYPE: nucleic acid
      (C) STRANDEDNESS: single
      (D) TOPOLOGY: linear

      (ii) MOLECULE TYPE: cDNA
      (vi) ORIGINAL SOURCE:
         (A) ORGANISM: HOBO SAPIENS
         (F) TISSUE TYPE: HIPPOCAHPUS
      (ix) FEATURE:
         (A) NAME/KEY: CDS
         (B) LOCATION: 49..1341
         (D) OTHER INFORMATION:/standard_name= "hOP1"
      (xi) SEQUENCE DESCRIPTION: SEQ ID NO:16:
   GGTGCGGGCC CGGAGCCCGG AGCCCGGGTA GCGCGTAGAG CCGGCGCG ATG CAC GTG       57
                                                        Met His Val
                                                          1
   CGC TCA CTG CGA GCT GCG GCG CCG CAC AGC TTC GTG GCG CTC TGG GCA       105
   Arg Ser Leu Arg Ala Ala Ala Pro His Ser Phe Val Ala Leu Trp Ala
         5                  10                  15
   CCC CTG TTC CTG CTG CGC TCC GCC CTG GCC GAC TTC AGC CTG GAC AAC       153
   Pro Leu Phe Leu Leu Arg Ser Ala Leu Ala Asp Phe Ser Leu Asp Asn
    20                  25                  30                  35
   GAG GTG CAC TCG AGC TTC ATC CAC CGG CGC CTC CGC AGC CAG GAG CGG       201
   Glu Val His Ser Ser Phe Ile His Arg Arg Leu Arg Ser Gln Glu Arg
                    40                  45                  50
   CGG GAG ATG CAG CGC GAG ATC CTC TCC ATT TTG GGC TTG CCC CAC CGC       249
   Arg Glu Met Gln Arg Glu Ile Leu Ser Ile Leu Gly Leu Pro His Arg
                55                  60                  65
   CCG CGC CCG CAC CTC CAG GGC AAG CAC AAC TCG GCA CCC ATG TTC ATG       297
   Pro Arg Pro His Leu Gln Gly Lys His Asn Ser Ala Pro Met Phe Met
            70                  75                  80
   CTG GAC CTG TAC AAC GCC ATG GCG GTG GAG GAG GGC GGC GGG CCC GGC       345
   Leu Asp Leu Tyr Asn Ala Met Ala Val Glu Glu Gly Gly Gly Pro Gly
        85                  90                  95
   GGC CAG GGC TTC TCC TAC CCC TAC AAG GCC GTC TTC AGT ACC CAG GGC       393
   Gly Gln Gly Phe Ser Tyr Pro Tyr Lys Ala Val Phe Ser Thr Gln Gly
   100                 105                 110                 115
   CCC CCT CTG GCC AGC CTG CAA GAT AGC CAT TTC CTC ACC GAC GCC GAC      441
   Pro Pro Leu Ala Ser Leu Gln Asp Ser His Phe Leu Thr Asp Ala Asp
                   120                 125                 130
   ATG GTC ATG AGC TTC GTC AAC CTC GTG GAA CAT GAC AAG GAA TTC TTC      489
   Met Val Met Ser Phe Val Asn Leu Val Glu His Asp Lys Glu Phe Phe
               135                 140                 145
   CAC CCA CGC TAC CAC CAT CGA GAG TTC CGG TTT GAT CTT TCC AAG ATC      537
   His Pro Arg Tyr His His Arg Glu Phe Arg Phe Asp Leu Ser Lys Ile
           150                 155                 160
   CCA GAA GGG GAA GCT GTC ACG GCA GCC GAA TTC CGG ATC TAC AAG GAC      585
   Pro Glu Gly Glu Ala Val Thr Ala Ala Glu Phe Arg Ile Tyr Lys Asp
       165                 170                 175
   TAC ATC CGG GAA CGC TTC GAC AAT GAG ACG TTC CGG ATC AGC GTT TAT      633
   Tyr Ile Arg Glu Arg Phe Asp Asn Glu Thr Phe Arg Ile Ser Val Tyr
   180                 185                 190                 195
   CAG GTG CTC CAG GAG CAC TTG GGC AGG GAA TCG GAT CTC TTC CTG CTC      681
   Gln Val Leu Gln Glu His Leu Gly Arg Glu Ser Asp Leu Phe Leu Leu
                   200                 205                 210
   GAC AGC CGT ACC CTC TGG GCC TCG GAG GAG GGC TGG CTG GTG TTT GAC      729
   Asp Ser Arg Thr Leu Trp Ala Ser Glu Glu Gly Trp Leu Val Phe Asp
               215                 220                 225
   ATC ACA GCC ACC AGC AAC CAC TGG GTG GTC AAT CCG CGG CAC AAC CTG      777
   Ile Thr Ala Thr Ser Asn His Trp Val Val Asn Pro Arg His Asn Leu
           230                 235                 240
   GGC CTG CAG CTC TCG GTG GAG ACG CTG GAT GGG CAG AGC ATC AAC CCC      825
   Gly Leu Gln Leu Ser Val Glu Thr Leu Asp Gly Gln Ser Ile Asn Pro
       245                 250                 255
   AAG TTG GCG GGC CTG ATT GGG CGG CAC GGG CCC CAG AAC AAG CAG CCC      873
   Lys Leu Ala Gly Leu Ile Gly Arg His Gly Pro Gln Asn Lys Gln Pro
   260                 265                 270                 275
   TTC ATG GTG GCT TTC TTC AAG GCC ACG GAG GTC CAC TTC CGC AGC ATC      921
   Phe Met Val Ala Phe Phe Lys Ala Thr Glu Val His Phe Arg Ser Ile
                   280                 285                 290
   CGG TCC ACG GGG AGC AAA CAG CGC AGC CAG AAC CGC TCC AAG ACG CCC      969
   Arg Ser Thr Gly Ser Lys Gln Arg Ser Gln Asn Arg Ser Lys Thr Pro
               295                 300                 305
   AAG AAC CAG GAA GCC CTG CGG ATG GCC AAC GTG GCA GAG AAC AGC AGC     1017
   Lys Asn Gln Glu Ala Leu Arg Met Ala Asn Val Ala Glu Asn Ser Ser
           310                 315                 320
   AGC GAC CAG AGG CAG GCC TGT AAG AAG CAC GAG CTG TAT GTC AGC TTC         1065
   Ser Asp Gln Arg Gln Ala Cys Lys Lys His Glu Leu Tyr Val Ser Phe
       325                 330                 335
   CGA GAC CTG GGC TGG CAG GAC TGG ATC ATC GCG CCT GAA GGC TAC GCC         1113
   Arg Asp Leu Gly Trp Gln Asp Trp Ile Ile Ala Pro Glu Gly Tyr Ala
   340                 345                 350                 355
   GCC TAC TAC TGT GAG GGG GAG TGT GCC TTC CCT CTG AAC TCC TAC ATG         1161
   Ala Tyr Tyr Cys Glu Gly Glu Cys Ala Phe Pro Leu Asn Ser Tyr Met
                   360                 365                 370
   AAC GCC ACC AAC CAC GCC ATC GTG CAG ACG CTG GTC CAC TTC ATC AAC         1209
   Asn Ala Thr Asn His Ala Ile Val Gln Thr Leu Val His Phe Ile Asn
               375                 380                 385
   CCG GAA ACG GTG CCC AAG CCC TGC TGT GCG CCC ACG CAG CTC AAT GCC         1257
   Pro Glu Thr Val Pro Lys Pro Cys Cys Ala Pro Thr Gln Leu Asn Ala
           390                 395                 400
   ATC TCC GTC CTC TAC TTC GAT GAC AGC TCC AAC GTC ATC CTG AAG AAA         1305
   Ile Ser Val Leu Tyr Phe Asp Asp Ser Ser Asn Val Ile Leu Lys Lys
       405                 410                 415
   TAC AGA AAC ATG GTG GTC CGG GCC TGT GGC TGC CAC TAGCTCCTCC              1351
   Tyr Arg Asn Met Val Val Arg Ala Cys Gly Cys His
   420                 425                 430
   GAGAATTCAG ACCCTTTGGG GCCAAGTTTT TCTGGATCCT CCATTGCTCG CCTTGGCCAG       1411
   GAACCAGCAG ACCAACTGCC TTTTGTGAGA CCTTCCCCTC CCTATCCCCA ACTTTAAAGG       1471
   TGTGAGAGTA TTAGGAAACA TGAGCAGCAT ATGGCTTTTG ATCAGTTTTT CAGTGGCAGC       1531
   ATCCAATGAA CAAGATCCTA CAAGCTGTGC AGGCAAAACC TAGCAGGAAA AAAAAACAAC       1591
   GCATAAAGAA AAATGGCCGG GCCAGGTCAT TGGCTGGGAA GTCTCAGCCA TGCACGGACT       1651
   CGTTTCCAGA GGTAATTATG AGCGCCTACC AGCCAGGCCA CCCAGCCGTG GGAGGAAGGG       1711
   GGCGTGGCAA GGGGTGGGCA CATTGGTGTC TGTGCGAAAG GAAAATTGAC CCGGAAGTTC       1771
   CTGTAATAAA TGTCACAATA AAACGAATGA ATGAAAAAAA AAAAAAAAAA A                1822
(2) INFORMATION FOR SEQ ID NO:17:
   (i) SEQUENCE CHARACTERISTICS:
      (A) LENGTH: 431 amino acids
      (B) TYPE: amino acid
      (D) TOPOLOGY: linear
   (ii) MOLECULE TYPE: protein
   (ix) FEATURE:
      (D) OTHER INFORMATION: /Product="OP1-PP"
   (xi) SEQUENCE DESCRIPTION: SEQ ID NO:17:
   Met His Val Arg Ser Leu Arg Ala Ala Ala Pro His Ser Phe Val Ala
    1               5                  10                  15
   Leu Trp Ala Pro Leu Phe Leu Leu Arg Ser Ala Leu Ala Asp Phe Ser
               20                  25                  30
   Leu Asp Asn Glu Val His Ser Ser Phe Ile His Arg Arg Leu Arg Ser
           35                  40                  45
   Gln Glu Arg Arg Glu Met Gln Arg Glu Ile Leu Ser Ile Leu Gly Leu
       50                  55                  60
   Pro His Arg Pro Arg Pro His Leu Gln Gly Lys His Asn Ser Ala Pro
   65                  70                  75                  80
   Met Phe Met Leu Asp Leu Tyr Asn Ala Met Ala Val Glu Glu Gly Gly
                   85                  90                  95
   Gly Pro Gly Gly Gln Gly Phe Ser Tyr Pro Tyr Lys Ala Val Phe Ser
               100                 105                 110
   Thr Gln Gly Pro Pro Leu Ala Ser Leu Gln Asp Ser His Phe Leu Thr
           115                 120                 125
   Asp Ala Asp Met Val Met Ser Phe Val Asn Leu Val Glu His Asp Lys
       130                 135                 140
   Glu Phe Phe His Pro Arg Tyr His His Arg Glu Phe Arg Phe Asp Leu
   145                 150                 155                 160
   Ser Lys Ile Pro Glu Gly Glu Ala Val Thr Ala Ala Glu Phe Arg Ile
                   165                 170                 175
   Tyr Lys Asp Tyr Ile Arg Glu Arg Phe Asp Asn Glu Thr Phe Arg Ile
               180                 185                 190
   Ser Val Tyr Gln Val Leu Gln Glu His Leu Gly Arg Glu Ser Asp Leu
           195                 200                 205
   Phe Leu Leu Asp Ser Arg Thr Leu Trp Ala Ser Glu Glu Gly Trp Leu
       210                 215                 220
   Val Phe Asp Ile Thr Ala Thr Ser Asn His Trp Val Val Asn Pro Arg
   225                 230                 235                 240
   His Asn Leu Gly Leu Gln Leu Ser Val Glu Thr Leu Asp Gly Gln Ser
                   245                 250                 255
   Ile Asn Pro Lys Leu Ala Gly Leu Ile Gly Arg His Gly Pro Gln Asn
               260                 265                 270
   Lys Gln Pro Phe Met Val Ala Phe Phe Lys Ala Thr Glu Val His Phe
           275                 280                 285
   Arg Ser Ile Arg Ser Thr Gly Ser Lys Gln Arg Ser Gln Asn Arg Ser
       290                 295                 300
   Lys Thr Pro Lys Asn Gln Glu Ala Leu Arg Met Ala Asn Val Ala Glu
   305                 310                 315                 320
   Asn Ser Ser Ser Asp Gln Arg Gln Ala Cys Lys Lys His Glu Leu Tyr
                   325                 330                 335
   Val Ser Phe Arg Asp Leu Gly Trp Gln Asp Trp Ile Ile Ala Pro Glu
               340                 345                 350
   Gly Tyr Ala Ala Tyr Tyr Cys Glu Gly Glu Cys Ala Phe Pro Leu Asn
           355                 360                 365
   Ser Tyr Met Asn Ala Thr Asn His Ala Ile Val Gln Thr Leu Val His
       370                 375                 380
   Phe Ile Asn Pro Glu Thr Val Pro Lys Pro Cys Cys Ala Pro Thr Gln
   385                 390                 395                 400
   Leu Asn Ala Ile Ser Val Leu Tyr Phe Asp Asp Ser Ser Asn Val Ile
                   405                 410                 415
   Leu Lys Lys Tyr Arg Asn Met Val Val Arg Ala Cys Gly Cys His
               420                 425                 430
(2) INFORMATION FOR SEQ ID NO:18:
   (i) SEQUENCE CHARACTERISTICS:
      (A) LENGTH: 1873 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: MURIDAE
      (F) TISSUE TYPE: EMBRYO
   (ix) FEATURE:
      (A) NAME/KEY: CDS
      (B) LOCATION: 104..1393
      (D) OTHER INFORMATION: /note= "MOP1 (CDNA)"
   (xi) SEQUENCE DESCRIPTION: SEQ ID NO:18:
   CTGCAGCAAG TGACCTCGGG TCGTGGACCG CTGCCCTGCC CCCTCCGCTG CCACCTGGGG        60
   CGGCGCGGGC CCGGTGCCCC GGATCGCGCG TAGAGCCGGC GCG ATG CAC GTG CGC         115
                                                   Met His Val Arg
                                                    1
   TCG CTG CGC GCT GCG GCG CCA CAC AGC TTC GTG GCG CTC TGG GCG CCT         163
   Ser Leu Arg Ala Ala Ala Pro His Ser Phe Val Ala Leu Trp Ala Pro
    5                  10                  15                  20
   CTG TTC TTG CTG CGC TCC GCC CTG GCC GAT TTC AGC CTG GAC AAC GAG         211
   Leu Phe Leu Leu Arg Ser Ala Leu Ala Asp Phe Ser Leu Asp Asn Glu
                   25                  30                  35
   GTG CAC TCC AGC TTC ATC CAC CGG CGC CTC CGC AGC CAG GAG CGG CGG         259
   Val His Ser Ser Phe Ile His Arg Arg Leu Arg Ser Gln Glu Arg Arg
               40                  45                  50
   GAG ATG CAG CGG GAG ATC CTG TCC ATC TTA GGG TTG CCC CAT CGC CCG         307
   Glu Met Gln Arg Glu Ile Leu Ser Ile Leu Gly Leu Pro His Arg Pro
           55                  60                  65
   CGC CCG CAC CTC CAG GGA AAG CAT AAT TCG GCG CCC ATG TTC ATG TTG         355
   Arg Pro His Leu Gln Gly Lys His Asn Ser Ala Pro Met Phe Met Leu
       70                  75                  80
   GAC CTG TAC AAC GCC ATG GCG GTG GAG GAG AGC GGG CCG GAC GGA CAG         403
   Asp Leu Tyr Asn Ala Met Ala Val Glu Glu Ser Gly Pro Asp Gly Gln
    85                 90                  95                 100
   GGC TTC TCC TAC CCC TAC AAG GCC GTC TTC AGT ACC CAG GGC CCC CCT         451
   Gly Phe Ser Tyr Pro Tyr Lys Ala Val Phe Ser Thr Gln Gly Pro Pro
                   105                 110                 115
   TTA GCC AGC CTG CAG GAC AGC CAT TTC CTC ACT GAC GCC GAC ATG GTC         499
   Leu Ala Ser Leu Gln Asp Ser His Phe Leu Thr Asp Ala Asp Met Val
               120                 125                 130
   ATG AGC TTC GTC AAC CTA GTG GAA CAT GAC AAA GAA TTC TTC CAC CCT         547
   Met Ser Phe Val Asn Leu Val Glu His Asp Lys Glu Phe Phe His Pro
           135                 140                 145
   CGA TAC CAC CAT CGG GAG TTC CGG TTT GAT CTT TCC AAG ATC CCC GAG         595
   Arg Tyr His His Arg Glu Phe Arg Phe Asp Leu Ser Lys Ile Pro Glu
       150                 155                 160
   GGC GAA GCG GTG ACC GCA GCC GAA TTC AGG ATC TAT AAG GAC TAC ATC         643
   Gly Glu Ala Val Thr Ala Ala Glu Phe Arg Ile Tyr Lys Asp Tyr Ile
   165                 170                 175                 180
   CGG GAG CGA TTT GAC AAC GAG ACC TTC CAG ATC ACA GTC TAT CAG TGG     691
   Arg Glu Arg Phe Asp Asn Glu Thr Phe Gln Ile Thr Val Tyr Gln Trp
                   185                 190                 195
   CTC CAG GAG CAC TCA GGC AGG GAG TCG GAC CTC TTC TTG CTG GAC AGC     739
   Leu Gln Glu His Ser Gly Arg Glu Ser Asp Leu Phe Leu Leu Asp Ser
               200                 205                 210
   CGC ACC ATC TGG GCT TCT GAG GAG GGC TGG TTG GTG TTT GAT ATC ACA     787
   Arg Thr Ile Trp Ala Ser Glu Glu Gly Trp Leu Val Phe Asp Ile Thr
           215                 220                 225
   GCC ACC AGC AAC CAC TGG GTG GTC AAC CCT CGG CAC AAC CTG GGC TTA     835
   Ala Thr Ser Asn His Trp Val Val Asn Pro Arg His Asn Leu Gly Leu
       230                 235                 240
   CAG CTC TCT GTG GAG ACC CTG GAT GGG CAG AGC ATC AAC CCC AAG TTG     883
   Gln Leu Ser Val Glu Thr Leu Asp Gly Gln Ser Ile Asn Pro Lys Leu
   245                 250                 255                 260
   GCA GGC CTG ATT GGA CGG CAT GGA CCC CAG AAC AAG CAA CCC TTC ATG     931
   Ala Gly Leu Ile Gly Arg His Gly Pro Gln Asn Lys Gln Pro Phe Met
                   265                 270                 275
   GTG GCC TTC TTC AAG GCC ACG GAA GTC CAT CTC CGT AGT ATC CGG TCC     979
   Val Ala Phe Phe Lys Ala Thr Glu Val His Leu Arg Ser Ile Arg Ser
               280                 285                 290
   ACG GGG GGC AAG CAG CGC AGC CAG AAT CGC TCC AAG ACG CCA AAG AAC    1027
   Thr Gly Gly Lys Gln Arg Ser Gln Asn Arg Ser Lys Thr Pro Lys Asn
           295                 300                 305
   CAA GAG GCC CTG AGG ATG GCC AGT GTG GCA GAA AAC AGC AGC AGT GAC    1075
   Gln Glu Ala Leu Arg Met Ala Ser Val Ala Glu Asn Ser Ser Ser Asp
       310                 315                 320
   CAG AGG CAG GCC TGC AAG AAA CAT GAG CTG TAC GTC AGC TTC CGA GAC    1123
   Gln Arg Gln Ala Cys Lys Lys His Glu Leu Tyr Val Ser Phe Arg Asp
   325                 330                 335                 340
   CTT GGC TGG CAG GAC TGG ATC ATT GCA CCT GAA GGC TAT GCT GCC TAC    1171
   Leu Gly Trp Gln Asp Trp Ile Ile Ala Pro Glu Gly Tyr Ala Ala Tyr
                   345                 350                 355
   TAC TGT GAG GGA GAG TGC GCC TTC CCT CTG AAC TCC TAC ATG AAC GCC    1219
   Tyr Cys Glu Gly Glu Cys Ala Phe Pro Leu Asn Ser Tyr Met Asn Ala
               360                 365                 370
   ACC AAC CAC GCC ATC GTC CAG ACA CTG GTT CAC TTC ATC AAC CCA GAC    1267
   Thr Asn His Ala Ile Val Gln Thr Leu Val His Phe Ile Asn Pro Asp
           375                 380                 385
   ACA GTA CCC AAG CCC TGC TGT GCG CCC ACC CAG CTC AAC GCC ATC TCT       1315
   Thr Val Pro Lys Pro Cys Cys Ala Pro Thr Gln Leu Asn Ala Ile Ser
       390                 395                 400
   GTC CTC TAC TTC GAC GAC AGC TCT AAT GTC ATC CTG AAG AAG TAC AGA       1363
   Val Leu Tyr Phe Asp Asp Ser Ser Asn Val Ile Leu Lys Lys Tyr Arg
   405                 410                 415                 420
   AAC ATG GTG GTC CGG GCC TGT GGC TGC CAC TAGCTCTTCC TGAGACCCTG         1413
   Asn Met Val Val Arg Ala Cys Gly Cys His
                   425                 430
   ACCTTTGCGG GGCCACACCT TTCCAAATCT TCGATGTCTC ACCATCTAAG TCTCTCACTG    1473
   CCCACCTTGG CGAGGAGAAC AGACCAACCT CTCCTGAGCC TTCCCTCACC TCCCAACCGG    1533
   AAGCATGTAA GGGTTCCAGA AACCTGAGCG TGCAGCAGCT GATGAGCGCC CTTTCCTTCT    1593
   GGCACGTGAC GGACAAGATC CTACCAGCTA CCACAGCAAA CGCCTAAGAG CAGGAAAAAT    1653
   GTCTGCCAGG AAAGTGTCCA GTGTCCACAT GGCCCCTGGC GCTCTGAGTC TTTGAGGAGT    1713
   AATCGCAAGC CTCGTTCAGC TGCAGCAGAA GGAAGGGCTT AGCCAGGGTG GGCGCTGGCG    1773
   TCTGTGTTGA AGGGAAACCA AGCAGAAGCC ACTGTAATGA TATGTCACAA TAAAACCCAT    1833
   GAATGAAAAA AAAAAAAAAA AAAAAAAAAA AAAAGAATTC                          1873
(2) INFORMATION FOR SEQ ID NO:19:
   (i) SEQUENCE CHARACTERISTICS:
      (A) LENGTH: 430 amino acids
      (B) TYPE: amino acid
      (D) TOPOLOGY: linear
   (ii) MOLECULE TYPE: protein
   (ix) FEATURE:
      (D) OTHER INFORMATION: /product= "mOP1-PP"
   (xi) SEQUENCE DESCRIPTION: SEQ ID NO:19:
   Met His Val Arg Ser Leu Arg Ala Ala Ala Pro His Ser Phe Val Ala
    1               5                  10                  15
   Leu Trp Ala Pro Leu Phe Leu Leu Arg Ser Ala Leu Ala Asp Phe Ser
               20                  25                  30
   Leu Asp Asn Glu Val His Ser Ser Phe Ile His Arg Arg Leu Arg Ser
           35                  40                  45
   Gln Glu Arg Arg Glu Met Gln Arg Glu Ile Leu Ser Ile Leu Gly Leu
        50                  55                  60
   Pro His Arg Pro Arg Pro His Leu Gln Gly Lys His Asn Ser Ala Pro
    65                  70                  75                  80
   Met Phe Met Leu Asp Leu Tyr Asn Ala Met Ala Val Glu Glu Ser Gly
                    85                  90                  95
   Pro Asp Gly Gln Gly Phe Ser Tyr Pro Tyr Lys Ala Val Phe Ser Thr
               100                 105                 110
   Gln Gly Pro Pro Leu Ala Ser Leu Gln Asp Ser His Phe Leu Thr Asp
           115                 120                 125
   Ala Asp Met Val Met Ser Phe Val Asn Leu Val Glu His Asp Lys Glu
       130                 135                 140
   Phe Phe His Pro Arg Tyr His His Arg Glu Phe Arg Phe Asp Leu Ser
   145                 150                 155                 160
   Lys Ile Pro Glu Gly Glu Ala Val Thr Ala Ala Glu Phe Arg Ile Tyr
                   165                 170                 175
   Lys Asp Tyr Ile Arg Glu Arg Phe Asp Asn Glu Thr Phe Gln Ile Thr
               180                 185                 190
   Val Tyr Gln Trp Leu Gln Glu His Ser Gly Arg Glu Ser Asp Leu Phe
           195                 200                 205
   Leu Leu Asp Ser Arg Thr Ile Trp Ala Ser Glu Glu Gly Trp Leu Val
       210                 215                 220
   Phe Asp Ile Thr Ala Thr Ser Asn His Trp Val Val Asn Pro Arg His
   225                 230                 235                 240
   Asn Leu Gly Leu Gln Leu Ser Val Glu Thr Leu Asp Gly Gln Ser Ile
                   245                 250                 255
   Asn Pro Lys Leu Ala Gly Leu Ile Gly Arg His Gly Pro Gln Asn Lys
               260                 265                 270
   Gln Pro Phe Met Val Ala Phe Phe Lys Ala Thr Glu Val His Leu Arg
           275                 280                 285
   Ser Ile Arg Ser Thr Gly Gly Lys Gln Arg Ser Gln Asn Arg Ser Lys
       290                 295                 300
   Thr Pro Lys Asn Gln Glu Ala Leu Arg Met Ala Ser Val Ala Glu Asn
   305                 310                 315                 320
   Ser Ser Ser Asp Gln Arg Gln Ala Cys Lys Lys His Glu Leu Tyr Val
                   325                 330                 335
   Ser Phe Arg Asp Leu Gly Trp Gln Asp Trp Ile Ile Ala Pro Glu Gly
               340                 345                 350
   Tyr Ala Ala Tyr Tyr Cys Glu Gly Glu Cys Ala Phe Pro Leu Asn Ser
           355                 360                 365
   Tyr Met Asn Ala Thr Asn His Ala Ile Val Gln Thr Leu Val His Phe
       370                 375                 380
   Ile Asn Pro Asp Thr Val Pro Lys Pro Cys Cys Ala Pro Thr Gln Leu
   385                 390                 395                 400
   Asn Ala Ile Ser Val Leu Tyr Phe Asp Asp Ser Ser Asn Val Ile Leu
                   405                 410                 415
   Lys Lys Tyr Arg Asn Met Val Val Arg Ala Cys Gly Cys His
               420                 425                 430
(2) INFORMATION FOR SEQ ID NO:20:
   (i)SEQUENCE CHARACTERISTICS:
      (A) LENGTH: 1723 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
      (F) TISSUE TYPE: HIPPOCAMPUS
   (ix)FEATURE:
      (A) NAME/KEY: CDS
      (B) LOCATION: 490..1696
      (D) OTHER INFORMATION: /note= "hOP2 (cDNA)"
   (xi)SEQUENCE DESCRIPTION: SEQ ID NO:20:
   GGCGCCGGCA GAGCAGGAGT GGCTGGAGGA GCTGTGGTTG GAGCAGGAGG TGGCACGGCA    60
   GGGCTGGAGG GCTCCCTATG AGTGGCGGAG ACGGCCCAGG AGGCGCTGGA GCAACAGCTC   120
   CCACACCGCA CCAAGCGGTG GCTGCAGGAG CTCGCCCATC GCCCCTGCGC TGCTCGGACC   180
   GCGGCCACAG CCGGACTGGC GGGTACGGCG GCGACAGAGG CATTGGCCGA GAGTCCCAGT   240
   CCGCAGAGTA GCCCCGGCCT CGAGGCGGTG GCGTCCCGGT CCTCTCCGTC CAGGAGCCAG   300
   GACAGGTGTC GCGCGGCGGG GCTCCAGGGA CCGCGCCTGA GGCCGGCTGC CCGCCCGTCC   360
   CGCCCCGCCC CGCCGCCCGC CGCCCGCCGA GCCCAGCCTC CTTGCCGTCG GGGCGTCCCC   420
   AGGCCCTGGG TCGGCCGCGG AGCCGATGCG CGCCCGCTGA GCGCCCCAGC TGAGCGCCCC        480
   CGGCCTGCC ATG ACC GCG CTC CCC GGC CCG CTC TGG CTC CTG GGC CTG            528
   GCG CTA TGC GCG CTG GGC GGG GGC GGC CCC GGC CTG CGA CCC CCG CCC          576
   GGC TGT CCC CAG CGA CGT CTG GGC GCG CGC GAG CGC CGG GAC GTG CAG          624
   CGC GAG ATC CTG GCG GTG CTC GGG CTG CCT GGG CGG CCC CGG CCC CGC          672
   GCG CCA CCC GCC GCC TCC CGG CTG CCC GCG TCC GCG CCG CTC TTC ATG          720
   CTG GAC CTG TAC CAC GCC ATG GCC GGC GAC GAC GAC GAG GAC GGC GCG          768
   CCC GCG GAG CGG CGC CTG GGC CGC GCC GAC CTG GTC ATG AGC TTC GTT          816
   AAC ATG GTG GAG CGA GAC CGT GCC CTG GGC CAC CAG GAG CCC CAT TGG          864
   AAG GAG TTC CGC TTT GAC CTG ACC CAG ATC CCG GCT GGG GAG GCG GTC          912
   ACA GCT GCG GAG TTC CGG ATT TAC AAG GTG CCC AGC ATC CAC CTG CTC          960
   AAC AGG ACC CTC CAC GTC AGC ATG TTC CAG GTG GTC CAG GAG CAG TCC         1008
   AAC AGG GAG TCT GAC TTG TTC TTT TTG GAT CTT CAG ACG CTC CGA GCT         1056
   GGA GAC GAG GGC TGG CTG GTG CTG GAT GTC ACA GCA GCC AGT GAC TGC         1104
   TGG TTG CTG AAG CGT CAC AAG GAC CTG GGA CTC CGC CTC TAT GTG GAG      1152
   ACT GAG GAC GGG CAC AGC GTG GAT CCT GGC CTG GCC GGC CTG CTG GGT      1200
   CAA CGG GCC CCA CGC TCC CAA CAG CCT TTC GTG GTC ACT TTC TTC AGG      1248
   GCC AGT CCG AGT CCC ATC CGC ACC CCT CGG GCA GTG AGG CCA CTG AGG      1296
   AGG AGG CAG CCG AAG AAA AGC AAC GAG CTG CCG CAG GCC AAC CGA CTC      1344
   CCA GGG ATC TTT GAT GAC GTC CAC GGC TCC CAC GGC CGG CAG GTC TGC      1392
   CGT CGG CAC GAG CTC TAC GTC AGC TTC CAG GAC CTC GGC TGG CTG GAC      1440
   TGG GTC ATC GCT CCC CAA GGC TAC TCG GCC TAT TAC TGT GAG GGG GAG      1488
   TGC TCC TTC CCA CTG GAC TCC TGC ATG AAT GCC ACC AAC CAC GCC ATC      1536
   CTG CAG TCC CTG GTG CAC CTG ATG AAG CCA AAC GCA GTC CCC AAG GCG      1584
   TGC TGT GCA CCC ACC AAG CTG AGC GCC ACC TCT GTG CTC TAC TAT GAC      1632
   AGC AGC AAC AAC GTC ATC CTG CGC AAA CAC CGC AAC ATG GTG GTC AAG      1680
   GCC TGC GGC TGC CAC T GAGTCAGCCC GCCCAGCCCT ACTGCAG                  1723
(2) INFORMATION FOR SEQ ID NO:21:
   (i)SEQUENCE CHARACTERISTICS:
      (A) LENGTH: 402 amino acids
      (B) TYPE: amino acid
      (D) TOPOLOGY: linear
   (ii)MOLECULE TYPE: protein
   (ix)FEATURE:
      (A)OTHER INFORMATION: /product= "hOP2-PP"
   (xi)SEQUENCE DESCRIPTION: SEQ ID NO:21:
(2) INFORHATION FOR SEQ ID NO:22:
   (i) SEQUENCE CHARACTERISTICS:
      (A) LENGTH: 1926 base pairs
      (B) TYPE: nucleic acid
      (C) STRANDEDNESS: single
      (D TOPOLOGY: linear
   (ii) MOLECULE TYPE: cDNA
   (vi) ORIGINAL SOURCE:
      (A) ORGANISM: MURIDAE
      (F) TISSUE TYPE: EMBRYO
   (ix) FEATURE:
      (A) NAME/KEY: CDS
      (B) LOCATION: 93..1289
      (D) OTHER INFORMATION: /note= "mOP2 cDNA"
   (xi) SEQUENCE DESCRIPTION: SEQ ID NO:22:
(2) INFORMATION FOR SEQ ID NO:23:
   (i) SEQUENCE CHARACTERISTICS:
      (A) LENGTH: 399 amino acids
      (B) TYPE: amino acid
      (D) TOPOLOGY: linear
   (ii) MOLECULE TYPE: protein
   (ix) FEATURE:
      (D) OTHER INFORMATION: /product= "m0P2-PP"
   (xi) SEQUENCE DESCRIPTION: SEQ ID NO:23:
   Met Ala Met Arg Pro Gly Pro Leu Trp Leu Leu Gly Leu Ala Leu Cys
    1               5                  10                  15
   Ala Leu Gly Gly Gly His Gly Pro Arg Pro Pro His Thr Cys Pro Gln
               20                  25                  30
   Arg Arg Leu Gly Ala Arg Glu Arg Arg Asp Met Gln Arg Glu Ile Leu Ala
           35                  40                  45
   Val Leu Gly Leu Pro Gly Arg Pro Arg Pro Arg Ala Gln Pro Ala Ala
    50                  55                  60                  65
   Ala Arg Gln Pro Ala Ser Ala Pro Leu Phe Met Leu Asp Leu Tyr His Ala
                    70                  75                  80
   Met Thr Asp Asp Asp Asp Gly Gly Pro Pro Gln Ala His Leu Gly Arg
            85                  90                  95
   Ala Asp Leu Val Met Ser Phe Val Asn Met Val Glu Arg Asp Arg Thr
       100                 105                 110
   Leu Gly Tyr Gln Glu Pro His Trp Lys Glu Phe His Phe Asp Leu Thr
   115                 120                 125                 130
   Gln Ile Pro Ala Gly Glu Ala Val Thr Ala Ala Glu Phe Arg Ile Tyr
                   135                 140                 145
   Lys Glu Pro Ser Thr His Pro Leu Asn Thr Thr Leu His Ile Ser Met
               150                 155                 160
   Phe Glu Val Val Gln Glu His Ser Asn Arg Glu Ser Asp Leu Phe Phe
           165                 170                 175
   Leu Asp Leu Gln Thr Leu Arg Ser Gly Asp Glu Gly Trp Leu Val Leu
       180                 185                 190
   Asp Ile Thr Ala Ala Ser Asp Arg Trp Leu Leu Asn His His Lys Asp
   195                 200                 205                 210
   Leu Gly Leu Arg Leu Tyr Val Glu Thr Ala Asp Gly His Ser Met Asp
                   215                 220                 225
   Pro Gly Leu Ala Gly Leu Leu Gly Arg Gln Ala Pro Arg Ser Arg Gln
               230                 235                 240
   Pro Phe Met Val Thr Phe Phe Arg Ala Ser Gln Ser Pro Val Arg Ala
           245                 250                 255
   Pro Arg Ala Ala Arg Pro Leu Lys Arg Arg Gln Pro Lys Lys Thr Asn
       260                 265                 270
   Glu Leu Pro His Pro Asn Lys Leu Pro Gly Ile Phe Asp Asp Gly His
   275                 280                 285                 290
   Gly Ser Arg Gly Arg Glu Val Cys Arg Arg His Glu Leu Tyr Val Ser
                   295                 300                 305
   Phe Arg Asp Leu Gly Trp Leu Asp Trp Val Ile Ala Pro Gln Gly Tyr
               310                 315                 320
   Ser Ala Tyr Tyr Cys Glu Gly Glu Cys Ala Phe Pro Leu Asp Ser Cys
   325 330 335
   Met Asn Ala Thr Asn His Ala Ile Leu Gln Ser Leu Val His Leu Met
   340 345 350
   Lys Pro Asp Val Val Pro Lys Ala Cys Cys Ala Pro Thr Lys Leu Ser
   355 360 365 370
   Ala Thr Ser Val Leu Tyr Tyr Asp Ser Ser Asn Asn Val Ile Leu Arg
   375 380 385
   Lys His Arg Asn Met Val Val Lys Ala Cys Gly Cys His
   390 395

## Description

### Background of the Invention

Cell differentiation is the central characteristic of morphogenesis which initiates in the embryo, and continues to various degrees throughout the life of an organism in adult tissue repair and regeneration mechanisms. The degree of morphogenesis in adult tissue varies among different tissues and is related, among other things, to the degree of cell turnover in a given tissue. On this basis, tissues can be divided into three broad categories: (1) tissues with static cell populations such as nerve and skeletal muscle where there is no cell division and most of the cells formed during early development persist throughout adult life; (2) tissues containing conditionally renewing populations such as liver where there is generally little cell division but, in response to an appropriate stimulus, cells can divide to produce daughters of the same differentially defined type; and (3) tissues with permanently renewing populations including blood, testes and stratified squamous epithelia which are characterized by rapid and continuous cell turnover in the adult. Here, the terminally differentiated cells have a relatively short life span and are replaced through proliferation of a distinct subpopulation of cells, known as stem or progenitor cells.

The cellular and molecular events which govern the stimulus for differentiation of these cells is an area of intensive research. In the medical field, it is anticipated that the discovery of factor(s) which control cell differentiation and tissue morphogenesis will significantly advance medicine's ability to repair and regenerate diseased or damaged mammalian tissues and organs. Particularly useful areas include reconstructive surgery and in the treatment of tissue degenerative diseases including arthritis, emphysema, osteoporosis, cardiomyopathy, cirrhosis, and degenerative nerve diseases.

A number of different factors have been isolated in recent years which appear to play a role in cell differentiation. Some of these factors are gene transcription activators such as the NOTCH gene, identified in Drosophila and the related XOTCH gene identified in Xenopus, as well as a number of transcription activators identified in Caenorhabditis elegans.

The hemopoietic system, because of its continually renewing cell population, is an area of concentrated study. Factors identified in this system which may be involved in cell renewal include interleukin 3 (IL-3), erythropoietin, the CSFs (GM-CSF, G-CSF, M-CSF et al.) and various stem cell growth factors.

Other proteins thought to play a role in cell differentiation include proteins that are members of the family of insulin-like growth factors (IGF), members of the family of heparin-binding growth factors, (e.g., FGF - acidic and basic fibroblast growth factors, and ECDGF - embryonal carcinoma-derived growth factor) as well as several transforming oncogenes (hst and int-2, see for example, Heath et al., (1988), J. Cell Sci. Suppl. 10:256-256.) DIF (Differentiation Inducing Factor), identified in Dictyostelium discoideum, is another bioregulatory protein, directing prestock cell differentiation in that organism.

The structurally related proteins of the TGF-β superfamily of proteins also have been identified as involved in a variety of developmental events. For example, TGF-β and the polypeptides of the inhibin/activin group appear to play a role in the regulation of cell growth and differentiation. MIS (Mullerian Inhibiting Substance) causes regression of the Mullerian duct in development of the mammalian male embryo, and DPP, the gene product of the Drosophila decapentaplegic complex is required for appropriate dorsal-ventral specification. Similarly, Vg-1 is involved in mesoderm induction in Xenopus, and Vgr-1 has been identified in a variety of developing murine tissues.

Another source that has revealed a wealth of information is in the area of bone morphogenesis. The development and study of a bone model system has identified the developmental cascade of bone differentiation as consisting of chemotaxis of mesenchymal cells, proliferation of these progenitor cells, differentiation of these cells into chrondroblasts, cartilage calcification, vascular invasion, bone formation, remodeling, and finally, marrow differentiation(Reddi (1981) Collagen Rel. Res. 1:209-206). Proteins capable of inducing endochondral bone formation in a mammal when implanted in association with a matrix now have been identified in a number of different mammalian species, as have the genes encoding these proteins, (see, for example, U.S. Patent No. 4,968,590 and U.S. Patent No. 5,011,691, Ozkaynak, et al., (1990) EMBO J 9:2085-2093, and Ozkaynak et al., (1991) Biochem. Biophys. Res. Commn. 179:116-123. WO-A-89 09788, WO-A-89 09787, WO-A-90 03733, WO-A-91 05802 and WO-A-91 18558. These proteins, which share significant amino acid sequence homology with one another as well as structural similarities with various members of the TGF-β super family of proteins, have been shown to induce endochondral bone formation and/or cartilage formation when implanted, in a mammal in association with a suitably modified matrix. Proteins capable of inducing a similar developmental cascade of tissue morphogenesis of other tissues have not been identified.

It is an object of this invention to provide morphogenic proteins ("morphogens"), and methods for identifying these proteins, which are capable of inducing the developmental cascade of tissue morphogenesis for a variety of tissues in mammals different from bone or cartilage. This morphogenic activity includes the ability to induce proliferation and differentiation of progenitor cells, and the ability to support and maintain the differentiated phenotype through the progression of events that results in the formation of adult tissue. Another object is to provide genes encoding these proteins as well as methods for the expression and isolation of these proteins, from either natural sources or biosynthetic sources, using recombinant DNA techniques. Still another object is to provide tissue-specific acellular matrices that may be used in combination with these proteins, and methods for their production.
Other objects include providing methods for increasing a progenitor cell population in a mammal, methods for stimulating progenitor cells to differentiate in vivo or in vitro and maintain their differentiated phenotype, methods for inducing tissue-specific growth in vivo and methods for the replacement of diseased or damaged tissue in vivo. These and other objects and features of the invention will be apparent from the description, drawings, and claims which follow.

### Summary or the Invention

This invention is defined by the claims appended hereto.

Thus, described herein are morphogenic proteins ("morphogens") capable of inducing the developmental cascade of tissue-specific non skeletal morphogenesis in a mammal. In particular, these proteins are capable of inducing the proliferation of uncommitted progenitor cells, and inducing the differentiation of these stimulated progenitor cells in a tissue-specific manner under appropriate environmental conditions. In addition, the morphogens are capable of supporting the growth and maintenance of these differentiated cells. These morphogenic activities allow the proteins to initiate and maintain the developmental cascade of tissue morphogenesis in an appropriate, morphogenically permissive environment, stimulating stem cells to proliferate and differentiate in a tissue-specific manner, and inducing the progression of events that culminate in new tissue formation. These morphogenic activities also allow the proteins to stimulate the "redifferentiation" of cells previously induced to stray from their differentiation path. Under appropriate environmental conditions it is anticipated that these morphogens also may stimulate the "dedifferentiation" of committed cells (see infra.)

As described herein, the proteins and compositions are useful in the replacement of diseased or damaged tissue in a mammal, particularly when the damaged tissue interferes with normal tissue or organ function. Accoraingly, it is anticipated that the proteins will be useful in the repair of damaged tissue such as, for example, damaged lung tissue resulting from emphysema, cirrhotic kidney or liver tissue, damaged heart or blood vessel tissue, as may result from cardiomyopathies and/or atherothrombotic or cardioembolic strokes, damaged stomach tissue resulting from ulceric perforations or their repair, damaged neural tissue as may result from physical injury, degenerative diseases such as Alzheimer's disease or multiple sclerosis or strokes, damaged dentin tissu as may result from disease or mechanical injury. When the proteins are provided to, or their expression stimulated at, a tissue-specific locus, the developmental cascade of tissue morphogenesis is induced (see infra). Cells stimulated ex vivo by contact with the proteins or agents capable of stimulating morphogen expression in these cells also may be provided to the tissue locus. In these cases the existing tissue provides the necessary matrix requirements, providing a suitable substratum for the proliferating and differentiating cells in a morphogenically permissive environment, as well as providing the necessary signals for directing the tissue-specificity of the developing tissue.
Alternatively, the proteins or stimulated cells may be combined with a formulated matrix and implanted as a device at a locus in vivo. The formulated matrix should be a biocompatible, preferably biodegradable, appropriately modified tissue-specific acellular matrix having the characteristics described below.

In many instances, the loss of tissue function results from scar tissue, formed in response to an initial or repeated injury to the tissue. The degree of scar tissue formation generally depends on the regenerative properties of the injured tissue, and on the degree and type of injury. Thus, described herein are morphogens that may be used to prevent or substantially inhibit the formation of scar tissue by providing the morphogens, or morphogen-stimulated cells, to a newly injured tissue loci (see infra).

The morphogens also may be used to increase or regenerate a progenitor or stem cell population in a mammal. For example, progenitor cells may be isolated from an individual's bone marrow, stimulated ex vivo for a time and at a morphogen concentration sufficient to induce the cells to proliferate, and returned to the bone marrow. Other sources of progenitor cells that may be suitable include biocompatible cells obtained from a cultured cell line, stimulated in culture, and subsequently provided to the body. Alternatively, the morphogen may be provided systemically, or implanted, injected or otherwise provided to a progenitor cell population in an individual to induce its mitogenic activity in vivo. For example, an agent capable of stimulating morphogen expression in the progenitor cell population of interest may be provided to the cells in vivo, for example systemically, to induce mitogenic activity. Similarly, a particular population of hemopoietic stem cells may be increased by the morphogens, for example by perfusing an individual's blood to extract the cells of interest, stimulating these cells ex vivo, and returning the stimulated cells to the blood. It is anticipated that the ability to augment an individual's progenitor cell population will significantly enhance existing methods for treating disorders resulting from a loss or reduction of a renewable cell population. Two particularly significant applications include the treatment of blood disorders and impaired or lost immune function. Other cell populations whose proliferation may be exploited include the stem cells of the epidermis, which may be used in skin tissue regeneration, and the stem cells of the gastrointestinal lining, for example, in the healing of ulcers.

As described herein, the morphogens also may be used to support the growth and maintenance of differentiated cells, inducing existing differentiated cells to continue expressing their phenotype. It is anticipated that this activity will be particularly useful in the treatment of tissue disorders where loss of function is caused by cells becoming senescent or quiescent, such as may occur in osteoporosis. Application of the protein directly to the cells to be treated, or providing it by systemic injection, can be used to stimulate these cells to continue expressing their phenotype, thereby significantly reversing the effects of the dysfunction (see infra). In addition, the morphogens also may be used in gene therapy protocols to stimulate the growth of quiescent cells, thereby potentially enhancing the ability of these cells to incorporate exogenous DNA.

As described herein, the morphogens also may be used to induce "redifferentiation" of cells that have strayed from their differentiation pathway, such as can occur during tumorgenesis. It is anticipated that this activity of the proteins will be particularly useful in treatments to reduce or substantially inhibit the growth of neoplasms. The method also is anticipated to induce the de-and re-differentiation of these cells. As described supra, the proteins may be provided to the cells directly or systemically, or an agent capable of stimulating morphogen expression in vivo may be provided.

Finally, modulations of endogenous morphogen levels may be monitored as part of a method for detecting non-skeletal tissue dysfunction. Specifically, modulations in endogenous morphogen levels are anticipated to reflect changes in tissue or organ stasis. Tissue stasis may be monitored by detecting changes in the levels of the morphogen itself. For example, tissue samples may be obtained at intervals and the concentration of the morphogen present in the tissue detected by standard protein detection means known to those skilled in the art. As an example, a binding protein capable of interacting specifically with the morphogen of interest, such as an anti-morphogen antibody, may be used to detect the morphogen in a standard immunoassay. The morphogen levels detected then may be compared, the changes in the detected levels being indicative of the status of the tissue. Modulations in endogenous morphogen levels also may be monitored by detecting fluctuations in the body's natural antibody titer to morphogens (see infra.)

The morphogenic proteins and compositions described herein can be isolated from a variety of naturally-occurring sources, or they may be constructed biosynthetically using conventional recombinant DNA technology. Similarly, the matrices may be derived from organ-specific tissue, or they may be formulated synthetically, as described below.

A key to these developments was the discovery and characterization of naturally-occurring osteogenic proteins followed by observation of their remarkable properties. These proteins, originally isolated from bone, are capable of inducing the full developmental cascade of bone formation, including vascularization, mineralization, and bone marrow differentiation, when implanted in a mammalian body in association with a suitably modified matrix. Native proteins capable of inducing this developmental cascade, as well as DNA sequences encoding these proteins now have been isolated and characterized for a number of different species (e.g., human and mouse OP-1, OP-2, and CBMP-2. See, for example, U.S. Patent Nos. 4,968,590 and 5,011,691; Sampath et al. (1990) J. Bio. Chem 265:13198-13205; Ozkaynak, et al. (1990) EMBO J 9:2085-2 093 and Ozkaynak, et al. (1991) Biochem. Biophys. Res. Commn. 179:116-123.) The mature forms of these proteins share substantial amino acid sequence homology, especially in the C-terminal regions of the mature proteins. In particular, the proteins share a conserved six or seven cysteine skeleton in this region (e.g., the linear arrangement of these C-terminal cysteine residues is essentially conserved in the different proteins, in addition to other, apparently required amino acids (see Table II, infra)).

Polypeptide chains not normally associated with bone or bone formation, but sharing substantial amino acid sequence homology with the C-terminus of the osieogenic proteins, including the conserved six or seven cysteine skeleton, also have been identified as competent for inducing bone in mammals. Among these are amino acid sequences identified in Drosophila and Xenopus, (e.g., DPP and Vgl; see, for example, U.S. Patent No. 5,011,691 and Table II, infra). In addition, non-native biosynthetic constructs designed based on extrapolation from these sequence homologies, including the conserved six or seven cysteine skeleton, have been shown to induce endochondral bone formation in mammals when implanted in association with an appropriate matrix (see U.S. Pat. No. 5,011,691 and Table III, infra).

It has now been discovered that this "family" of proteins sharing substantial amino acid sequence homology and the conserved six or seven cysteine skeleton are true morphogens, capable of inducing, in addition to bone and cartilage, tissue-specific morphogenesis for a variety of other organs and tissues. The proteins apparently bind to surface receptors or otherwise contact and interact with progenitor cells, predisposing or stimulating the cells to proliferate and differentiate in a morphogenically permissive environment. The morphogens are capable of inducing the developmental cascade of cellular and molecular events that culminate in the formation of new organ-specific tissue, including any vascularization, connective tissue formation, and nerve ennervation as required by the naturally occurring tissue.

The term "morphogen" as used herein, refers to the aforementioned osteogenic proteing and polypeptide chains sharing substantial amino acid sequence, homology with the C-terminus of said osteogenic proteins.

It also has been discovered that the way in which the cells differentiate, whether, for example, they differentiate into bone-producing osteoblasts, hemopoietic cells, or liver cells, depends on the nature of their local environment (see infra). Thus, in addition to requiring a suitable substratum on which to anchor, the proliferating and differentiating cells also require appropriate signals to direct their tissue-specificity. These signals may take the form of cell surface markers.

When the morphogens (or progenitor cells stimulated by these morphogens) are provided at a tissue-specific locus (e.g., by systemic injection or by implantation or injection at a tissue-specific locus), the existing tissue at that locus, whether diseased or damaged, has the capacity of acting as a suitable matrix. Alternatively, a formulated matrix may be externally provided together with the stimulated progenitor cells or morphogen, as may be necessary when the extent of injury sustained by the damaged tissue is large. The matrix should be a biocompatible, suitably modified acellular matrix having dimensions such that it allows the influx, differentiation, and proliferation of migratory progenitor cells, and is capable of providing a morphogenically permissive environment (see infra). The matrix preferably is tissue-specific, and biodegradable.

Formulated matrices may be generated from dehydrated organ-specific tissue, prepared for example, by treating the tissue with solvents to substantially remove the non-structural components from the tissue. Alternatively, the matrix may be formulated synthetically using a biocompatible, preferably in vivo biodegradable, structural polymer such as collagen in association with suitable tissue-specific cell attachment factors. Currently preferred structural polymers comprise tissue-specific collagens. Currently preferred cell attachment factors include glycosaminoglycans and proteoglycans. The matrix further may be treated with an agent or agents to increase the number of pores and micropits on its surfaces, so as to enhance the influx, proliferation and differentiation of migratory progenitor cells from the body of the mammal.

Among the morphogens useful are proteins originally identified as osieogenic proteins, such as the OP-1, OP-2 and CBMP2 proteins, as well as amino acid sequence-related proteins such as DPP (from Drosophila), Vgl (from Xenopus), Vgr-1 (from mouse, see Table II and Seq. ID Nos.5-14), and the recently identified GDF-1 protein (Seq. ID No. 14). The members of this family, which include members of the TGF-β super-family of proteins, share substantial amino acid sequence homology in their C-terminal regions. Table I, below, describes the various morphogens identified to date, including their nomenclature as used herein, and Seq. ID references.

**TABLE I**

| | |
|---|---|
| "OP-1" | Refers generically to the group of morphogenically active proteins expressed from part or all of a DNA sequence encoding OP-1 protein, including allelic and species variants thereof, e.g., human OP-1 ("hOP-1", Seq. ID No. 5, mature |
| | protein amino acid sequence), or mouse OP-1 ("mOP-1", Seq. ID No. 6, mature protein amino acid sequence.) The conserved seven cysteine skeleton is defined by residues 38 to 139 of Seq. ID Nos. 5 and 6. The cDNA sequences and the amino acids encoding the full length proteins are provided in Seq. Id Nos. 16 and 17 (hOP1) and Seq. ID Nos. 18 and 19 (mOP1.) The mature proteins are defined by residues 293-431 (hOP1) and 292-430 (mOP1). The "pro"regions of the proteins, cleaved to yield the mature, morphogenically active proteins are defined essentially by residues 30-292 (hOP1) and residues 30-291 (mOP1). |
| "OP-2" | refers generically to the group of active proteins expressed from part or all of a DNA sequence encoding OP-2 protein, including allelic and species variants thereof, e.g., human OP-2 ("hOP-2", Seq. ID No. 7, mature protein amino acid sequence) or mouse OP-2 ("mOP-2", Seq. ID No. 8, mature protein amino acid sequence). The conserved seven cysteine skeleton is defined by residues 38 to 139 of Seq. ID Nos. 7 and 8. The cDNA sequences and the amino acids encoding the full length proteins are provided in Seq. Id Nos. 20 and 21 (hOP2) and Seq. ID Nos. 22 and 23 (mOP2.) The mature proteins are defined essentially by residues 264-402 (hOP2) and 261-399 (mOP2). The "pro" regions of the proteins, cleaved to yield |
| | the mature, morphogenically active proteins are defined essentially by residues 18-263 (hOP2) and residues 18-260 (mOP1). |
| "CBMP2" | refers generically to the morphogenically active proteins expressed from a DNA sequence encoding the CBMP2 proteins, including allelic and species variants thereof, e.g., human CBMP2A ("CBMP2A(fx)", Seq ID No. 9) or human CBMP2B DNA ("CBMP2B(fx)", Seq. ID No. 10). |
| "DPP(fx)" | refers to protein sequences encoded by the Drosophila DPP gene and defining the conserved seven cysteine skeleton (seq. ID No. 11). |
| "Vgl(fx)" | refers to protein sequences encoded by the Xenopus Vgl gene and defining the conserved seven cysteine skeleton (Seq. ID No. 12). |
| "Vgr-1(fx)" | refers to protein sequences encoded by the murine Vgr-1 gene and defining the conserved seven cysteine skeleton (Seq. ID No. 13). |
| "GDF-1(fx)" | refers to protein sequences encoded by the human GDF-1 gene and defining the conserved seven cysteine skeleton (seq. ID No. 14). |

The OP-2 proteins have an additional cysteine residue in this region (e.g., see residue 41 of Seq. ID Nos. 7 and 8), in addition to the conserved cysteine skeleton in common with the other proteins in this family. The GDF-1 protein has a four amino acid insert within the conserved skeleton (residues 44-47 of Seq. ID No. 14) but this insert likely does not interfere with the relationship of the cysteines in the folded structure. In addition, the CBMP2 proteins are missing one amino acid residue within the cysteine skeleton.

The morphogens are inactive when reduced, but are active as oxidized homodimers and when oxidized in combination with other morphogens of this invention. Thus, as defined herein, a morphogen of this invention is a dimeric protein comprising a pair of polypeptide chains, wherein each polypeptide chain comprises at least the C-terminal six cysteine skeleton defined by residues 43-139 of Seq. ID No. 5, including functionally equivalent arrangements of these cysteines (e.g., amino acid insertions or deletions which alter the linear arrangement of the cysteines in the sequence but not their relationship in the folded structure), such that, when the polypeptide chains are folded, the dimeric protein species comprising the pair of polypeptide chains has the appropriate three-dimensional structure, including the appropriate intra-or inter-chain disulfide bonds such that the protein is capable of acting as a morphogen as defined herein. Specifically, the protein is capable of all of the following biological functions in a morphogenically permissive environment: stimulating proliferation of progenitor cells; stimulating the differentiation of progenitor cells; stimulating the proliferation of differentiated cells; and supporting the growth and maintenance of differentiated cells, including the "redifferentiation" of these cells. In addition, it is also anticipated that the morphogens will be capable of inducing dedifferentiation of committed cells under appropriate environmental conditions.

Preferably, the morphogens comprise one of two species of generic amino acid sequences: Generic Sequence 1 (Seq. ID No. 1) or Generic Sequence 2 (Seq. ID No. 2); where each Xaa indicates one of the 20 naturally-occurring L-isomer, α-amino acids or a derivative thereof. Generic Sequence 1 comprises the conserved six cysteine skeleton and Generic Sequence 2 comprises the conserved six cysteine skeleton plus the additional cysteine identified in OP-2 (see residue 36, Seq. ID No. 2). In another preferred aspect, these sequences further comprise the following additional sequence at their N-terminus:

Preferred amino acid sequences within the foregoing generic sequences include: Generic Sequence 3 (Seq. ID No. 3) and Generic Sequence 4 (Seq. ID No. 4), listed below, which accommodate the homologies shared among the various preferred members of this morphogen family identified to date (see Table II), as well as the amino acid sequence variation among them. Generic Sequences 3 and 4 are composite amino acid sequences of the proteins presented in Table II and identified in Seq. ID Nos. 5-14. The generic secuences include both the amino acid identity shared by the sequences in Table II, as well as alternative residues for the variable positions within the sequence. Note that these generic sequences allow for an additional cysteine at position 41 or 46 in Generic Sequences 3 or 4, respectively, providing an appropriate cysteine skeleton where inter- or intramolecular disulfide bonds can form, and contain certain critical amino acids which influence the tertiary structure of the proteins. wherein each Xaa is independently selected from a group of one or more specified amino acids defined as follows: "Res." means "residue" and Xaa at res.4 = (Ser, Asp or Glu); Xaa at res.6 = (Arg, Gln, Ser or Lys); Xaa at res.7 = (Asp or Glu); Xaa at res.8 = (Leu or Val); Xaa at res.11 = (Gln, Leu, Asp, His or Asn); Xaa at res.12 = (Asp, Arg or Asn); Xaa at res.14 = (Ile or Val); Xaa at res.15 = (Ile or Val); Xaa at res.18 = (Glu, Gln, Leu, Lys, Pro or Arg); Xaa at res.20 = (Tyr or Phe); Xaa at res.21 = (Ala, Ser, Asp, Met, His, Leu or Gln); Xaa at res.23 = (Tyr, Asn or Phe); Xaa at res.26 = (Glu, His, Tyr, Asp or Gln); Xaa at res.28 = (Glu, Lys, Asp or Gln); Xaa at res.30 = (Ala, Ser, Pro or Gln); Xaa at res.31 = (Phe, Leu or Tyr); Xaa at res.33 = (Leu or Val); Xaa at res.34 = (Asn, Asp, Ala or Thr); Xaa at res.35 = (Ser, Asp, Glu, Leu or Ala); Xaa at res.36 = (Tyr, Cys, His, Ser or Ile); Xaa at res.37 = (Met, Phe, Gly or Leu); Xaa at res.38 = (Asn or Ser); Xaa at res.39 = (Ala, Ser or Gly); Xaa at res.40 = (Thr, Leu or Ser); Xaa at res.44 = (Ile or Val); Xaa at res.45 = (Val or Leu); Xaa at res.46 = (Gln or Arg); Xaa at res.47 = (Thr, Ala or Ser); Xaa at res.49 = (Val or Met); Xaa at res.50 = (His or Asn); Xaa at res.51 = (Phe, Leu, Asn, Ser, Ala or Val); Xaa at res.52 = (Ile, Met, Asn, Ala or Val); Xaa at res.53 = (Asn, Lys, Ala or Glu); Xaa at res.54 = (Pro or Ser); Xaa at res.55 = (Glu, Asp, Asn, or Gly); Xaa at res.56 = (Thr, Ala, Val, Lys, Asp, Tyr, Ser or Ala); Xaa at res.57 = (Val, Ala or Ile); Xaa at res.58 = (Pro or Asp); Xaa at res.59 = (Lys or Leu); Xaa at res.60 = (Pro or Ala); Xaa at res.63 = (Ala or Val); Xaa at res.65 = (Thr or Ala); Xaa at res.66 = (Gln, Lys, Arg or Glu); Xaa at res.67 = (Leu, Met or Val); Xaa at res.68 = (Asn, Ser or Asp); Xaa at res.69 = (Ala, Pro or Ser); Xaa at res.70 = (Ile, Thr or Val); Xaa at res.71 = (Ser or Ala); Xaa at res.72 = (Val or Met); Xaa at res.74 = (Tyr or Phe); Xaa at res.75 = (Phe, Tyr or Leu); Xaa at res.76 = (Asp or Asn); Xaa at res.77 = (Asp, Glu, Asn or Ser); Xaa at res.78 = (Ser, Gln, Asn or Tyr); Xaa at res.79 = (Ser, Asn, Asp or Glu); Xaa at res.80 = (Asn, Thr or Lys); Xaa at res.82 = (Ile or Val); Xaa at res.84 = (Lys or Arg); Xaa at res.85 = (Lys, Asn, Gln or His); Xaa at res.86 = (Tyr or His); Xaa at res.87 = (Arg, Gln or Glu); Xaa at res.88 = (Asn, Glu or Asp); Xaa at res.90 = (Val, Thr or Ala); Xaa at res.92 = (Arg, Lys, Val, Asp or Glu); Xaa at res.93 = (Ala, Gly or Glu); and Xaa at res.97 = (His or Arg); and Generic Seq. 4: wherein each Xaa is independently selected from a group of one or more specified amino acids as defined by the following: "Res." means "residue" and Xaa at res.2 = (Lys or Arg); Xaa at res.3 = (Lys or Arg); Xaa at res.4 = (His or Arg); Xaa at res.5 = (Glu, Ser, His, Gly, Arg or Pro); Xaa at res.9 = (Ser, Asp or Glu); Xaa at res.11 = (Arg, Gln, Ser or Lys); Xaa at res.12 = (Asp or Glu); Xaa at res.13 = (Leu or Val); Xaa at res.16 = (Gln, Leu, Asp, His or Asn); Xaa at res.17 = (Asp, Arg, or Asn); Xaa at res.19 = (Ile or Val); Xaa at res.20 = (Ile or Val); Xaa at res.23 = (Glu, Gln, Leu, Lys, Pro or Arg); Xaa at res.25 = (Tyr or Phe); Xaa at res.26 = (Ala, Ser, Asp, Met, His, Leu, or Gln); Xaa at res.28 = (Tyr, Asn or Phe); Xaa at res.31 = (Glu, His, Tyr, Asp or Gln); Xaa at res.33 = Glu, Lys, Asp or Gln); Xaa at res.35 = (Ala, Ser or Pro); Xaa at res.36 = (Phe, Leu or Tyr); Xaa at res.38 = (Leu or Val); Xaa at res.39 = (Asn, Asp, Ala or Thr); Xaa at res.40 = (Ser, Asp, Glu, Leu or Ala); Xaa at res.41 = (Tyr, Cys, His, Ser or Ile) ; Xaa at res.42 = (Met, Phe, Gly or Leu); Xaa at res.44 = (Ala, Ser or Gly); Xaa at res.45 = (Thr, Leu or Ser); Xaa at res.49 = (Ile or Val); Xaa at res.50 = (Val or Leu); Xaa at res.51 = (Gln or Arg); Xaa at res.52 = (Thr, Ala or Ser); Xaa at res.54 = (Val or Met); Xaa at res.55 = (His or Asn); Xaa at res.56 = (Phe, Leu, Asn, Ser, Ala or Val); Xaa at res.57 = (Ile, Met, Asn, Ala or Val); Xaa at res.58 = (Asn, Lys, Ala or Glu); Xaa at res.59 = (Pro or Ser); Xaa at res.60 = (Glu, Asp, or Gly); Xaa at res.61 = (Thr, Ala, Val, Lys, Asp, Tyr, Ser or Ala); Xaa at res.62 = (Val, Ala or Ile); Xaa at res.63 = (Pro or Asp); Xaa at res.64 = (Lys or Leu); Xaa at res.65 = (Pro or Ala); Xaa at res.68 = (Ala or Val); Xaa at res.70 = (Thr or Ala); Xaa at res.71 = (Gln, Lys, Arg or Glu); Xaa at res. 72 = (Leu, Met or Val); Xaa at res.73 = (Asn, Ser or Asp); Xaa at res.74 = (Ala, Pro or Ser); Xaa at res.75 = (Ile, Thr or Val); Xaa at res.76 = (Ser or Ala); Xaa at res.77 = (Val or Met); Xaa at res.79 = (Tyr or Phe); Xaa at·res.80 = (Phe, Tyr or Leu); Xaa at res.81 = (Asp or Asn); Xaa at res.82 = (Asp, Glu, Asn or Ser); Xaa at res.83 = (Ser, Gln, Asn or Tyr); Xaa at res.84 = (Ser, Asn, Asp or Glu); Xaa at res.85 = (Asn, Thr or Lys); Xaa at res.87 = (Ile or Val); Xaa at res.89 = (Lys or Arg); Xaa at res.90 = (Lys, Asn, Gln or His); Xaa at res.91 = (Tyr or His); Xaa at res.92 = (Arg, Gln or Glu); Xaa at res.93 = (Asn, Glu or Asp); Xaa at res.95 = (Val, Thr or Ala); Xaa at res.97 = (Arg, Lys, Val, Asp or Glu); Xaa at res.98 = (Ala, Gly or Glu); and Xaa at res.102 = (His or Arg).

Particularly useful sequences for use as morphogens include the C-terminal domains, e.g., the C-terminal 96-102 amino acid residues of Vgl, Vgr-1, DPP, OF-1, OP-2, CBMP-2A, CBMP-2B and GDF-1 (see Table II, infra, and Seq. ID Nos. 5-14) which include at least the conserved six or seven cysteine skeleton. In addition, biosynthetic constructs designed from the generic sequences, such as COP-1, 3-5, 7, 16 (see Table III, infra) also are useful. Other sequences include the C-terminal CBMP3 and the inhibins/activin proteins (see, for example, U.S. Pat. Nos. 4,968,590 and 5,011,691). Accordingly, other useful sequences are those sharing at least 70% amino acid sequence homology, and preferably 80% homology with any of the sequences above. These are anticipated to include allelic and species variants and mutants, and biosynthetic muteins, as well as novel members of this morphogenic family of proteins. Particularly envisioned in the family of related proteins are those proteins exhibiting morphogenic activity and wherein the amino acid changes from the preferred sequences include conservative changes, e.g., those as defined by Dayoff et al., Atlas of Protein Seguence and Structure; vol. 5, Suppl. 3, pp. 345-362, (M.O. Dayoff, ed., Nat'l BioMed. Research Fdn., Washington, D.C. 1979).

The currently most preferred protein sequences useful as morphogens include those having greater than 60% identity, preferably greater than 65% identity, with the amino acid sequence defining the conserved six cysteine skeleton of hOP1 (e.g., residues 43-139 of Seq. ID No. 5). These most preferred sequences include both allelic and species variants of the OP1 and OP2 proteins.

Thus, described herein are proteins comprising any of the polypeptide chains described above, whether isolated from naturally-occurring sources, or produced by recombinant DNA techniques, and includes allelic and species variants of these proteins, naturally-occurring or biosynthetic mutants thereof, as well as various truncated and fusion constructs. Deletion or addition mutants also are envisioned to be active (see infra), including those which may alter the conserved C-terminal cysteine skeleton, provided that the alteration does not functionally disrupt the relationship of these cysteines in the folded structure. Accordingly, such active forms are considered the equivalent of the specifically described constructs disclosed herein. The proteins may include forms having varying glycosylation patterns, varying N-termini, a family of related proteins having regions of amino acid sequence homology, and active truncated or mutated forms of native or biosynthetic proteins, produced by expression of recombinant DNA in host cells.

The morphogenic proteins can be expressed from intact or truncated cDNA or from synthetic DNAs in procaryotic or eucaryotic host cells, and purified, cleaved, refolded, and dimerized to form morphogenically active compositions. Currently preferred host cells include E. coli or mammalian cells, such as CHO, COS or BSC cells.

Thus, in view of this disclosure, skilled genetic engineers can isolate genes from cDNA or genomic libraries of various different species which encode appropriate amino acid sequences, or construct DNAs from oligonucleotides, and then can express them in various types of host cells, including both procaryotes and eucaryotes, to produce large quantities of active proteins capable of inducing tissue-specific cell differentiation and tissue morphogenesis in a variety of mammals including humans.

A further utility described herein comprises inducing tissue-specific morphogenesis using the morphogenic proteins and pharmaceutical and therapeutic agents comprising the morphogens. Also defined (herein is) the use of these morphogens in the manufacture of pharmaceuticals for various medical procedures, including procedures for inducing tissue growth, procedures for inducing progenitor cell proliferation, procedures to inhibit neoplasm growth and procedures to promote phenotypic cell expression of differentiated cells.

### Brief Description of the Drawings

The foregoing and other objects and features of this invention, as well as the invention itself, may be more fully understood from the following description, when read together with the accompanying drawings, in which:
FIGURE 1 is a photomicrograph of a Northern Blot identifying Vgr-1 specific transcripts in various adult murine tissues;
FIGURE 2 is a photomicrograph of a Northern Blot identifying mOP-1-specific mRNA expression in various murine tissues prepared from 2 week old mice (panel A) and 5 week old mice (Panel B);
FIGURE 3 is a photomicrograph of Northern Blots identifying mRNA expression of EF-Tu (A, control), mOP-1 (B, D), and Vgr-1 (C) in (1) 17-day embryos and (2) 3-day post natal mice;
FIGURE 4A and 4B are photomicrographs showing the presence of OP-1 (by immunofluorescence staining) in the cerebral cortex (A) and spinal cord (B);
FIGURE 5A and 5B are photomicrographs illustrating the ability of morphogen (OP-1) to induce undifferentiated NG108 calls (5A) to undergo differentiation of neural morphology (5B).
FIGURE 6A-6D are photomicrographs showing the effect of morphogen (OP-1) on human embryo carcinoma cell redifferentiation;
FIGURE 7 is a photomicrograph showing the effects of phosphate buffered saline (PBS, animal 1) or morphogen (OP-1, animal 2) on partially hepatectomized rats;
FIGURE 8A - 8C are photomicrographs showing the effect of no treatment (8A), carrier matrix treatment (8B) and morphogen treatment (OP-1,8C) on dentin regeneration.

### Detailed Description

Purification protocols first were developed which enabled isolation of the osteogenic (bone inductive) protein present in crude protein extracts from mammalian bone. (See WO-A-89 09787, and U.S. 4,968,590.) The development of the procedure, coupled with the availability of fresh calf bone, enabled isolation of substantially pure bovine osteogenic protein (BOP). BOP was characterized significantly; its ability to induce cartilage and ultimately endochondral bone growth in cat, rabbit, and rat were demonstrated and studied; it was shown to be able to induce the full developmental cascade of bone formation previously ascribed to unknown protein or proteins in heterogeneous bone extracts. This dose dependent and highly specific activity was present whether or not the protein was glycosylated (see U.S. Patent No. 4, 968, 958, filed 4/8/88 and Sampath et al., (1990) J. Biol. Chem. 265: pp. 13198-13205). Sequence data obtained from the bovine materials suggested probe designs which were used to isolate genes encoding osteogenic proteins from different species. Human and murine osteogenic protein counterparts have now been identified and characterized (see, for example, U.S. Pat. No. 5,011,691, Ozkaynak, et al., (1990) EMBO J 9:2085-2093, and Ozkaynak et al., (1991) Biochem. Biochys. Res. Commn. 179:116-123, the disciosures of which are herein incorporated by reference.)

Sequence data from the bovine materials also suggested substantial homology with a number of proteins known in the art which were not known to play a role in bone formation. Bone formation assays performed with these proteins showed that, when these proteins were implanted in a mammal in association with a suitable matrix, cartilage and endochondral bone formation was induced (see, for example, U.S. Patent No. 5,011,691.) One of these proteins is DPP, a Drosophila protein known to play a role in dorsal-ventral specification and required for the correct morphogenesis of the imaginal discs. Two other proteins are related sequences identified in Xenopus and mouse (Vgl and Vgr-1, respectively), thought to play a role in the control of growth and differentiation during embryogenesis. While DPP and Vgr-1 (or Vgr-1-like) transcripts have been identified in a variety of tissues (embryonic, neonatal and adult, Lyons et al., (1989) PNAS 86:4554-4 558, and see infra), Vgl transcripts, which are maternally inherited and spacially restricted to the vegetal endoderm, decline dramatically after gastrulation.

From these homologies a generic consensus sequence was derived which encompasses the active sequence required for inducing bone morphogenesis in a mammal when implanted in association with a matrix. The generic sequence has at least a conserved six cysteine skeleton (Generic Sequence 1, Seq. ID No. 1) or, optionally, a 7-cysteine skeleton (Generic Sequence 2, Seq. ID No. 2), which includes the conserved six cysteine skeleton defined by Generic Sequence 1, and an additional cysteine at residue 36, accomodating the additional cysteine residue identified in the OP2 proteins. Each "Xaa" in the generic sequences indicates that any one of the 20 naturally-occurring L-isomer, ∝-amino acids or a derivative thereof may be used at that position. Longer generic sequences which also are useful further comprise the following sequence at their N-termini:

Biosynthetic constructs designed from this generic consensus sequence also have been shown to induce cartilage and/or endochondral bone formation (e.g., COP-1, COP-3, COP-4, COP-5, COP-7 and COP-16, described in U.S. Patent No. 5,011,691 and presented below in Table III.) Table II, set forth below, compares the amino acid sequences of the active regions of native proteins that have been identified as morphogens, including human OP-1 (hOP-1, Seq. ID Nos. 5 and 16-17), mouse OP-1 (mOP-1, Seq. ID Nos. 6 and 18-19), human and mouse OP-2 (Seq. ID Nos. 7, 8, and 20-22), CBMP2A (Seq. ID No. 9), CBMP2B (Seq. ID No. 10), DPP (from Drosophila, Seq. ID No. 11), Vgl, (from Xenopus, Seq. ID No. 12), Vgr-1 (from mouse, Seq. ID No. 13), and GDF-1 (Seq. ID No. 14.) In the table, three dots indicates that the amino acid in that position is the same as the amino acid in hOP-1. Three dashes indicates that no amino acid is present in that position, and are included for purposes of illustrating homologies. For example, amino acid residue 60 of CBMP-2A and CBMP-2B is "missing". Of course, both these amino acid sequences in this region comprise Asn-Ser (residues 58, 59), with CBMP-2A then comprising Lys and Ile, whereas CBMP-2B comprises Ser and Ile.

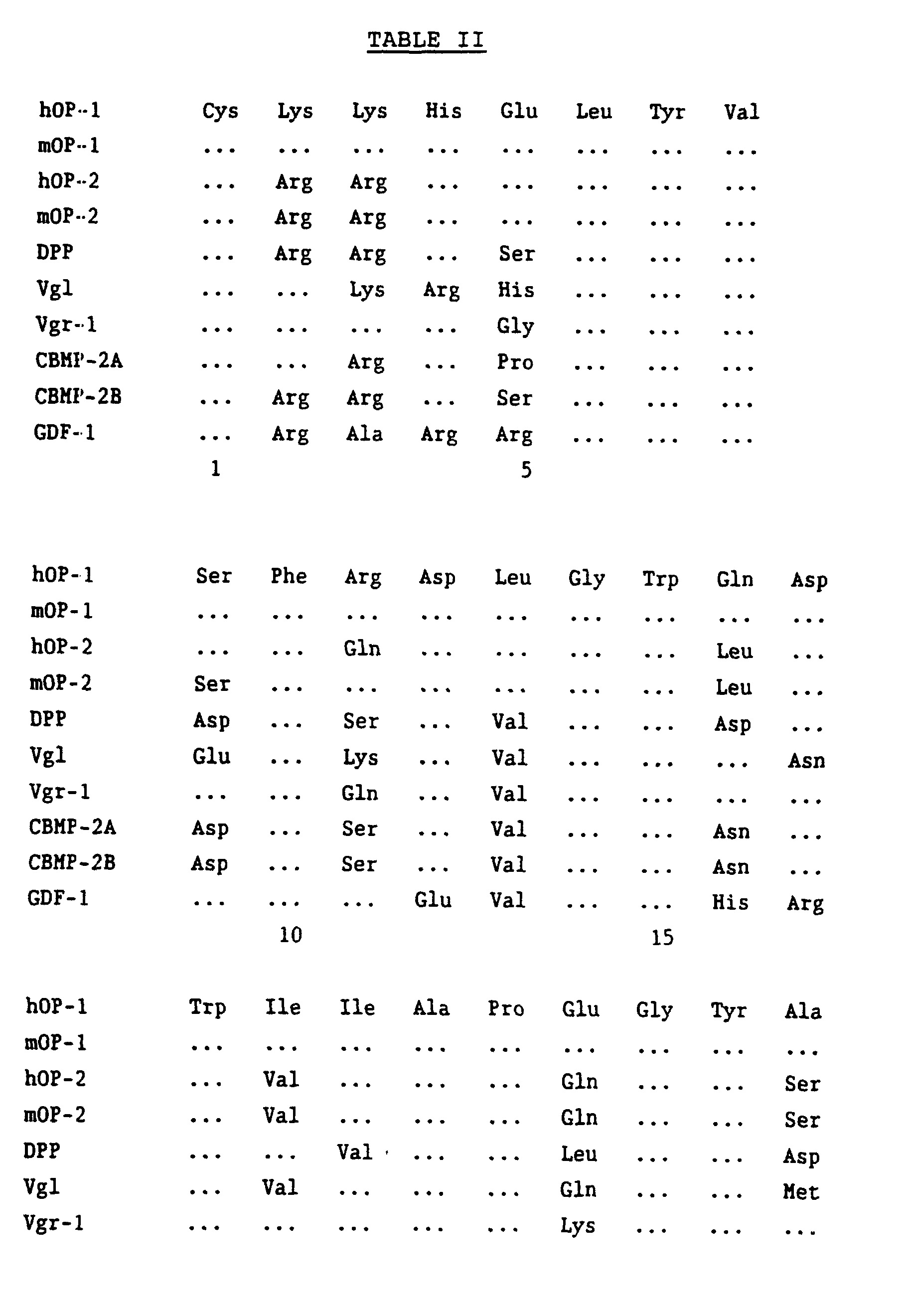

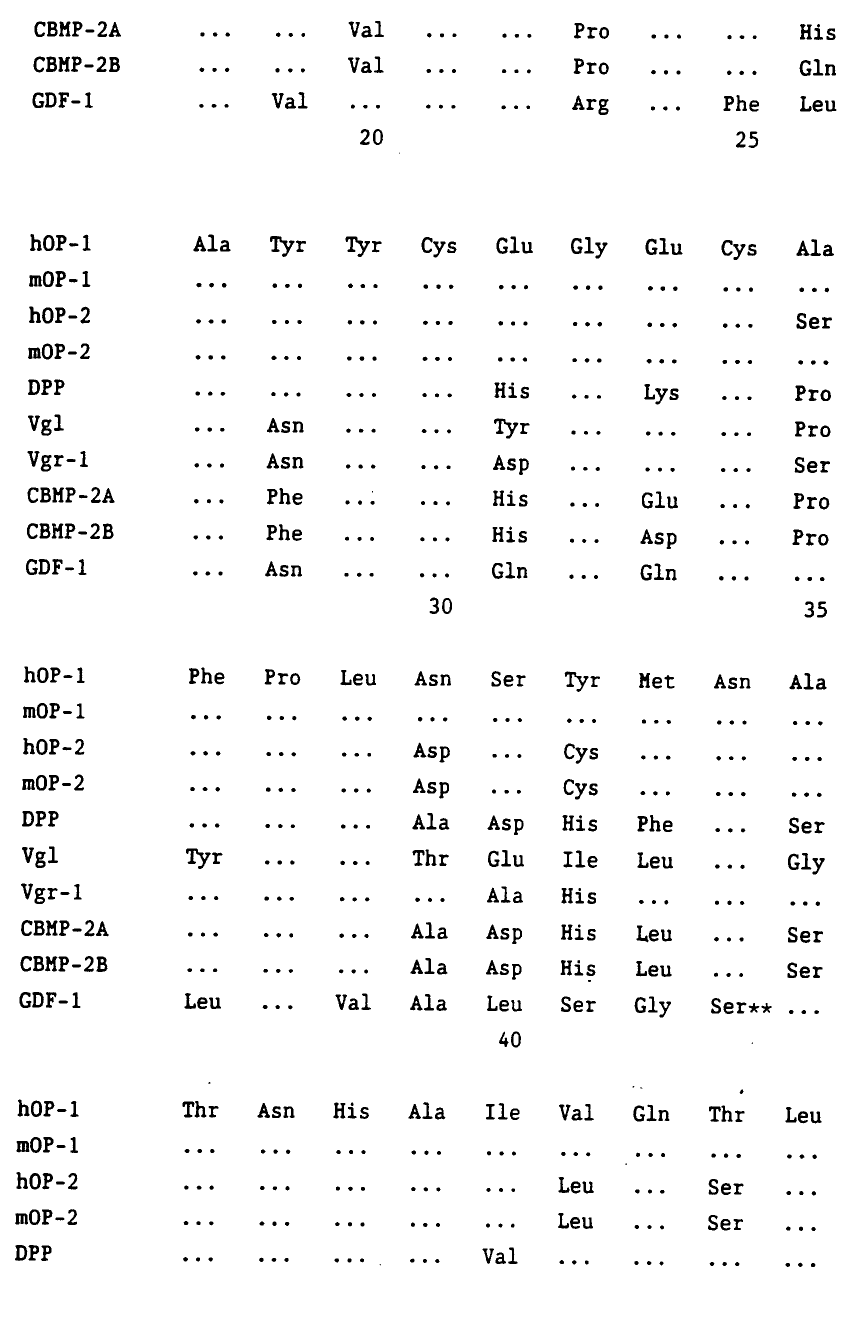

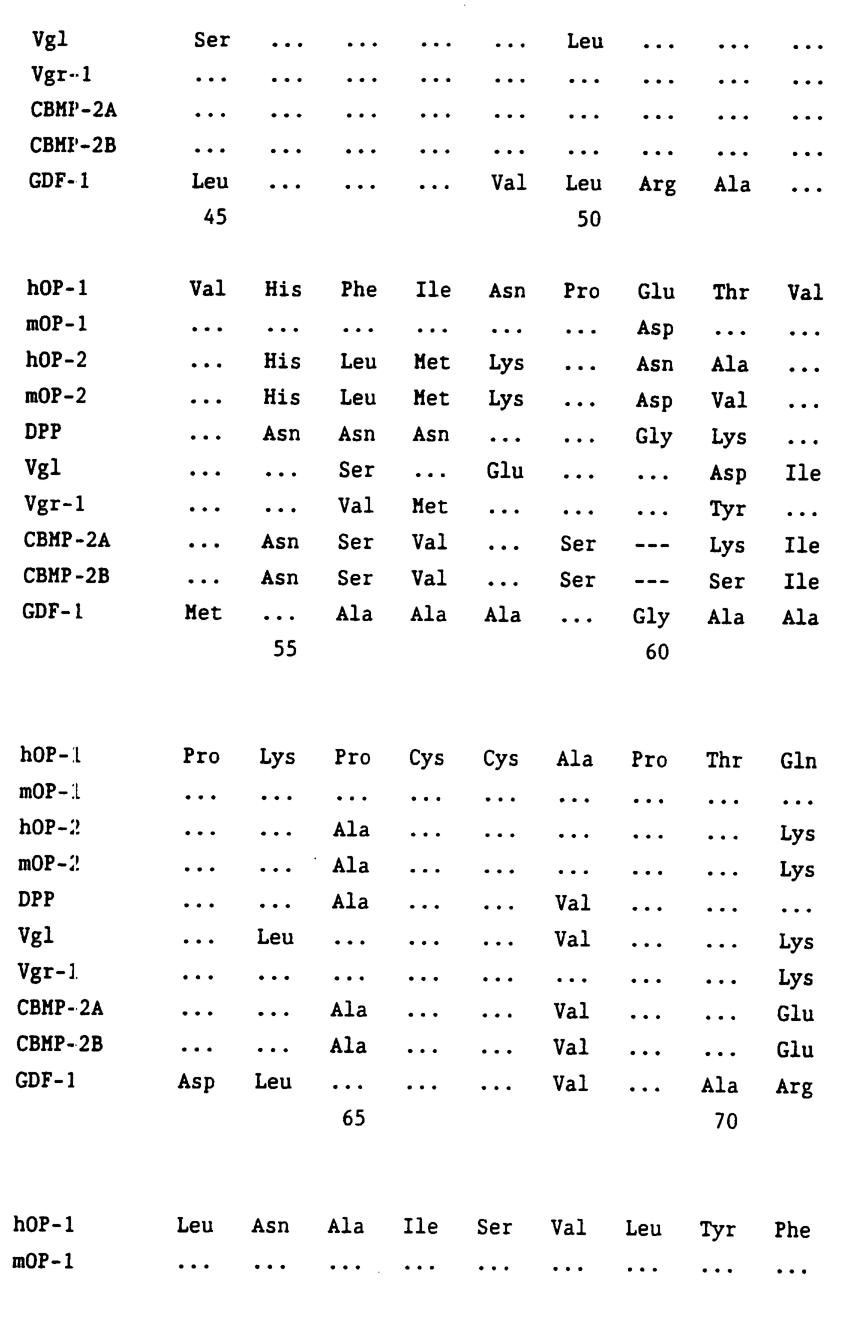

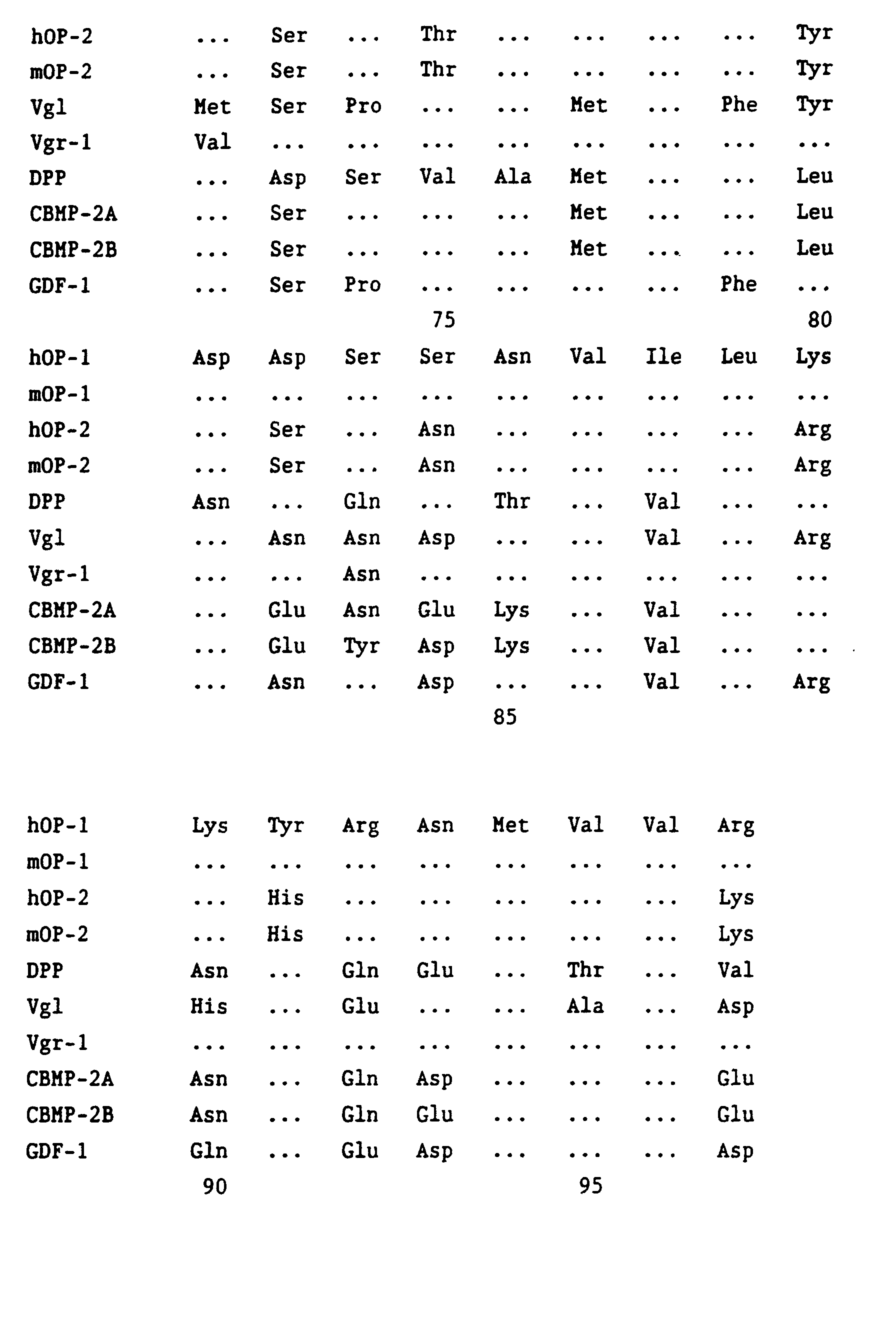

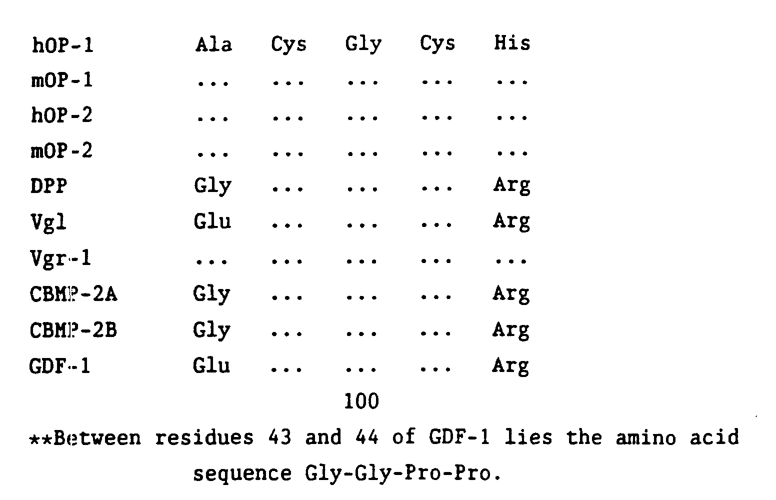

Table III, set forth below, compares the amino acid sequence data for six related biosynthetic constructs designated COPs 1, 3, 4, 5, 7, and 16. These sequences also are presented in U.S. Pat. No. 5,011,691. As with Table II, the dots mean that in that position there is an identical amino acid to that of COP-1, and dashes mean that the COP-1 amino acid is missing at that position.

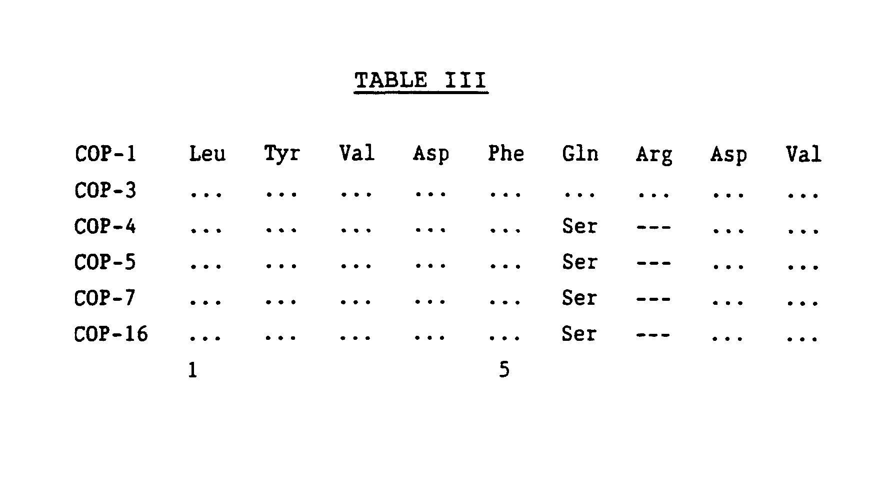

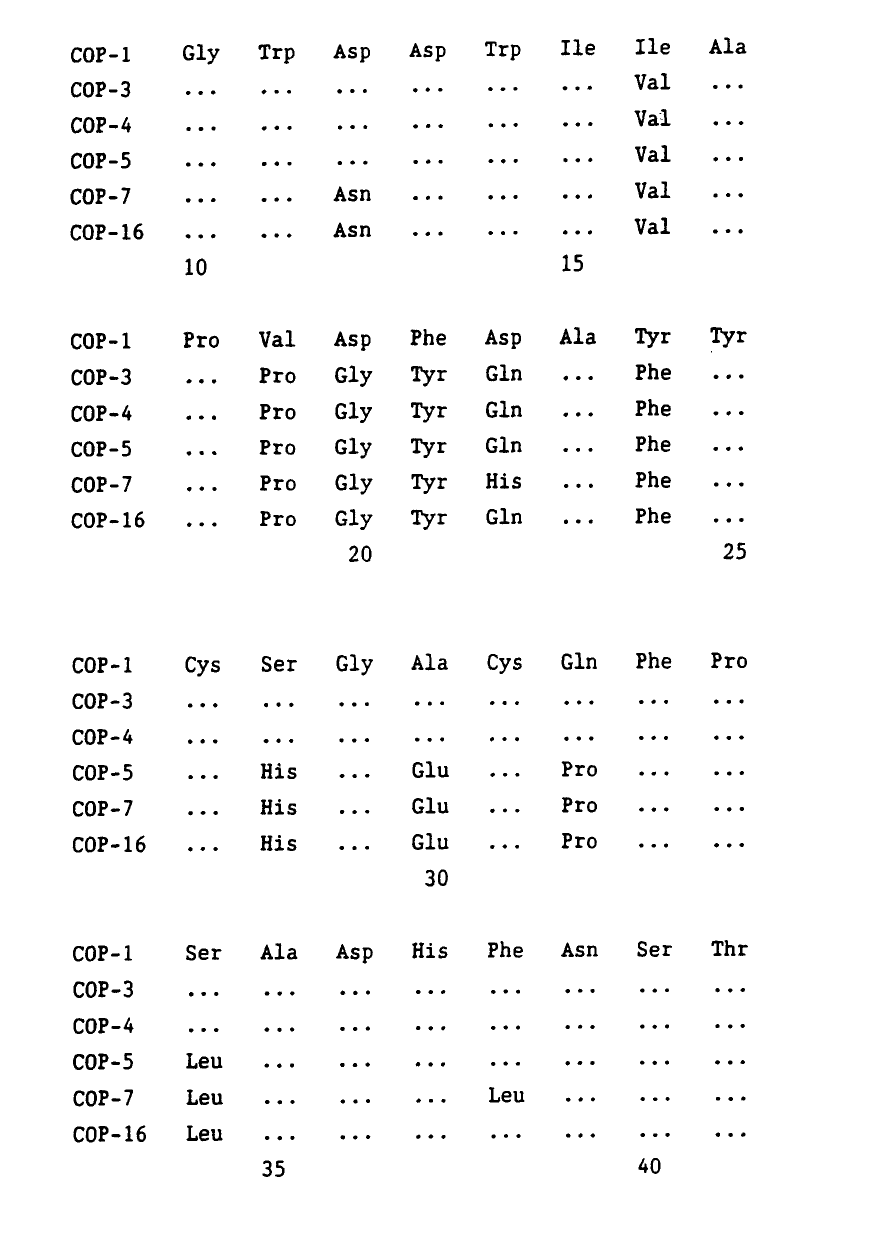

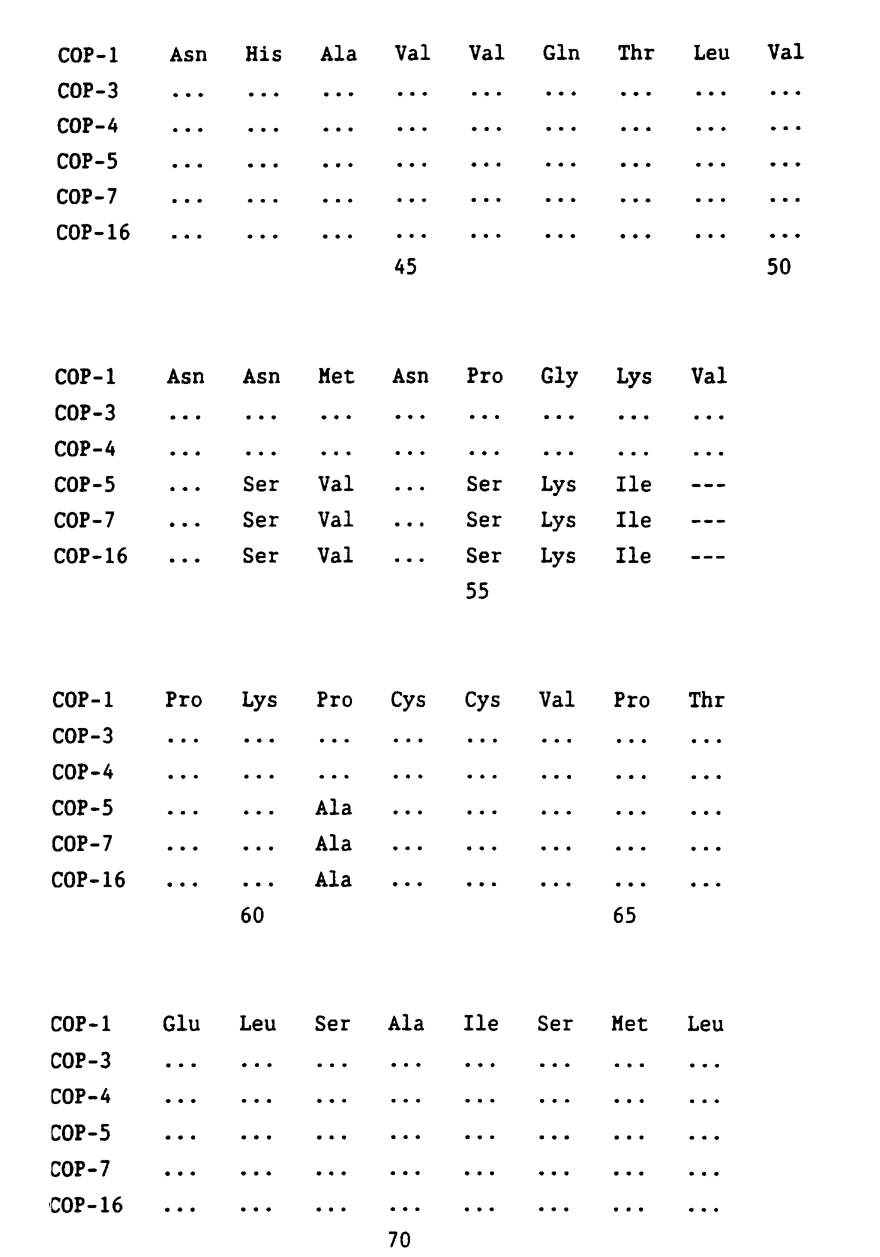

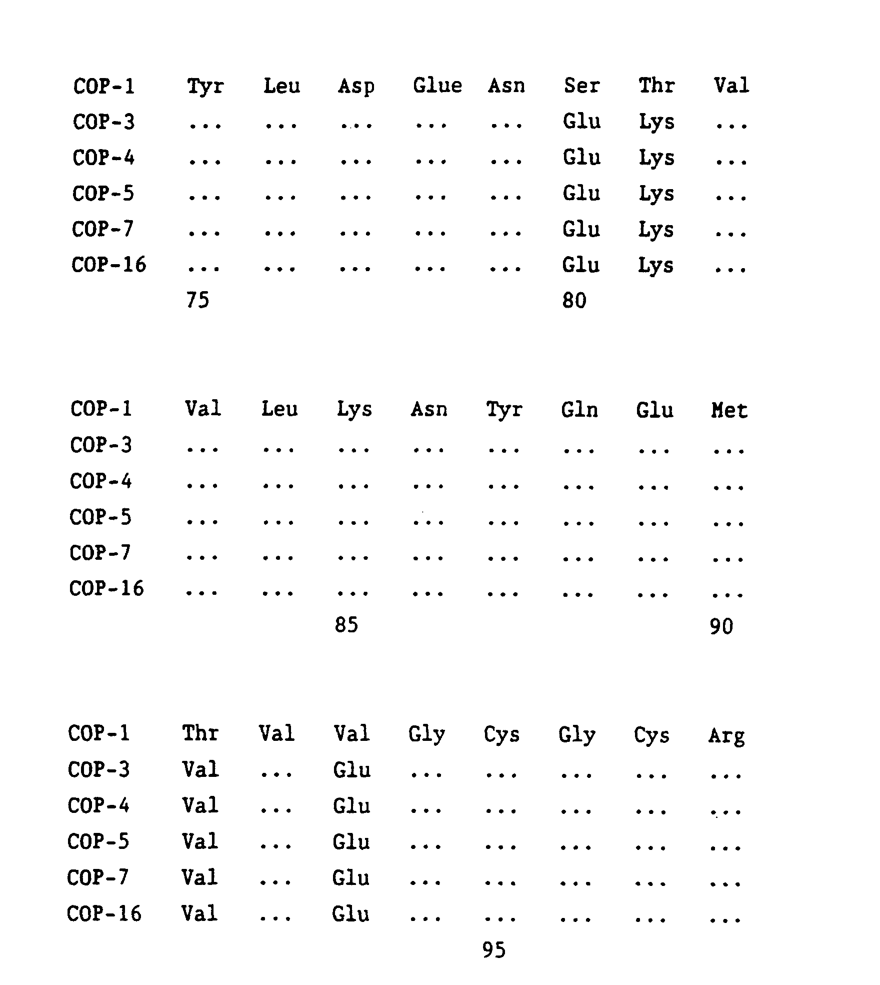

As is apparent from the foregoing amino acid sequence comparisons, significant amino acid changes can be made within the generic sequences while retaining the morphogenic activity. For example, while the GDF-1 protein sequence depicted in Table II shares only about 50% amino acid identity with the hOP1 sequence described therein, the GDF-1 sequence shares greater than 70% amino acid sequence homology with the hOP1 sequence, where homology is defined by allowed conservative amino acid changes within the sequence as defined by Dayoff, et al., Atlas of Protein Sequence and Structure vol.5, supp.3, pp.345-362, (M.O. Dayoff, ed., Nat'1 BioMed. Res. Fd'n, Washington D.C. 1979.)

It now has been discovered that the family of proteins described by these sequences also is capable of initiating and maintaining the tissue-specific developmental cascade in tissues other than bone and cartilage. When combined with naive progenitor cells as disclosed herein, these proteins, termed morphogens, are capable of inducing the proliferation and differentiation of the progenitor cells. In the presence of appropriate tissue-specific signals to direct the differentiation of these cells, and a morphogenically permissive environment, these morphogens are capable of reproducing the cascade of cellular and molecular events that occur during embryonic development to yield functional tissue.

A key to these developments was the creation of a mammalian tissue model system, namely a model system for endochondral bone formation, and investigation of the cascade of events important for bone tissue morphogenesis. Work on this system has enabled discovery not only of bone inductive morphogens, but also of tissue inductive morphogens and their activities. The methods used to develop the bone model system, now well known in the art, along with the proteins of this invention, can be used to create model systems for other tissues, such as liver (see infra).

Using the model system for endochondral bone formation, it also has been discovered that the local environment in which the morphogenic material is placed is important for tissue morphogenesis. As used herein, "local environment" is understood to include the tissue structural matrix and the environment surrounding the tissue. For example, in addition to needing an appropriate anchoring substratum for their proliferation, the morphogen-stimulated cells need signals to direct the tissue-specificity of their differentiation. These signals vary for the different tissues and may include cell surface markers. In addition, vascularization of new tissue requires a local environment which supports vascularization.
Using the bone model system as an example, it is known that, under standard assay conditions, implanting osteoinductive morphogens into loose mesenchyme in the absence of a tissue-specifying matrix generally does not result in endochondral bone formation unless very high concentrations of the protein are implanted. By contrast, implanting relatively low concentrations of the morphogen in association with a suitably modified bone-derived matrix results in the formation of fully functional endochondral bone (see, for example, Sampath et al. (1981) PNAS 78:7599-7 603 and U.S. Patent No. 4,975,526). In addition, a synthetic matrix comprised of a structural polymer such as tissue-specific collagen and tissue-specific cell attachment factors such as tissue-specific glycosylaminoglycans, will allow endochondral bone formation (see, for example, PCT publication US91/03603, published December 12, 1991 (WO 91/18558). Finally, if the morphogen and a suitable bone or cartilage-specific matrix (e.g., comprising Type I cartilage) are implanted together in loose mesenchyme, cartilage and endochondral bone formation will result, including the formation of bone marrow and a vascular system. However, if the same composition is provided to a nonvascular environment, such as to cultured cells in vitro or at an cartilage-specific locus, tissue development does not continue beyond cartilage formation (see infra). Similarly, a morphogenic composition containing a cartilage-specific matrix composed of Type 2 collagen is expected to induce formation of non-cartilage tissue in vivo (e.g., hyaline). However, if the composition is provided to a vascular-supporting environment, such as loose mesenchyme, the composition is capable of inducing the differentiation of proliferating progenitor cells into chondrocytes and osteoblasts, resulting in bone formation.

It also has been discovered that tissue morphogenesis requires a morphogenically permissive environment. Clearly, in fully-functioning healthy tissue that is not composed of a permanently renewing cell population, there must exist signals to prevent continued tissue growth. Thus, it is postulated that there exists a control mechanism, such as a feedback control mechanism, which regulates the control of cell growth and differentiation. In fact, it is known that both TGF-β, and MIS are capable of inhibiting cell growth when present at appropriate concentrations. In addition, using the bone model system it can be shown that osteogenic devices comprising a bone-derived carrier (matrix) that has been demineralized and guanidine-extracted to substantially remove the noncollagenous proteins does allow endochondral bone formation when implanted in association with an osteoinductive morphogen. If, however, the bone-derived carrier is not demineralized but rather is washed only in low salt, for example, induction of endochondral bone formation is inhibited, suggesting the presence of one or more inhibiting factors within the carrier.

Another key to these developments was determination of the broad distribution of these morphogens in developing and adult tissue. For example, DPP is expressed in both embryonic and developing Drosophila tissue. Vgl has been identified in Xenopus embryonic tissue. Vgr-1 transcripts have been identified in a variety of murine tissues, including embryonic and developing brain, lung, liver, kidney and calvaria (dermal bone) tissue. Recently, Vgr-1 transcripts also have been identified in adult murine lung, kidney, heart, and brain tissue, with especially high abundance in the lung (see infra).

OP-1 and the CBMP2 proteins, both first identified as bone morphogens, have been identified in mouse and human placenta, hippocampus, calvaria and osteosarcoma tissue as determined by identification of OP-1 and CMBP2-specific sequences in cDNA libraries constructed from these tissues (see Ozkaynak, et al., (1990) EMBO J 9:2085-2093, and Ozkaynak et al., (1991) Biochem. Biophys. Res. Commn. 179:116-123). Additionally, the OP-1 protein is present in a variety of embryonic and developing tissues including kidney, liver, heart, adrenal tissue and brain as determined by Western blot analysis and immunolocalization (see infra). OP-1-specific transcripts also have been identified in both embryonic and developing tissues, most abundantly in developing kidney, bladder and brain (see infra). OP-1 also has been identified as a mesoderm inducing factor present during embryogenesis (see infra). Moreover, OP-1 has been shown to be associated with in satellite muscle cells and associated with pluripotential stem cells in bone marrow following damage to adult murine endochondral bone, indicating its morphogenic role in tissue repair and regeneration. In addition, the recently identified protein GDF-1 (see Table II) has been identified in neural tissue (Lee, (1991) PNAS 88 4250-4254).

### Exemplification

### IDENTIFICATION AND ISOLATION OF MORPHOGENS

Among the proteins useful in this invention are proteins originally identified as bone inductive proteins, such as the OP-1, OP-2 and the CBMP proteins, as well as amino acid sequence-related proteins such as DPP (from Drosophila), Vgl (from Xenopus) and Vgr-1 (from mouse, see Table II and Sequence Listing). The members of this family, which include particular members of the TGF-β super family of structurally related proteins, share substantial amino acid sequence homology in their C-terminal regions. The OP-2 proteins have an extra cysteine residue in this region (position 41 of Seq. ID Nos. 7 and 8), in addition to the conserved cysteine skeleton in common with the other proteins in this family. The proteins are inactive when reduced, but are active as oxidized, homodimeric species as well as when oxidized in combination with other morphogens.

Accordingly, the morphogens of this invention can be described by either of the following two species of generic amino acid sequences: Generic Sequence 1 or Generic Sequence 2, (Seq. ID Nos. 1 and 2), where each Xaa indicates one of the 20 naturally-occurring L-isomer, α-amino acids or a derivative thereof. Particularly useful sequences that fall within this family of proteins include the 96-102 C-terminal residues of Vgl, Vgr-1, DPP, OP-1, OP-2, CBMP-2A, CBMP-2B, and GDF-1, as well as their intact mature amino acid sequences. In addition, biosynthetic constructs designed from the generic sequences, such as COP-1, COP-3-5, COP-7, and COP-16 also are useful (see, for example, U.S. Pat. No. 5,011,691.)

Generic sequences showing preferred amino acids compiled from sequences identified to date and useful as morphogens (e.g., Tables II and III) are. described by Generic Sequence 3 (Seq. ID No. 3) and Generic Sequence 4 (Seq. ID No. 4). Note that these generic sequences have a 7 or 8-cysteine skeleton where inter- or intramolecular disulfide bonds can form, and contain certain critical amino acids which influence the tertiary structure of the proteins. It is also contemplated that the differing N-termini of the naturally occurring proteins provide a tissue-specific or other, important modulating activity of these proteins.

Given the foregoing amino acid and DNA sequence information, the level of skill in the art, and the disclosures of U.S. Patent Nos. 4,968,590 and 5,011,691, PCT application US 89/01469, published October 19, 1989 (WO89/09788), and Ozkaynak, et al., (1990) EMBO J 9:2085-2093, and Ozkaynak et al., (1991) Biochem. Biophys. Res. Commn. 179:116-123, various DNAs can be constructed which encode at least the active region of a morphogen of this invention, and various analogs thereof (including allelic variants and those containing genetically engineered mutations), as well as fusion proteins, truncated forms of the mature proteins, deletion and insertion mutants, and similar constructs. Moreover, DNA hybridization probes can be constructed from fragments of the genes encoding any of these proteins, including seqeunces encoding the active regions or the pro regions of the proteins (see infra), or designed de novo from the generic sequence. These probes then can be used to screen different genomic and cDNA libraries to identify additional morphogenic proteins from different tissues.

The DNAs can be produced by those skilled in the art using well known DNA manipulation techniques involving genomic and cDNA isolation, construction of synthetic DNA from synthesized oligonucleotides, and cassette mutagenesis techniques. 15-100mer oligonucleotides may be synthesized on a Biosearch DNA Model 8600 Synthesizer, and purified by polyacrylamide gel electrophoresis (PAGE) in Tris-Borate-EDTA buffer. The DNA then may be electroeluted from the gel. Overlapping oligomers may be phosphorylated by T4 polynucleotide kinase and ligated into larger blocks which also may be purified by PAGE.

The DNA from appropriately identified clones then can be isolated, subcloned (preferably into an expression vector), and sequenced. Plasmids containing sequences of interest then can be transfected into an appropriate host cell for expression of the morphogen and further characterization. The host may be a procaryotic or eucaryotic cell since the former's inability to glycosylate protein will not destroy the protein's morphogenic activity. Useful host cells include E. coli, Saccharomyces, the insect/baculovirus cell system, myeloma cells, and various other mammalian cells. The vectors additionally may encode various sequences to promote correct expression of the recombinant protein, including transcription promoter and termination sequences, enhancer sequences, preferred ribosome binding site sequences, preferred mRNA leader sequences, preferred signal sequences for protein secretion, and the like.

The DNA sequence encoding the gene of interest also may be manipulated to remove potentially inhibiting sequences or to minimize unwanted secondary and tertiary structure formation. The recombinant morphogen also may be expressed as a fusion protein. After being translated, the protein may be purified from the cells themselves or recovered from the culture medium. All biologically active protein forms comprise dimeric species joined by disulfide bonds or otherwise associated, produced by refolding and oxidizing one or more of the various recombinant polypeptide chains within an appropriate eucaryotic cell or in vitro after expression of individual subunits. A detailed description of morphogens expressed from recombinant DNA in E. coli and in numerous different mammalian cells is disclosed in PCT publication US90/05903, published May 2, 1991 (WO91/05802).

Alternatively, morphogenic polypeptide chains can be synthesized chemically using conventional peptide synthesis techniques well known to those having ordinary skill in the art. For example, the proteins may be synthesized intact or in parts on a Biosearch solid phase peptide synthesizer, using standard operating procedures. Completed chains then are deprotected and purified by HPLC (high pressure liquid chromatography). If the protein is synthesized in parts, the parts may be peptide bonded using standard methodologies to form the intact protein. In general, the manner in which the morphogens are made can be conventional and does not form a part of this invention.

### MORPHOGEN DISTRIBUTION

The generic function of the morphogens of this invention throughout the life of the organism can be evidenced by their expression in a variety of disparate mammalian tissues. Determination of the tissue distribution of morphogens also may be used to identify different morphogens expressed in a given tissue, as well as to identify new, related morphogens. The proteins (or their mRNA transcripts) are readily identified in different tissues using standard methodologies and minor modifications thereof in tissues where expression may be low. For example, protein distribution may be determined using standard Western blot analysis or immunofluorescent techniques, and antibodies specific to the morphogen or morphogens of interest. Similarly, the distribution of morphogen transcripts may be determined using standard Northern hybridization protocols and transcript-specific probes.

Any probe capable of hybridizing specifically to a transcript, and distinguishing the transcript of interest from other, related transcripts may be used. Because the morphogens of this invention share such high sequence homology in their active, C-terminal domains, the tissue distribution of a specific morphogen transcript may best be determined using a probe specific for the pro region of the immature protein and/or the N-terminal region of the mature protein. Another useful sequence is the 3' non-coding region flanking and immediately following the stop codon. These portions of the sequence vary substantially among the morphogens of this invention, and accordingly, are specific for each protein. For example, a particularly useful Vgr-1-specific probe sequence is the PvuII-SacI fragment, a 265 bp fragment encoding both a portion of the untranslated pro region and the N-terminus of the mature sequence (see Lyons et al. (1989) PNAS 86:4554-4558 for a description of the cDNA sequence). Similarly, particularly useful mOP-1-specific probe sequences are the BstX1-BglI fragment, a 0.68 Kb sequence that covers approximately two-thirds of the mOP-1 pro region; a StuI-StuI fragment, a 0.2 Kb sequence immediately upstream of the 7-cysteine domain; and the Ear1-Pst1 fragment, an 0.3 Kb fragment containing a portion of the 3'untranslated sequence (See Seq. ID No. 18, where the pro region is defined essentially by residues 30-291.) Similar approaches may be used, for example, with hOP1 (Seq. ID No. 16) or human or mouse OP2 (Seq. ID Nos. 20 and 22.)

Using these morphogen-specific probes, which may be synthetically engineered or obtained from cloned sequences, morphogen transcripts can be identified in mammalian tissue, using standard methodologies well known to those having ordinary skill in the art. Briefly, total RNA is prepared from various adult murine tissues (e.g., liver, kidney, testis, heart, brain, thymus and stomach) by a standard methodology such as by the method of Chomczyaski et al. ((1987) Anal. Biochem 162:156-159) and described below. Poly (A)+ RNA is prepared by using oligo (dT)-cellulose chromatography (e.g., Type 7, from Pharmacia LKB Biotechnology, Inc.). Poly (A)+ RNA (generally 15 *µ*g) from each tissue is fractionated on a 1% agarose/formaldehyde gel and transferred onto a Nytran membrane (Schleicher & Schuell). Following the transfer, the membrane is baked at 80°C and the RNA is cross-linked under UV light (generally 30 seconds at 1 mW/cm²). Prior to hybridization, the appropriate probe (e.g., the PvuII-SacI Vgr-1 fragment) is denatured by heating. The hybridization is carried out in a lucite cylinder rotating in a roller bottle apparatus at approximately 1 rev/min for approximately 15 hours at 37°C using a hybridization mix of 40% formamide, 5 x Denhardts, 5 x SSPE, and 0.1% SDS. Following hybridization, the non-specific counts are washed off the filters in 0.1 x SSPE, 0.1% SDS at 50°C. Northern blots performed using Vgr-1 probes specific to the variable N terminus of the mature sequence indicate that the Vgr-1 message is approximately 3.5 Kb.

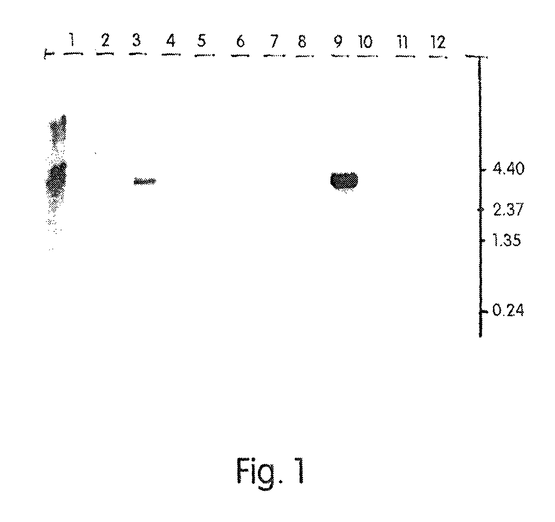

Figure 1 is a photomicrograph representing a Northern blot analysis probing a number of adult murine tissues with the Vgr-1 specific probes: liver, kidney, testis, heart, brain, thymus and stomach, represented in lanes 3-10, respectively. Lanes 1 and 12 are size standards and lanes 2 and 11 are blank. Among the tissues tested, Vgr-1 appears to be expressed most abundantly in adult lung, and to a lesser extent in adult kidney, heart and brain. These results confirm and expand on earlier studies identifying Vgr-1 and Vgr-1-like transcripts in several embryonic and adult murine tissue (Lyons et al. (1989) PNAS 86:4554-4558), as well as studies identifying OP-1 and CBMP2 in various human cDNA libraries (e.g., placenta, hippocampus, calvaria, and osteosarcoma, see Ozkaynak et al., (1990) EMBO 9:2085-2093).

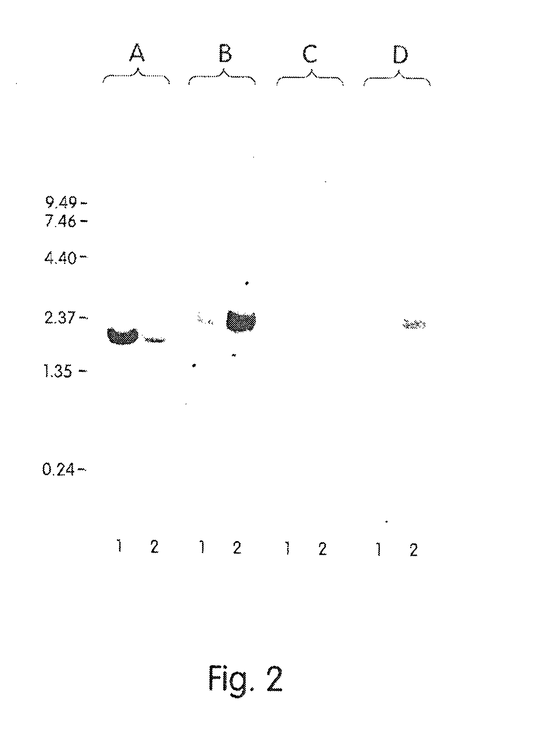

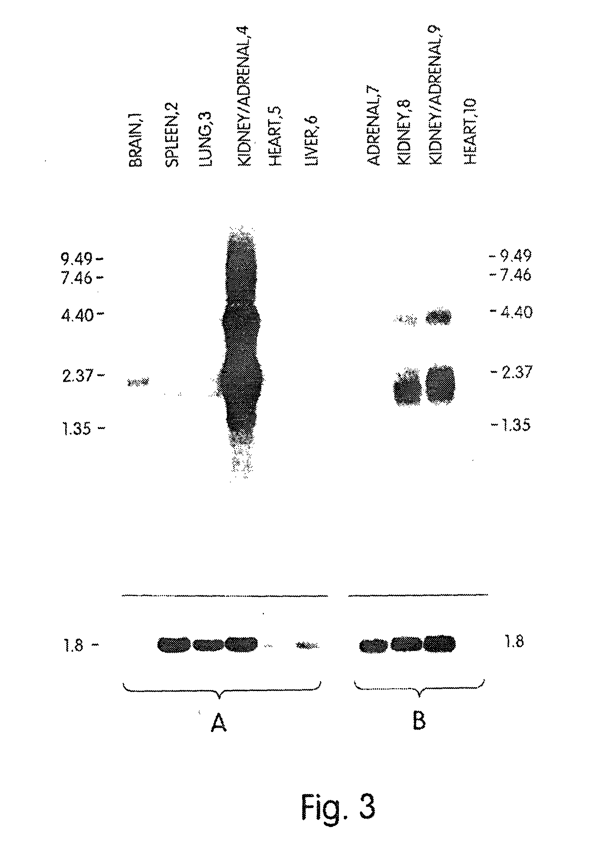

Using the same general probing methodology, mOP-1 transcripts also have been identified in a variety of murine tissues, including embryo and various developing tissues, as can be seen in Figures 2 and 3. Details of the probing methodology are disclosed in Ozkaynak, et al., (1991) Biochem. Biophys. Res. Commn. 179:116-123, the disclosure of which is incorporated herein. The Northern blots represented in Figure 2 probed RNA prepared from developing brain, spleen, lung, kidney (and adrenal gland), heart, and liver in 13 day post natal mice (panel A) or 5 week old mice (panel B). The OP-1 specific probe was a probe containing the 3' untranslated sequences described supra (0.34 Kb EarI-Pst I fragment). As a control for RNA recovery, EF-Tu (translational elongation factor) mRNA expression also was measured (EF-Tu expression is assumed to be relatively uniform in most tissues).

The arrowheads indicate the OP1-specific messages observed in the various tissues. As can be seen in Fig. 2, OP-1 expression levels vary significantly in the spleen, lung, kidney and adrenal tissues, while the EF-Tu mRNA levels are constant. Uniformly lower levels of EF-Tu mRNA levels were found in the heart, brain and liver. As can be seen from the photomicrograph, the highest levels of OP-1 mRNA appear to be in kidney and adrenal tissue, followed by the brain. By contrast, heart and liver did not give a detectable signal. Not shown are additional analyses performed on bladder tissue, which shows significant OP-1 mRNA expression, at levels close to those in kidney/adrenal tissue. The Northern blots also indicate that, like GDF-1, OP-1 mRNA expression may be bicistonic in different tissues. Four transcripts can be seen: 4 Kb, 2.4 Kb, 2.2 Kb, and 1.8 Kb transcripts can be identified in the different tissues, and cross probing with OP-1 specific probes from the proregion and N-terminal sequences of the gene indicate that these transcripts are OP-1 specific.

A side by side comparison of OP-1 and Vgr-1 in Figure 3 shows that the probes distinguish between the morphogens Vgr-1 and OP-1 transcripts in the different tissues, and also highlights the multiple transcription of OP-1 in different tissues. Specifically, Fig. 3 compares the expression of OP-1 (Panels B and D), Vgr-1 (Panel C) and EF-Tu (Panel A) (control) mRNA in 17 day embryos (lane 1) and 3 day post-natal mice (lane 2). The same filter was used for sequential hybridizations with labeled DNA probes specific for OP-1 (Panels B and D), Vgr-1 (Panel C), and EF-Tu (Panel A). Panel A: the EF-Tu specific probe (control) was the 0.4 Kb HindIII-SacI fragment (part of the protein coding region), the SacI site used belonged to the vector; Panel B: the OP-1 specific probe was the 0.68 Kb BstXI-BglI fragment containing pro region sequences; Panel D; the OP-1 specific probe was the 0.34 Kb EarI-PstI fragment containing the 3' untranslated sequence; Panel C: the Vgr-1 specific probe was the 0.26 Kb PvuII-SacI fragment used in the Vgr-I blots described above.

The 1.8-2.5 Kb OP-1 mRNA appears approximately two times higher in three day post natal mice than in 17 day embryos, perhaps reflecting phases in bone and/or kidney development. In addition, of the four messages found in brain, the 2.2 Kb transcript appears most abundant, whereas in lung and spleen the 1.8 Kb message predominates. Finally, careful separation of the renal and adrenal tissue in five week old mice reveals that the 2.2 Kb transcripts were derived from renal tissue and the 4 Kb mRNA is more prominent in adrenal tissue (see Figure 2).

Similarly, using the same general probing methodology, BMP3 and CBMP2B transcripts recently have been identified in abundance in lung tissue.

Morphogen distribution in embryonic tissue can be determined using five or six-day old mouse embryos and standard immunofluorescence techniques in concert with morphogen-specific antisera. For example, rabbit anti-OP-1 antisera is readily obtained using any of a number of standard antibody protocols well known to those having ordinary skill in the art. The antibodies then are fluorescently labelled using standard procedures. A five or six-day old mouse embryo then is thin-sectioned and the various developing tissues probed with the labelled antibody, again following standard protocols. Using this technique, OP-1 protein has been detected in developing brain and heart.

This method also may be used to identify morphogens in adult tissues undergoing repair. For example, a fracture site can be induced in a rat long bone such as the femur. The fracture then is allowed to heal for 2 or 3 days. The animal then is sacrificed and the fractured site sectioned and probed for the presence of the morphogen e.g., OP-1, with fluorescently labelled rabbit anti-OP-1 antisera using standard immunolocalization methodology. This technique identifies OP-1 in muscle satellite cells, the progenitor cells for the development of muscle, cartilage and endochondral bone. In addition, OP-1 is detected with potential pluripotential stem cells in the bone marrow, indicating its morphogenic role in tissue repair and regeneration.

OP-1 protein also has been identified in rat brain using standard immunofluorescence staining technique. Specifically, adult rat brain (2-3 months old) and spinal cord is frozen and sectioned. Anti-OP-1, raised in rabbits and purified on an OP-1 affinity column prepared using standard methodologies, was added to the sections under standard conditions for specific binding. Goat anti-rabbit IgG, labelled with fluorescence, then was used to visualize OP-1 antibody binding to tissue sections.

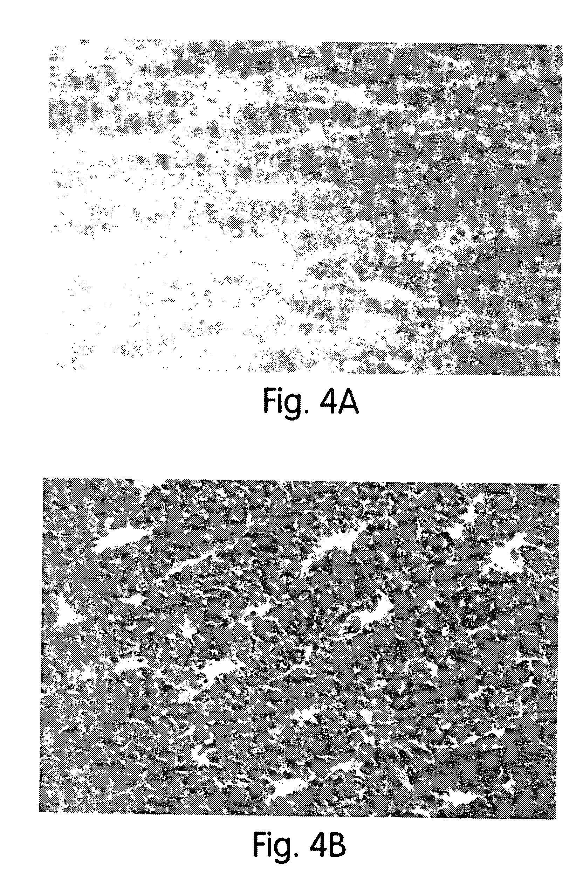

As can be seen in FIG 4A and 4B, immunofluorescence staining demonstrates the presence of OP-1 in adult rat central nervous system (CNS.) Similar and extensive staining is seen in both the brain (4A) and spinal cord (4B). OP-1 appears to be predominantly localized to the extracellular matrix of the grey matter, present in all areas except the neuronal cell bodies. In white matter, staining appears to be confined to astrocytes. A similar staining pattern also was seen in newborn rat (10 day old) brain sections.

### CELL DIFFERENTIATION

The ability of morphogens of this invention to induce cell differentiation can be determined by culturing early mesenchymal cells in the presence of the morphogen and then studying the histology of the cultured cells by staining with toluidine blue. For example, it is known that rat mesenchymal cells destined to become mandibular bone, when separated from the overlying epithelial cells at stage 11 and cultured in vitro under standard tissue culture conditions, will not continue to differentiate. However, if these same cells are left in contact with the overlying endoderm for an additional day, at which time they become stage 12 cells, they will continue to differentiate on their own in vitro to form chondrocytes. Further differentiation into obsteoblasts and, ultimately, mandibular bone, requires an appropriate local environment, e.g., a vascularized environment.

It has now been discovered that stage 11 mesenchymal cells, cultured in vitro in the presence of a morphogen, e.g., OP-1, continue to differentiate in vitro to form chondrocytes. These stage 11 cells also continue to differentiate in vitro if they are cultured with the cell products harvested from the overlying endodermal cells. Moreover, OP-1 can be identified in the medium conditioned by endodermal cells either by Western blot or immunofluorescence. This experiment may be performed with other morphogens and with different mesenchymal cells to assess the cell differentiation capability of different morphogens, as well as their distribution in different developing tissues.

As another example of morphogen-induced cell differentiation, the effect of OP-1 on the differentiation of neuronal cells has been tested in culture. Specifically, the effect of OP-1 on the NG108-15 neuroblastoma x glioma hybrid clonal cell line has been assessed. The cell line shows a fibroblastic-type morphology in culture. The cell line can be induced to differentiate chemically using 0.5 mM butyrate, 1% DMSO or 500 mM Forskolin, inducing the expression of virtually all important neuronal properties of cultured primary neurons. However, chemical induction of these cells also induces cessation of cell division.

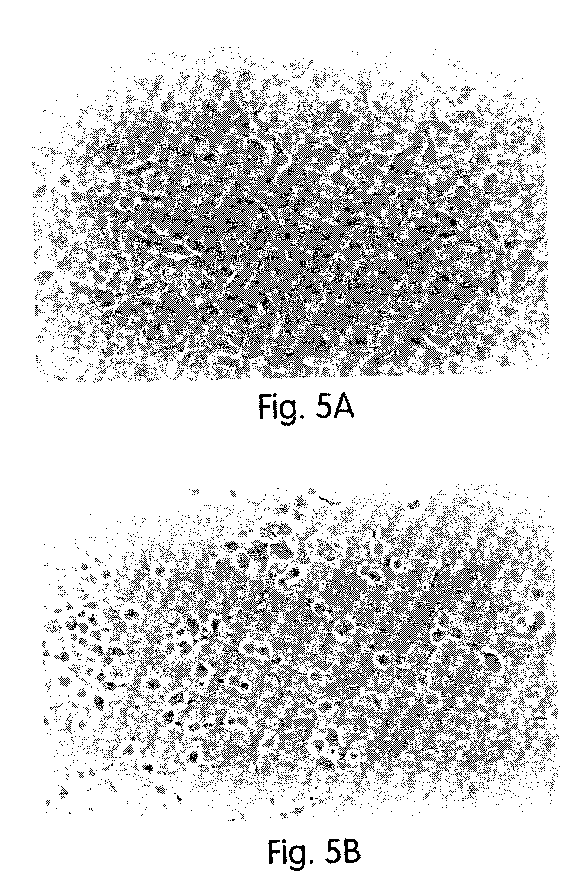

In the present experiment NG108-15 cells were subcultured on poly-L-lysine coated 6 well plates. Each well contained 40-50,000 cells in 2.5 ml of chemically defined medium. On the third day 2.5 *µ*l of OP-1 in 60% ethanol containing 0.025% trifluoroacetic was added to each well. OP-1 concentrations of 0, 1, 10, 40 and 100 ng/ml were tested. The media was changed daily with new aliquots of OP-1. After four days with 40 and 100 ng OP-1/ml concentrations, OP-1 induced differentiation of the NG108-15 cells. Figure 5 shows the morphological changes that occur. The OP-1 induces clumping and rounding of the cells and the production of neurite outgrowths (processes). Compare FIG 5A (naive NG108-15 cells) with FIG 5B, showing the effects of OPI-treated cells. Thus the OP-1 can induce the cells to differentiate into a neuronal cell morphology. Some of the outgrowths appear to join in a synaptic-type junction. This effect was not seen in cells incubated with TGF-B1 at concentrations of 1 to 100 ng/ml.

The neuroprotective effects of OP-1 were demonstrated by comparison with chemical differentiation agents on the NG108-15 cells. 50,000 cells were plated on 6 well plates and treated with butyrate, DMSO, Forskolin or OP-1 for four days. Cell counts demonstrated that in the cultures containing the chemical agents the differentiation was accompanied by a cessation of cell division. In contrast, the cells induced to differentiate by OP-1 continued to divide, as determined by H³-thymidine uptake. The data suggest that OP-1 is capable of maintaining the stability of the cells in culture after differentiation.

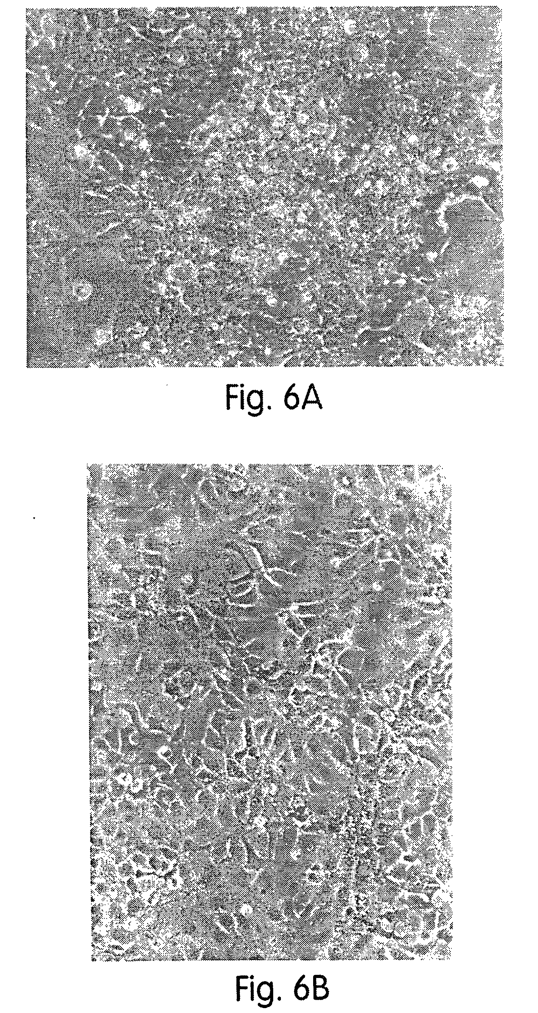

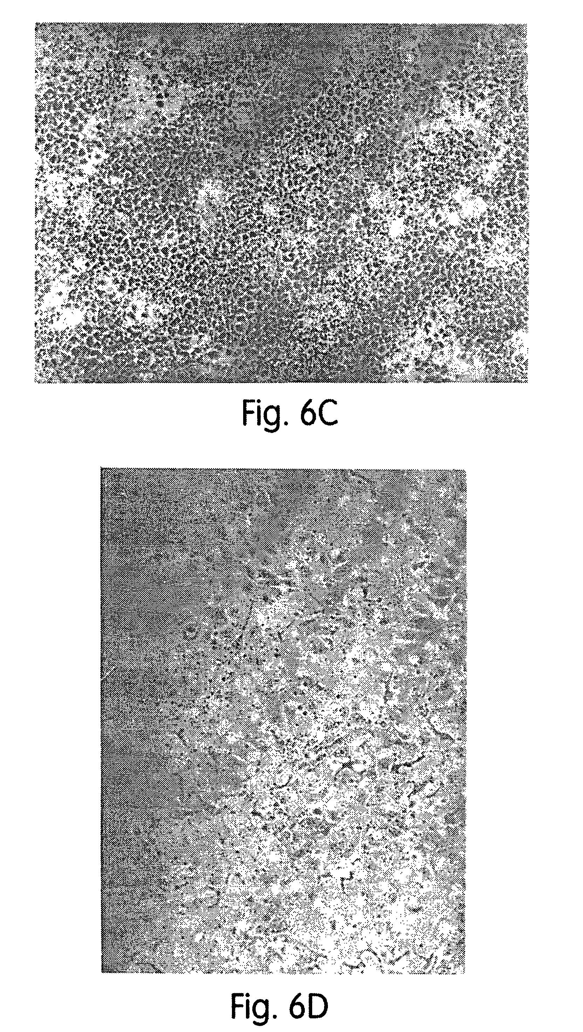

As yet another, related example, the ability of the morphogens of this invention to induce the "redifferentiation" of transformed cells also has been assessed. Specifically, the effect of OP-1 on human EC cells (embryo carcinoma cells, NTERA-Z CL.D1) is disclosed herein. In the absence of an external stimulant these cells can be maintained as undifferentiated stem cells, and can be induced to grow in serum free media (SFM). In the absence of morphogen treatment the cells proliferate rampantly and are anchorage-independent. The effect of morphogen treatment is seen in Figs. 6A-D. Figs 6A and 6B show 4 days of growth in SFM in the presence of OP-1 (25ng/ml, 6A) or the absence of morphogen (6B). Figs. 6C and 6D are 5 days growth in the presence of 10ng/ml OP-1 (6C) or no morphogen (6D). Figs. 6C and 6D are at 10x and 20x magnification compared to FIGs 6A and 5B. As can readily be seen, in the presence of OP-1, EC cells grow as flattened cells, becoming anchorage dependent. In addition, growth rate is reduced approximately 10 fold. Finally, the cells are induced to differentiate.

### MAINTENANCE OF PHENOTYPE

The morphogens of this invention also may be used to maintain a cell's differentiated phenotype. This morphogenic capability is particularly useful for inducing the continued expression of phenotype in senescent or quiescent cells.

The phenotypic maintenance capability of morphogens is readily assessed. A number of differentiated cells become senescent or quiescent after multiple passages under standard tissue culture conditions in vitro. However, if these cells are cultivated in vitro in association with a morphogen of this invention, the cells are induced to maintain expression of their phenotype through multiple passages. For example, the alkaline phosphatase activity of cultured osteoblasts, like cultured osteoscarcoma cells and calvaria cells, is significantly reduced after multiple passages in vitro. However, if the cells are cultivated in the presence of a morphogen (e.g., OP-1), alkaline phosphatase activity is maintained over extended periods of time. Similarly, phenotypic expression of myocytes also is maintained in the presence of the morphogen. This experiment may be performed with other morphogens and different cells to assess the phenotypic maintenance capability of different morphogens on cells of differing origins.

Phenotypic maintenance capability also may be assessed in vivo, using a rat model for osteoporosis, disclosed in co-pending. USSN 752,857, filed August 30, 1991,, (corresponding to granted US Patent No. 5,674,844). As disclosed therein, Long Evans rats are ovariectomized to produce an osteoporotic condition resulting from decreased estrogen production. Eight days after ovariectomy, rats are systemically provided with phosphate buffered saline (PBS) or OP-1 (21 *µ*g or 20 *µ*g) for 22 days. The rats then are sacrificed and serum alkaline phosphatase levels, serum calcium levels, and serum osteocalcin levels are determined, using standard methodologies. Three-fold higher levels of osteocalcin levels are found in rats provided with 1 or 20 *µ*g of OP-1. Increased alkaline phosphatase levels also were seen. Histomorphometric analysis on the tibial diaphysical bone shows OP-1 can reduce bone mass lost due to the drop in estrogen levels.

### CELL STIMULATION

The ability of the morphogens of this invention to stimulate the proliferation of progenitor cells also can be assayed readily in vitro. Useful naive stem cells include pluripotential stem cells, which may be isolated from bone marrow or umbilical cord blood using conventional methodologies, (see, for example, Faradji et al., (1988) Vox Sang. 55 (3):133-138 or Broxmeyer et al., (1989) PNAS 86 (10):3828-3832), as well as naive stem cells obtained from blood. Alternatively, embryonic cells (e.g., from a cultured mesodermal cell line) may be useful.

Another method for obtaining progenitor cells and for determining the ability of morphogens to stimulate cell proliferation is to capture progenitor cells from an in vivo source. For example, a biocompatible matrix material able to allow the influx of migratory progenitor cells may be implanted at an in vivo site long enough to allow the influx of migratory progenitor cells. For example, a bone-derived, guanidine-extracted matrix, formulated as disclosed for example in Sampath et al. ((1983) PNAS 80:6591-6595), or U.S. Patent No. 4,975,526, may be implanted into a rat at a subcutaneous site, essentially following the method of Sampath et al. (ibid). After three days the implant is removed, and the progenitor cells associated with the matrix dispersed and cultured.

Progenitor cells, however obtained, then are incubated in vitro with a suspected morphogen under standard cell culture conditions well known to those having ordinary skill in the art. In the absence of external stimuli, the progenitor cells do not, or minimally proliferate on their own in culture. However, if the cells are cultured in the presence of a morphogen, such as OP-1, they are stimulated to proliferate. Cell growth can be determined visually or spectrophotometrically using standard methods well known in the art.

### PROLIFERATION OF PROGENITOR CELL POPULATIONS

Progenitor cells may be stimulated to proliferate in vivo or ex vivo. The cells may be stimulated in vivo by injecting or otherwise providing a sterile preparation containing the morphogen into the individual. For example, the hemopoietic pluripotential stem cell population of an individual may be stimulated to proliferate by injecting or otherwise providing an appropriate concentration of the morphogen to the individual's bone marrow.

Progenitor cells may be stimulated ex vivo by contacting progenitor cells of the population to be enhanced with a morphogen under sterile conditions at a concentration and for a time sufficient to stimulate proliferation of the cells. In general, a period of from about 10 minutes to about 24 hours should be sufficient. The stimulated cells then are provided to the individual as, for example, by injecting the cells to an appropriate in vivo locus. Suitable biocompatible progenitor cells may be obtained by any of the methods known in the art or described herein.

### REGENERATION OF DAMAGED OR DISEASED TISSUE

The morphogens of this invention may be used to repair diseased or damaged mammalian tissue. The tissue to be repaired is preferably assessed, and excess necrotic or interfering scar tissue removed as needed, by surgical, chemical, ablating or other methods known in the medical arts.

The morphogen then may be provided directly to the tissue locus as part of a sterile, biocompatible composition, either by surgical implantation or injection. Alternatively, a sterile, biocompatible composition containing morphogen-stimulated progenitor cells may be provided to the tissue locus. The existing tissue at the locus, whether diseased or damaged, provides the appropriate matrix to allow the proliferation and tissue-specific differentiation of progenitor cells. In addition, a damaged or diseased tissue locus, particularly one that has been further assaulted by surgical means, provides a morphogenically permissive environment. For some tissues, it is envisioned that systemic provision of the morphogen will be sufficient.

In some circumstances, particularly where tissue damage is extensive, the tissue may not be capable of providing a sufficient matrix for cell influx and proliferation. In these instances, it may be necessary to provide the morphogen or morphogen-stimulated progenitor cells to the tissue locus in association with a suitable, biocompatible formulated matrix, prepared by any of the means described below. The matrix preferably is tissue-specific, in vivo biodegradable, and comprises particles having dimensions within the range of 70-850*µ*m, most preferably 150-420*µ*m.

The morphogens of this invention also may be used to prevent or substantially inhibit scar tissue formation following an injury. If a morphogen is provided to a newly injured tissue locus, it can induce tissue morphogenesis at the locus, preventing the aggregation of migrating fibroblasts into non-differentiated connective tissue. The morphogen preferably is provided as a sterile pharmaceutical preparation injected into the tissue locus within five hours of the injury. Several non-limiting examples follow, illustrating the morphogens regenerate capabilities in different issues. The proteins of this invention previously have been shown to be capable of inducing cartilage and endochondral bone formation (See, for example U.S. Patent No. 5,011,691).

As an example, protein-induced morphogenesis of substantially injured liver tissue following a partial hepatectomy is disclosed. Variations on this general protocol may be used to test morphogen activity in other different tissues. The general method involves excising an essentially nonregenerating portion of a tissue and providing the morphogen, preferably as a soluble pharmaceutical preparation to the excised tissue locus, closing the wound and examining the site at a future date. Like bone, liver has a potential to regenerate upon injury during post-fetal life.

Morphogen, (e.g., purified recombinant human OP-1, mature form), was solubilized (1 mg/ml) in 50% ethanol (or compatible solvent) containing 0.1% trifluoroacetic acid (or compatible acid). The injectable OP-1 solution was prepared by diluting one volume of OP-1/solvent-acid stock solution with 9 volumes of 0.2% rat serum albumin in sterile PBS (phosphate-buffered saline).

Growing rats or aged rats were anesthetized by using ketamine. Two of the liver lobes (left and right) were cut out (approximately 1/3 of the lobe) and the OP-1 was injected locally at multiple sites along the cut ends. The amount of OP-1 injected was 100 *µ*g in 100 of PBS/RSA (phosphate buffered saline/rat serum albumin) injection buffer. Placebo samples are injection buffer without OP-1. Five rats in each group were used. The wound was closed and the rats were allowed to eat normal food and drink tap water.

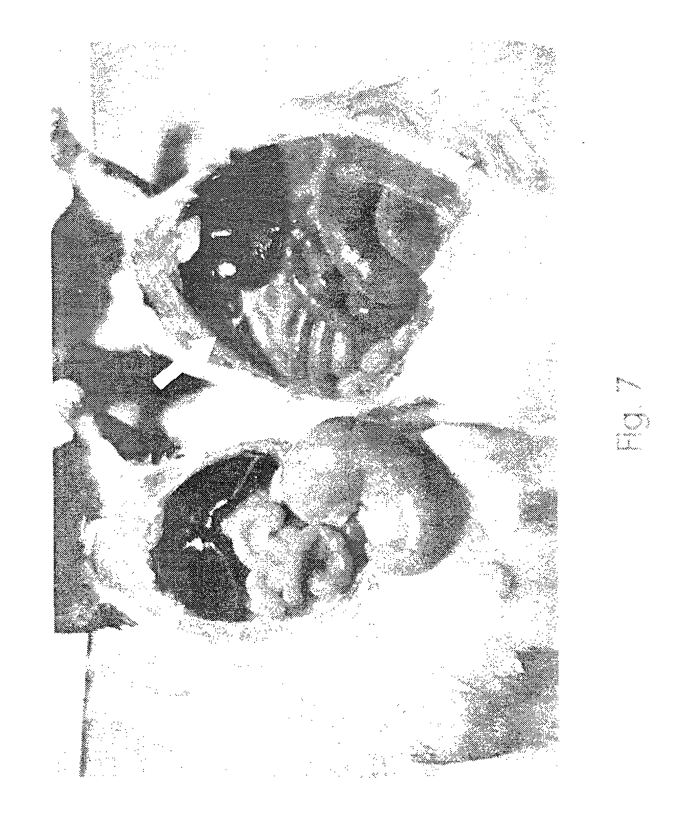

After 12 days, the rats were sacrificed and liver regeneration was observed visually. The photomicrograph in Fig. 7 illustrates dramatically the regenerative effects of OP-1 on liver regeneration. The OP-1-injected group showed complete liver tissue regeneration and no sign remained of any cut in the liver (animal 2). By contrast, in the control group into which only PBS was injected only minimal regeneration was evidenced (animal 1). In addition, the incision remains in this sample.

As another example, the ability of the morphogens of this invention to induce dentinogenesis also was assessed. To date, the unpredictable response of dental pulp tissue to injury is a basic clinical problem in dentistry. Cynomolgus monkeys were chosen as primate models as monkeys are presumed to be more indicative of human dental biology than models based on lower non-primate mammals.

Using standard dental surgical procedures, small areas (e.g., 2mm) of dental pulps were surgically exposed by removing the enamel and dentin immediately above the pulp (by drilling) of sample teeth, performing a partial amputation of the coronal pulp tissue, inducing hemostasis, application of the pulp treatment, and sealing and filling the cavity by standard procedures.

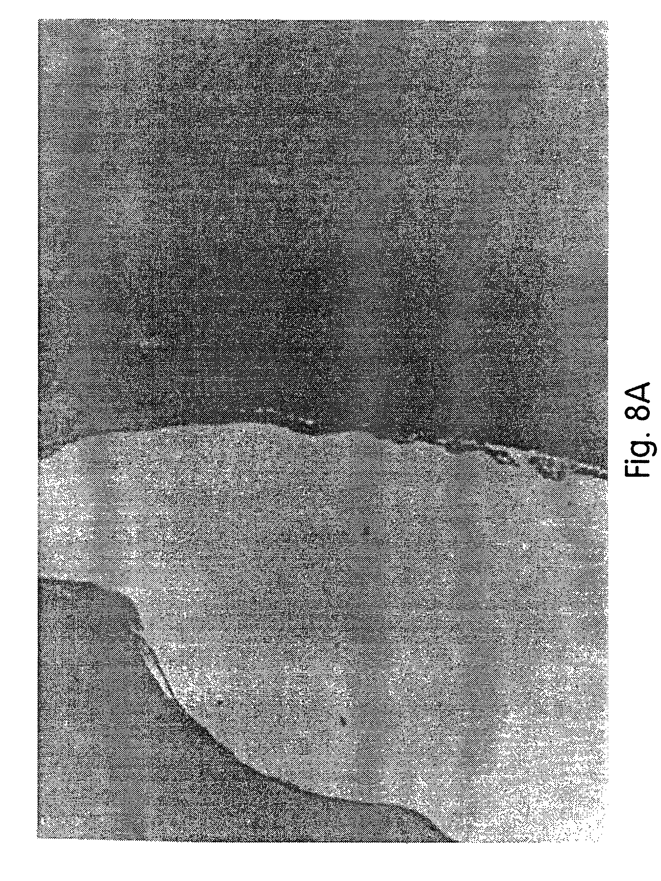

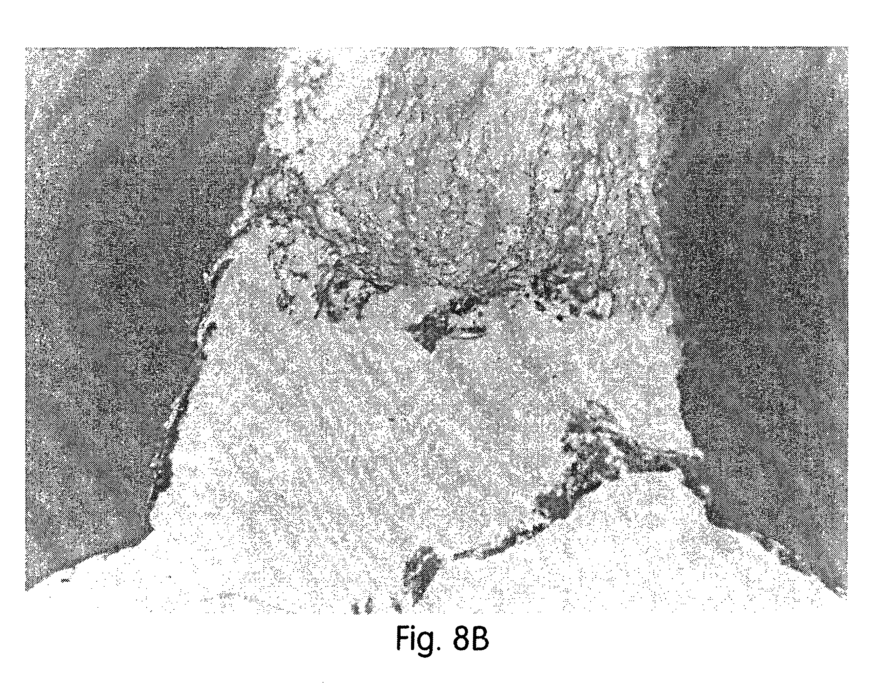

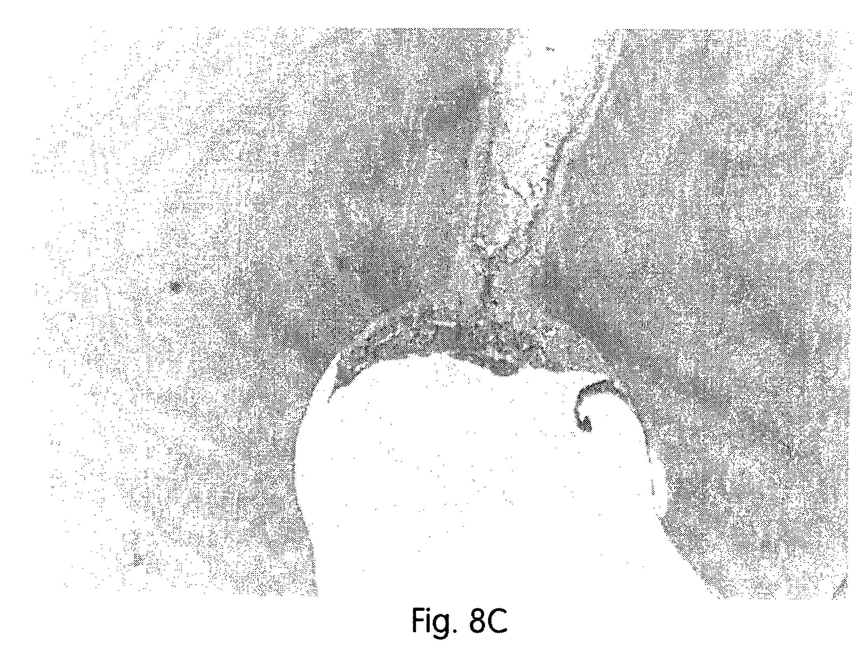

Pulp treatments used were: OP-1 dispersed in a carrier matrix; carrier matrix alone and no treatment. Twelve teeth per animal (four for each treatment) were prepared, and two animals were used. At four weeks, teeth were extracted and processed histologically for analysis of dentin formation, and/or ground to analyze dentin mineralization. FIG.8 illustrates dramatically the effect of morphogen on osteodentin reparation. FIG. 8A is a photomicrograph of the control treatment (PBS) and shows little or no reparation. FIG. 8B is a photomicrograph of treatment with carrier alone, showing minimal reparation. By contrast, treatment with morphogen (FIG. 8C) shows significant reparation. The results of FIG. 8 indicate that OP-1-CM (OP-1 plus carrier matrix) reliably induced formation of reparative or osteodentin bridges on surgically exposed healthy dental pulps. By contrast, pulps treated with carrier matrix alone, or not treated failed to form reparative dentin.

As another example, the morphogen-induced regenerative effects on central nervous system (CNS) repair may be assessed using a rat brain stab model. Briefly, male Long Evans rats are anesthesized and the head area prepared for surgery. The calvariae is exposed using standard surgical procedures and a hole drilled toward the center of each lobe using a 0.035K wire, just piercing the calvariae. 25*µ*l solutions containing either morphogen (OP-1, 25*µ*g) or PBS then is provided to each of the holes by Hamilton syringe. Solutions are delivered to a depth approximately 3 mm below the surface, into the underlying cortex, corpus callosum and hippocampus. The skin then is sutured and the animal allowed to recover.

Three days post surgery, rats are sacrificed by decapitation and their brains processed for sectioning. Scar tissue formation is evaluated by immunofluoresence staining for glial fibrillary acidic protein, a marker protein for glial scarring, to qualitatively determine the degree of scar formation. Sections also are probed with anti-OP-1 antibodies to determine the presence of OP-1. Reduced levels of glial fibrillary acidic protein are anticipated in the tissue sections of animals treated with morphogen, evidencing the ability of morphogen to inhibit glial scar formation, thereby stimulating nerve regeneration.

### MORPHOGEN ACTIVITY MODULATION

Antibodies to morphogens of this invention have been identified in healthy human sera. In addition, implanting devices comprising morphogens (e.g., OP-1) have been discovered to induce an increase in anti-morphogen antibodies (e.g., anti-OP-1 antibodies). It is anticipated that these antibodies comprise part of the body's regulation of morphogen activity in vivo. The presence of the antibodies, and fluctuations in their levels, which are readily monitored, can provide a useful method for monitoring tissue stasis and tissue viability (e.g., identification of a pathological state). For example, standard radioimmunoassays or ELISA may be used to detect and quantify endogeous anti-morphogen antibodies in sera. Antibodies or other binding proteins capable of detecting anti-morphogen antibodies may be obtained using standard methodologies.

### MATRIX PREPARATION

The morphogens of this invention may be implanted surgically, dispersed in a biocompatible, preferably in vivo biodegradable matrix appropriately modified to provide a structure in which the morphogen may be dispersed and which allows the influx, differentiation and proliferation of migrating progenitor cells. The matrix also should provide signals capable of directing the tissue specificity of the differentiating cells, as well as a morphogenically permissive environment, being essentially free of growth inhibiting signals.

In the absence of these features the matrix does not appear to be suitable as part of a morphogenic composition. Recent studies on osteogenic devices (morphogens dispersed within a formulated matrix) using matrices formulated from polylactic acid and/or polyglycolic acid biopolymers, ceramics (a-tri-calcium-phosphate), or hydroxyapatite show that these materials, by themselves, are unable to provide the appropriate environment for inducing de novo endochondral bone formation in rats by themselves. In addition, matrices formulated from commercially available highly purified, reconstituted collagens or naturally-derived non-bone, species-specific collagen (e.g., from rat tail tendon) also are unsuccessful in inducing bone when implanted in association with an osteogenic protein. These matrices apparently lack specific structurally-related features which aid in directing the tissue specificity of the morphogen-stimulated, differentiating progenitor cells.

The formulated matrix may be shaped as desired in anticipation of surgery or may be shaped by the physician or technician during surgery. Thus, the material may be used in topical, subcutaneous, intraperitoneal, or intramuscular implants to repair tissue or to induce its growth de novo. The matrix preferably is biodegradable in vivo, being slowly absorbed by the body and replaced by new tissue growth, in the shape or very nearly in the shape of the implant.

Details of how to make and how to use the matrices useful in this invention are disclosed below.

### TISSUE-DERIVED MATRICES

Suitable biocompatible, in vivo biodegradable acellular matrices may be prepared from naturally-occurring tissue. The tissue is treated with suitable agents to substantially extract the cellular, nonstructural components of the tissue. The agents also should be capable of extracting any growth inhibiting components associated with the tissue. The resulting material is a porous, acellular matrix, substantially depleted in nonstructurally-associated components.

The matrix also may be further treated with agents that modify the matrix, increasing the number of pores and micropits on its surfaces. Those skilled in the art will know how to determine which agents are best suited to the extraction of nonstructural components for different tissues. For example, soft tissues such as liver and lung may be thin-sectioned and exposed to a nonpolar solvent such as, for example, 100% ethanol, to destroy the cellular structure of the tissue and extract nonstructural components. The material then is dried and pulverized to yield nonadherent porous particles. Structural tissues such as cartilage and dentin where collagen is the primary component may be demineralized and extracted with guanidine, essentially following the method of Sampath et al. (1983) PNAS 80:6591-6595. For example, pulverized and demineralized dentin is extracted with five volumes of 4M guanidine-HCl, 50mM Tris-HCl, pH 7.0 for 16 hours at 4°C. The suspension then is filtered. The insoluble material that remains is collected and used to fabricate the matrix. The material is mostly collagenous in manner. It is devoid of morphogenic activity. The matrix particles may further be treated with a collagen fibril-modifying agent that extracts potentially unwanted components from the matrix, and alters the surface structure of the matrix material. Useful agents include acids, organic solvents or heated aqueous media. A detailed description of these matrix treatments are disclosed in U.S. Patent No. 4,975,526 and PCT publication US90/00912, published September 7, 1990 (WO90/10018).

The currently most preferred agent is a heated aqueous fibril-modifying medium such as water, to increase the matrix particle surface area and porosity. The currently most preferred aqueous medium is an acidic aqueous medium having a pH of less than about 4.5, e.g., within the range of about pH 2 - pH 4 which may help to "swell" the collagen before heating. 0.1% acetic acid, which has a pH of about 3, currently is most preferred. 0.1 M acetic acid also may be used.

Various amounts of delipidated, demineralized guanidine-extracted bone collagen are heated in the aqueous medium (1g matrix/30ml aqueous medium) under constant stirring in a water jacketed glass flask, and maintained at a given temperature for a predetermined period of time. Preferred treatment times are about one hour, although exposure times of between about 0.5 to two hours appear acceptable. The temperature employed is held constant at a temperature within the range of about 37°C to 65°C. The currently preferred heat treatment temperature is within the range of about 45°C to 60°C.

After the heat treatment, the matrix is filtered, washed, lyophilized and used for implant. Where an acidic aqueous medium is used, the matrix also is preferably neutralized prior to washing and lyophilization. A currently preferred neutralization buffer is a 200mM sodium phosphate buffer, pH 7.0. To neutralize the matrix, the matrix preferably first is allowed to cool following thermal treatment, the acidic aqueous medium (e.g., 0.1% acetic acid) then is removed and replaced with the neutralization buffer and the matrix agitated for about 30 minutes. The neutralization buffer then may be removed and the matrix washed and lyophilized.

Other useful fibril-modifying treatments include acid treatments (e.g., trifluoroacetic acid and hydrogen fluoride) and solvent treatments such as dichloromethane, acetonitrile, isopropanol and chloroform, as well as particular acid/solvent combinations.

After contact with the fibril-modifying agent, the treated matrix may be washed to remove any extracted components, following a form of the procedure set forth below:
1. Suspend matrix preparation in TBS (Tris-buffered saline) lg/200 ml and stir at 4°C for 2 hrs; or in 6 M urea, 50 mM Tris-HCl, 500 mM NaCl, pH 7.0 (UTBS) or water and stir at room temperature (RT) for 30 minutes (sufficient time to neutralize the pH);
2. Centrifuge and repeat wash step; and
3. Centrifuge; discard supernatant; water wash residue; and then lyophilize.

### SYNTHETIC TISSUE-SPECIFIC MATRICES

In addition to the naturally-derived tissue-specific matrices described above, useful tissue-specific matrices may be formulated synthetically if appropriately modified. These porous biocompatible, in vivo biodegradable synthetic matrices are disclosed in PCT publication US91/03603, published December 12, 1991 (WO91/18558). Briefly, the matrix comprises a porous crosslinked structural polymer of biocompatible, biodegradable collagen and appropriate, tissue-specific glycosaminoglycans as tissue-specific cell attachment factors. Collagen derived from a number of sources may be suitable for use in these synthetic matrices, including insoluble collagen, acid-soluble collagen, collagen soluble in neutral or basic aqueous solutions, as well as those collagens which are commercially available.

Glycosaminoglycans (GAGs) or mucopolysaccharides are hexosamine-containing polysaccharides of animal origin that have a tissue specific distribution, and therefore may be used to help determine the tissue specificity of the morphogen-stimulated differentiating cells. Reaction with the GAGs also provides collagen with another valuable property, i.e., inability to provoke an immune reaction (foreign body reaction) from an animal host.

Chemically, GAGs are made up of residues of hexoseamines glycosidically bound and alternating in a more-or-less regular manner with either hexouronic acid or hexose moieties (see, e.g., Dodgson et al. in Carbohydrate Metabolism and its Disorders (Dickens et al., eds.) Vol. 1, Academic Press (1968)). Useful GAGs include hyaluronic acid, heparin, heparin sulfate, chondroitin 6-sulfate, chondroitin 4-sulfate, dermatan sulfate, and keratin sulfate. Other GAGs are suitable for forming the matrix described herein, and those skilled in the art will either know or be able to ascertain other suitable GAGs using no more than routine experimentation. For a more detailed description of mucopolysaccharides, see Aspinall, Polysaccharides, Pergamon Press, Oxford (1970). For example, as disclosed in U.S. Application Serial No. 529,852 (corresponding to international publication number WO-A-91 18558, chondroitin-6-sulfate can be used where endochondral bone formation is desired. Heparin sulfate, on the other hand, may be used to formulate synthetic matrices for use in lung tissue repair.

Collagen can be reacted with a GAG in aqueous acidic solutions, preferably in diluted acetic acid solutions. By adding the GAG dropwise into the aqueous collagen dispersion, coprecipitates of tangled collagen fibrils coated with GAG results. This tangled mass of fibers then can be homogenized to form a homogeneous dispersion of fine fibers and then filtered and dried.

Insolubility of the collagen-GAG products can be raised to the desired degree by covalently cross-linking these materials, which also serves to raise the resistance to resorption of these materials. In general, any covalent cross-linking method suitable for cross-linking collagen also is suitable for cross-linking these composite materials, although crosslinking by a dehydrothermal process is preferred.

When dry, the crosslinked particles are essentially spherical, with diameters of about 500 *µ*m. Scanning electron miscroscopy shows pores of about 20 *µ*m on the surface and 40 *µ*m on the interior. The interior is made up of both fibrous and sheet-like structures, providing surfaces for cell attachment. The voids interconnect, providing access to the cells throughout the interior of the particle. The material appears to be roughly 99.5% void volume, making the material very efficient in terms of the potential cell mass that can be grown per gram of microcarrier.

The morphogens described herein can be combined and dispersed in an appropriately modified tissue-specific matrix using any of the methods described below:
1. Ethanol Precipitation
   Matrix is added to the morphogen dissolved in guanidine-HCl. Samples are vortexed and incubated at a low temperature. Samples are then further vortexed. Cold absolute ethanol is added to the mixture which is then stirred and incubated. After centrifugation (microfuge, high speed) the supernatant is discarded. The matrix is washed with cold concentrated ethanol in water and then lyophilized.
2. Acetonitrile Trifluoroacetic Acid Lyophilization
   In this procedure, morphogen in an acetonitrile trifluroacetic acid (ACN/TFA solution is added to the carrier material. Samples are vigorously vortexed many times and then lyophilized.
3. Buffered Saline Lyophilization
   Morphogen preparations in physiological saline may also be vortexed with the matrix and lyophilized to produce morphogenically active material.

### BIOASSAY

The following sets forth various procedures for evaluating the in vivo morphogenic utility of the morphogens and morphogenic compositions of this invention. The proteins and compositions may be injected or surgically implanted in a mammal, following any of a number of procedures well known in the art. For example, surgical implant bioassays may be performed essentially following the procedure of Sampath et al. (1983) PNAS 80:6591-6595.

### Histological Evaluation

Histological sectioning and staining is preferred to determine the extent of morphogenesis in vivo, particularly in tissue repair procedures. Excised implants are fixed in Bouins Solution, embedded in paraffin, and cut into 6-8 *µ*m sections. Staining with toluidine blue or hemotoxylin/eosin demonstrates clearly the ultimate development of the new tissue. Twelve day implants are usually sufficient to determine whether the implants contain newly induced tissue.

Successful implants exhibit a controlled progression through the stages of induced tissue development allowing one to identify and follow the tissue-specific events that occur. For example, in endochondral bone formation the stages include:
(1) leukocytes on day one; (2) mesenchymal cell migration and proliferation on days two and three;
(3) chondrocyte appearance on days five and six;
(4) cartilage matrix formation on day seven;
(5) cartilage calcification on day eight; (6) vascular invasion, appearance of osteoblasts, and formation of new bone on days nine and ten; (7) appearance of osteoblastic and bone remodeling and dissolution of the implanted matrix on days twelve to eighteen; and
(8) hematopoietic bone marrow differentiation in the ossicle on day twenty-one.

### Biological Markers

In addition to histological evaluation, biological markers may be used as a marker for tissue morphogenesis. Useful markers include tissue-specific enzymes whose activities may be assayed (e.g., spectrophotometrically) after homogenization of the implant. These assays may be useful for quantitation and for obtaining an estimate of tissue formation quickly after the implants are removed from the animal. For example, alkaline phosphatase activity may be used as a marker for osteogenesis.

Incorporation of systemically provided morphogens may be followed using tagged morphogens (e.g., radioactively labelled) and determining their localization in new tissue, and/or by monitoring their disappearance from the circulatory system using a standard pulse-chase labeling protocol. The morphogen also may be provided with a tissue-specific molecular tag, whose uptake may be monitored and correlated with the concentration of morphogen provided. As an example, ovary removal in female rats results in reduced bone alkaline phosphatase activity, rendering the rats predisposed to osteoporosis. If the female rats now are provided with a morphogen, e.g., OP-1, a reduction in the systemic concentration of calcium (CA²⁺) is seen, which correlates with the presence of the provided morphogen and can be shown to correspond to increased alkaline phosphatase activity.

The present embodiments are therefore to be considered in all respects as illustrative and not restrictive, the scope of the invention being indicated by the appended claims rather than by the foregoing description, and all changes which come within the meaning and range of equivalency of the claims are therefore intended to be embraced therein.

## Claims

1. A composition comprising: (1) a biocompatible acellular matrix that provides an anchoring substratum for mammalian progenitor cells and is essentially free of substances that inhibit morphogenesis; and (b) a morphogen sorbed on said matrix at a concentration sufficient for inducing the developmental cascade of tissue-specific morphogenesis when said composition is disposed at a non-skeletal tissue locus *in vivo*, said matrix having components specific for said tissue locus.

2. The composition of claim 1, wherein said matrix is biodegradable and/or porous (*e.g.* being derived from organ-specific tissue or comprising collagen or cell attachment factors selected from glycosaminoglycans and proteoglycan).

3. A composition comprising a morphogen in association with a matrix derived from dehydrated non-skeletal tissue.

4. The composition of claim 1, 2 or 3 wherein said matrix is derived from liver tissue, or from demineralised and guanidine-extracted dentin, for example for use in dentinogenesis, liver regeneration, dentin regeneration or the repair of damaged liver or dentin tissue (*e.g.* arising from disease or mechanical injury).

5. The composition of any one of claims 1 to 4 for:
(a) increasing a renewing progenitor cell population in a mammal;
(b) inducing non-skeletal tissue morphogenesis in a mammal;
(c) use in a method comprising the steps of providing progenitor cells disposed on said acellular matrix to a non-skeletal tissue locus *in vivo* to thereby induce non-skeletal tissue-specific differentiation and proliferation within said locus;
(d) maintaining or restoring the phenotypic expression of differentiated mammalian cells (*e.g*. cells which have become (or are at risk of becoming) senescent or quiescent);
(f) inducing the proliferation of mammalian progenitor cells (*e.g. ex vivo*).

6. Use of a morphogen (or the composition of any one of claims 1-5) for the manufacture of a medicament for promoting non-skeletal tissue regeneration.

7. The use of claim 6 wherein the tissue is tissue other than bone and cartilage, for example:
(a) lung tissue damaged by emphysema;
(b) cirrhotic liver or kidney tissue;
(c) damaged heart and/or blood vessel tissue (*e.g*. arising from cardiomyopathies, atherothrombotic or cardioembolic strokes);
(d) damaged stomach tissue arising from ulceration;
(e) damaged neural tissue (*e.g*. arising from physical injury or degenerative diseases);
(f) damaged dentin tissue (*e.g*. arising from disease or mechanical injury).

8. The use of claim 7 (e) wherein the degenerative disease is Alzheimer's disease, multiple sclerosis or stroke.

9. Use of a morphogen for the manufacture of a medicament for:
(a) stimulating redifferentiation of cells *in vivo* (*e.g.* to reduce or substantially inhibit tumorigenesis and/or growth of neoplasms);
(b) cancer therapy (*e.g.* via the inhibition of neoplasm growth);
(c) stimulating the redifferentiation of committed cells *in vivo*;
(d) inducing the de- or redifferentiation of neoplasms.

10. Use of a morphogen for the manufacture of a medicament for:
(a) preventing or substantially inhibiting the formation of scar tissue;
(b) the treatment of disorders arising from a loss or reduction of a renewable cell population (*e.g*. blood disorders and disorders arising from lost or impaired immune functions);
(c) the treatment of disorders arising from cell (*e.g*. liver cells) senescence or quiescence (*e.g.* osteoporosis) via stimulation of said cells *in vivo*;
(d) use in gene therapy to stimulate the growth of quiescent cells and thereby enhance incorporation of exogenous DNA:
(e) detecting tissue dysfunction by monitoring levels of endogenous morphogens (*e.g.* to detect changes in tissue or organ stasis);
(f) stimulating the proliferation of differentiated cells;
(g) promoting tissue stasis;
(h) dentinogenesis or dentin regeneration;
(i) liver tissue formation;
(j) skin tissue regeneration;
(k) the healing of gastrointestinal ulcers;
(l) non-skeletal tissue replacement in reconstructive surgery;
(m) the treatment of degenerative nerve disease;
(n) inducing enervation of a tissue;
(o) use as a neuroprotectant;
(p) use in CNS regeneration;
(q) inducing tissue morphogenesis in mammalian tissue, which tissue is not bone or cartilage;
(r) stimulating cells *ex vivo* followed by disposing said stimulated cells at a tissue-specific locus to induce tissue morphogenesis;
(s) use in a method comprising the steps of isolating progenitor cells from bone marrow, stimulating said cells *ex vivo* to induce proliferation and returning said cells to said bone marrow;
(t) use in a method comprising the steps of isolating biocompatible cells from a culture cell line, stimulating said cells *ex vivo* to induce proliferation and implanting said cells;
(u) induce mitogenic activity of a progenitor cell population *in vivo* (*e.g.* using a morphogen adapted for systemic administration, implantation or injection);
(v) increasing a selected population of hemopoietic stem cells (*e.g.* by extracting cells from blood by perfusion and stimulating said cells *ex vivo* to obtain stimulated cells suitable for being returned to the blood);
(w) increasing a renewing progenitor cell population in a mammal;
(x) inducing non-skeletal tissue morphogenesis in a mammal;
(y) use in a method comprising the steps of providing progenitor cells disposed on said acellular matrix to a non-skeletal tissue locus *in vivo* to thereby induce non-skeletal tissue-specific differentiation and proliferation within said locus;
(z) inducing non-skeletal replacement tissue at a non-skeletal tissue locus in a mammal;
(aa) maintaining or restoring the phenotypic expression of differentiated mammalian cells (*e.g.* liver cells), which have for example become (or are at risk of becoming) senescent or quiescent);
(ab) inducing the proliferation of mammalian progenitor cells (*e.g. ex vivo*), with the proviso that the cells in (b), (c), (f), (r), (s), (t), (u), (w), and (ab) are cells of non-skeletal tissue.

11. The composition or use of any one of the preceding claims wherein the morphogen comprises a dimeric protein that induces tissue-specific morphogenesis in said mammal and comprises a pair of folded polypeptides, the amino acid sequence of each of which comprises a sequence sharing at least 70%, *e.g.* at least 80%, homology with residues 38-139 of hOP1 (SEQ ID NO. 5).

12. The composition or use of claim 11 wherein said amino acid sequence has greater than 60%, *e.g.* greater than 65%, amino acid identity with residues 43-139 of hOP1 (SEQ ID NO. 5).

13. The composition or use of claim 12 wherein said amino acid sequence is selected from the group consisting of residues 38-139 of hOP1 (SEQ ID NO. 5); mOP1 (SEQ ID NO. 6); hOP2 (SEQ ID NO. 7) or mOP2 (SEQ ID NO. 8) or is a naturally-occurring or biosynthetic variant of a sequence selected from said group.

14. The composition or use of any one of claims 11-13 wherein said amino acid sequence is selected from the group consisting of SEQ ID NOs 9, 10, 11, 12, 13 and 14, or a naturally-occurring or biosynthetic variant of said sequence.

15. The composition or use of any one of claims 11-14 wherein the sequence of said morphogen polypeptides each further comprise an amino acid sequence selected from residues 1-37 of the group consisting of SEQ ID NOs 5, 6, 7 and 8, or a naturally-occurring or biosynthetic variant thereof, such that said sequences comprise a sequence selected from the sequences of mature hOP1, mOP1, hOP2 or naturally-occurring or biosynthetic variants thereof.

16. A method of detecting non-skeletal tissue dysfunction by monitoring levels of endogenous morphogens (*e*.*g*. to detect changes in tissue or organ stasis).

17. An *ex vivo* method of:
(a) increasing a population of mammalian progenitor cells; or
(b) maintaining the phenotypic expression of differentiated mammalian cells (*e.g.* cells at risk of becoming senescent or quiescent); comprising the step of contacting said cells *ex vivo* with a morphogen at a concentration and for a time sufficient such that said progenitor cells are stimulated to proliferate or to express (or re-express) their phenotype, respectively, wherein said cells are cells of non-skeletal tissue.

## Patentansprüche

1. Zusammensetzung umfassend: (1) Eine biokompatible azelluläre Matrix, die ein Haftsubstrat für Säugetier-Vorläuferzellen bereitstellt und im wesentlichen frei von Substanzen ist, die die Morphogenese hemmen; und (b) ein an dieser Matrix in einer zur Induzierung der Entwicklungskaskade einer gewebsspezifischen Morphogenese sorbiertes Morphogen, wobei die Zusammensetzung in vivo an einem nicht-Skelett-Gewebsort angeordnet wird, wobei die Matrix Bestandteile aufweist, die für diesen Gewebs-Ort spezifisch sind.

2. Zusammensetzung nach Anspruch 1, bei der die Matrix biologisch abbaubar und/oder porös ist (die beispielsweise aus organspezifischem Gewebe stammt oder Kollagen oder Zellanlagerungsfaktoren umfaßt, die aus Glykosaminoglykanen und Proteoglykanen ausgewählt sind).

3. Zusammensetzung, die ein Morphogen in Verbindung mit einer Matrix umfaßt, die aus dehydriertem nicht-Skelett-Gewebe gewonnen wird.

4. Zusammensetzung nach Anspruch 1, 2 oder 3, bei der die Matrix aus Lebergewebe oder aus demineralisiertem und Guanidinextrahiertem Dentin gewonnen wird, beispielsweise zur Verwendung in der Dentinogenese, der Leberregeneration, der Dentin-regeneration oder der Reparatur von beschädigtem Leber- oder Dentingewebe (wobei die Schädigung beispielsweise durch Krankheit oder mechanische Verletzung entstanden ist).

5. Zusammensetzung nach einem der Ansprüche 1 - 4 für:
(a) die Vermehrung einer sich erneuernden Vorläuferzell-Population in einem Säugetier;
(b) das Induzieren einer nicht-Skelett-Gewebsmorphogenese in einem Säugetier;
(c) die Verwendung in einem Verfahren, umfassend die Schritte, auf der azellulären Matrix angeordnete Vorläuferzellen in vivo einem nicht-Skelett-Gewebs-Ort zu verabreichen, um dadurch eine nicht-Skelett-gewebsspezifische Differenzierung und Proliferation innerhalb des Ortes zu induzieren;
(d) die Aufrechterhaltung oder Wiederherstellung der phänotypischen Expression differenzierter Säugetierzellen (beispielsweise Zellen, die alt sind oder ruhen (oder einem Risiko ausgesetzt sind, dieses zu werden));
(f) das Induzieren der Proliferation von Säugetier-Vorläuferzellen (beispielsweise ex vivo).

6. Verwendung eines Morphogens (oder der Zusammensetzung nach einem der Ansprüche 1 - 5) zur Herstellung eines Medikamentes zur Unterstützung einer nicht-Skelett-Gewebs-Regeneration.

7. Verwendung nach Anspruch 6, wobei das Gewebe ein anderes Gewebe als Knochen- und Knorpel-Gewebe ist, beispielsweise:
(a) Lungengewebe, das durch ein Emphysem geschädigt ist;
(b) zirrhotisches Leber- oder Nierengewebe;
(c) geschädigtes Herz- und/oder Blutgefäß-Gewebe (das beispielsweise aus Kardiomyopathien, artherothrombotischem oder kardioembolischem Schlaganfall entstanden sind);
(d) geschädigtes Magengewebe, das aus einer Ulzerierung entstanden ist;
(e) geschädigtes Nervengewebe (das beispielsweise aus einer körperlichen Verletzung oder degenerativen Erkrankungen entstanden ist);
(f) beschädigtes Dentingewebe (das beispielsweise aus einer Erkrankung oder mechanischen Verletzung entstanden ist).

8. Verwendung nach Anspruch 7 (e), bei der die degenerative Erkrankung die Alzheimer'sche Krankheit, multiple Sklerose oder ein Schlaganfall ist.

9. Verwendung eines Morphogens zur Herstellung eines Medikamentes für:
(a) die Stimulierung der Redifferenzierung von Zellen in vivo (beispielsweise zur Reduzierung oder zur im wesentlichen Hemmung der Tumorgenese und/oder des Wachstums von Neubildungen) ;
(b) die Krebstherapie (beispielsweise über Hemmung des Neubildungs-Wachstums);
(c) die Stimulierung der Redifferenzierung von determinierten Zellen in vivo;
(d) das Induzieren der De- oder Redifferenzierung von Neubildungen.

10. Verwendung eines Morphogens zur Herstellung eines Medikamentes für:
(a) die Vermeidung oder im wesentlichen Hemmung der Bildung von Narbengewebe;
(b) die Behandlung von Störungen, die sich aus einem Verlust oder einer Verminderung einer erneuerbaren Zellpopulation ergeben (beispielsweise Blutstörungen und Störungen, die sich aus verlorenen oder verschlechterten Immunfunktionen ergeben);
(c) die Behandlung von Störungen, die aus Zellalterung (beispielsweise von Leberzellen) oder Ruhezustand (beispielsweise Osteoporose) entstehen, über die Stimulierung der Zellen in vivo;
(d) die Verwendung in der Gentherapie zur Stimulierung des Wachstums von ruhenden Zellen und dadurch zur Steigerung des Einbaus von exogener DNA;
(e) den Nachweis der Gewebs-Dysfunktion durch Beobachten der Spiegel von endogenen Morphogenen (beispielsweise zum Nachweis von Veränderungen der Gewebs- oder Organstasis);
(f) das Stimulieren der Proliferation von differenzierten Zellen;
(g) die Förderung der Gewebs-Stasis;
(h) die Dentinogenese oder Dentin-Regeneration;
(i) die Lebergewebsbildung;
(j) die Hautgewebs-Regeneration;
(k) die Heilung von gastrointestinalen Ulzera;
(l) den nicht-Skelett-Gewebsersatz in der plastischen Chirurgie;
(m) die Behandlung einer degenerativen Nervenerkrankung;
(n) das Induzieren der Innervation eines Gewebes;
(o) die Verwendung als Nervenschutzmittel;
(p) die Verwendung in der ZNS-Regeneration;
(q) das Induzieren der Gewebsmorphogenese in Säugetiergewebe, wobei das Gewebe nicht Knochen oder Knorpel-Gewebe ist;
(r) das ex vivo-Stimulieren von Zellen gefolgt von dem Anordnen der stimulierten Zellen in einem gewebsspezifischen Ort, zum Induzieren einer Gewebsmorphogenese;
(s) die Verwendung in einem Verfahren, umfassend die Schritte, Vorläuferzellen aus Knochenmark zu isolieren, diese Zellen ex vivo zum Induzieren einer Proliferation zu stimulieren und diese Zellen zu dem Knochenmark zu übertragen;
(t) die Verwendung in einem Verfahren, umfassend die Schritte, biokompatible Zellen aus einer Kulturzell-Linie zu isolieren, Zellen ex vivo zum Induzieren einer Proliferation zu stimulieren und diese Zellen zu implantieren;
(u) das Induzieren einer mitogenen Aktivität einer Vorläuferzell-Population in vivo (beispielsweise unter Verwendung eines Morphogens, das zur systemischen Verabreichung, Implantation oder Injektion geeignet ist);
(v) die Vermehrung einer ausgewählten Population von hämatopoetischen Stammzellen (beispielsweise durch Extrahieren von Zellen aus dem Blut durch Perfusion und Stimulieren der Zellen ex vivo zur Gewinnung stimulierter Zellen, die dazu geeignet sind, wieder an das Blut zurückgegeben zu werden) ;
(w) die Vermehrung einer sich erneuernden Vorläuferzell-Population in einem Säugetier;
(x) das Induzieren einer nicht-Skelett-Gewebsmorphogenese in einem Säugetier;
(y) die Verwendung in einem Verfahren, umfassend die Schritte, auf der azellulären Matrix angeordnete Vorläuferzellen einem nicht-Skelett-Gewebs-Ort in vivo zu verabreichen, um dadurch eine nicht-Skelett-gewebsspezifische Differenzierung und Proliferation innerhalb des Ortes zu induzieren;
(z) das Induzieren eines nicht-Skelett-Ersatzgewebes in einem nicht-Skelett-Gewebs-Ort in einem Säugetier;
(aa) die Aufrechterhaltung oder Wiederherstellung der phänotypischen Expression von differenzierten Säugetierzellen (beispielsweise Leberzellen), die beispielsweise alt oder im Ruhezustand sind (oder einem Risiko ausgesetzt sind, dieses zu werden) ;
(ab) das Induzieren der Proliferation von Säugetier-Vorläuferzellen (beispielsweise ex vivo), unter dem Vorbehalt, daß die Zellen in (b), (c), (f), (r), (s), (t) , (u) , (w) und (ab) Zellen aus nicht-Skelett-Gewebe sind.

11. Zusammensetzung oder Verwendung nach einem der vorhergehenden Ansprüche, bei der das Morphogen ein dimeres Morphogen umfaßt, das eine gewebsspezifische Morphogenese bei dem Säugetier induziert und das zwei gefaltete Polypeptide umfaßt, wobei deren Aminosäuresequenz jeweils eine Sequenz umfaßt, die zumindest 70%, beispielsweise zumindest 80% Homologie mit den Resten 38 bis 139 von hOP1 (Sequenz ID Nr. 5) teilt.

12. Zusammensetzung oder Verwendung nach Anspruch 11, bei der die Aminosäuresequenz mehr als 60%, beispielsweise mehr als 65%, Aminosäureidentität mit den Resten 43 bis 139 von hOP1 (Sequenz ID Nr. 5) aufweist.

13. Zusammensetzung oder Verwendung nach Anspruch 12, bei der die Aminosäuresequenz aus der Gruppe ausgewählt ist, die aus den Resten 38 bis 139 von hOP1 (Sequenz ID Nr. 5); mOP1 (Sequenz ID Nr. 6); hOP2 (Sequenz ID Nr. 7) oder mOP2 (Sequenz ID Nr. 8) ausgewählt ist oder eine natürlich vorkommende oder biosynthetische Variante einer Sequenz ist, die aus dieser Gruppe ausgewählt ist.

14. Zusammensetzung oder Verwendung nach einem der Ansprüche 11 - 13, wobei die Aminosäuresequenz aus der Gruppe ausgewählt ist, die aus den Sequenz ID Nr. 9, 10, 11, 12, 13 und 14 ausgewählt ist oder eine natürlich vorkommende oder biosynthetische Variante dieser Sequenz ist.

15. Zusammensetzung oder Verwendung nach einem der Ansprüche 11 - 14, wobei die Sequenz der Morphogen-Polypeptide jeweils weiterhin eine Aminosäuresequenz umfassen, die aus den Resten 1 bis 37 der Gruppe ausgewählt ist, die aus den Sequenz ID Nr. 5, 6, 7 und 8, oder eine natürlich vorkommenden oder biosynthetischen Variante hiervon bestehen, derartig, daß die Sequenzen eine Sequenz umfassen, die aus den Sequenzen von reifem hOP1, mOP1, hOP2 oder natürlich vorkommenden oder biosynthetischen Varianten hiervon ausgewählt sind.

16. Verfahren zum Nachweis einer nicht-Skelett-Gewebs-Dysfunktion durch Beobachtung der Spiegel endogener Morphogene (beispielsweise zum Nachweis von Veränderungen in der Gewebsoder Organstasis).

17. Ex vivo-Verfahren der:
(a) Vermehrung einer Population von Säugetier-Vorläuferzellen; oder
(b) Aufrechterhaltung der phänotypischen Expression von differenzierten Säugetierzellen (beispielsweise Zellen, die einem Risiko ausgesetzt sind, alt oder ruhend zu werden);
Umfassend den Schritt, die Zellen ex vivo mit einem Morphogen in einer Konzentration und für eine ausreichende Zeit lang in Berührung zu bringen, derartig, daß die Vorläuferzellen zur Poliferation oder zum Exprimieren (oder wieder exprimieren) ihres Phänotyps stimuliert werden, wobei die Zellen Zellen aus nicht-Skelett-Gewebe sind.

## Revendications

1. Composition comprenant: (1) une matrice acellulaire biocompatible qui fournit un substrat d'ancrage pour les cellules progénitrices de mammifère et est essentiellement libre de substances qui inhibent la morphogénèse; et (b) un morphogène adsorbé sur cette matrice à une concentration suffisante pour induire la cascade du développement de la morphogénèse spécifique d'un tissu lorsqu'on introduit cette composition in vivo dans un locus d'un tissu non squelettique, cette matrice possédant les composants spécifiques pour ce locus de tissu.

2. La composition selon la revendication 1, dans laquelle cette matrice est biodégradable et/ou poreuse (p.ex. en étant dérivée d'un tissu spécifique d'un organe ou comprenant des facteurs de fixation aux cellules ou au collagène choisis parmi les glycosaminoglycanes et les protéoglycanes).

3. Composition comprenant un morphogène en association avec une matrice issue d'un tissu non squelettique déshydraté.

4. La composition selon la revendication 1, 2 ou 3 dans laquelle cette matrice est issue d'un tissu du foie, où d'une dentine déminéralisée et où l'on extrait de la guanidine, p.ex. pour une utilisation en dentinogénèse, régénération du foie, régénération de la dentine ou réparation de tissus endommagés de la dentine ou du foie (p.ex. provenant d'une maladie ou d'une lésion mécanique).

5. La composition selon l'une quelconque des revendications 1 à 4 pour:
(a) accroître le renouvellement d'une population de cellules progénitrices chez un mammifère;
(b) induire la morphogénèse d'un tissu non squelettique chez un mammifère;
(c) utiliser dans une méthode comprenant les étapes de fournir des cellules progénitrices disposées sur cette matrice acellulaire sur un locus in vivo d'un tissu non squelettique pour induire par conséquent une prolifération et différenciation spécifique d'un tissu non squelettique à l'intérieur de ce locus;
(e) conserver ou restaurer l'expression phénotypique des cellules différenciées de mammifères (p.ex. les cellules qui sont devenues (ou risquent de devenir) sénescentes ou quiescentes);
(f) induire la prolifération de cellules progénitrices de mammifères (p.ex. ex vivo).

6. Utilisation d'un morphogène (ou de la composition d'une quelconque des revendications 1 à 5) pour la fabrication d'un médicament pour favoriser la régénération d'un tissu non squelettique.

7. Utilisation selon la revendication 6 dans laquelle le tissu est un tissu autre qu'un os et un cartilage, p.ex.:
(a) un tissu d'un poumon endommagé par un emphysème pulmonaire;
(b) un tissu de foie ou de rein endommagé par une cirrhose;
(c) un tissu endommagé d'un vaisseau sanguin et/ou d'un vaisseau coronaire (p.ex. provenant d'attaques cardiomyopatiques, athérothrombotiques ou cardioemboliques);
(d) un tissu endommagé d'un estomac provenant d'un ulcère;
(e) un tissu neuronal endommagé (p.ex. provenant d'un lésion physique ou de maladie dégénérative);
(f) un tissu endommagé de la dentine (p.ex. provenant d'une maladie ou d'une lésion mécanique).

8. Utilisation selon la revendication 7 (e) dans laquelle la maladie dégénérative est la maladie d'Alzheimer, une sclérose multiple ou une attaque.

9. Utilisation d'un morphogène pour la fabrication d'un médicament pour:
(a) stimuler in vivo la redifférenciation des cellules (p.ex. pour diminuer ou inhiber essentiellement la tumorigénèse et/ou la croissance des néoplasmes);
(b) la thérapie contre le cancer (p.ex. via l'inhibition de la croissance des néoplasmes);
(c) stimuler la redifférenciation des cellules différentiées in vivo;
(d) induire la dé- ou redifférenciation des néoplasmes.

10. Utilisation d'un morphogène pour la fabrication d'un médicament pour:
(a) empêcher ou inhiber essentiellement la formation d'une lésion tissulaire;
(b) traiter les troubles provenant d'une perte ou d'une réduction d'une population de cellules renouvelables (p.ex. les troubles sanguins et les troubles provenant d'une perte ou d'un affaiblissement des fonctions immunitaires);
(c) traiter les troubles provenant de la sénescence ou de la quiescence des cellules (p.ex. cellules du foie) (p.ex. ostéoporose) via une stimulation de ces cellules in vivo;
(d) utilisation en thérapie génique pour stimuler la croissance des cellules quiescentes et par conséquent augmenter l'incorporation d'ADN exogène;
(e) détecter les dysfonctionnements de tissu en contrôlant le niveau des morphogènes endogènes (p.ex. détecter les changements de stase dans les tissus ou organes);
(f) stimuler la prolifération des cellules différentiées;
(g) améliorer la stase des tissus;
(h) régénérer la dentine ou la dentinogénèse;
(i) former un tissu hépatique;
(j) régénérer un tissu de la peau;
(k) guérir les ulcères gastro-intestinaux;
(I) remplacer les tissus non squelettiques grâce à une chirurgie reconstructive;
(m) traiter les maladies nerveuses dégénératives;
(n) induire l'énervation d'un tissu;
(o) utiliser sous forme d'un neuroprotecteur;
(p) utiliser dans la régénération CNS;
(q) induire la morphogénèse d'un tissu dans un tissu d'un mammifère, ce tissu n'étant pas un os ou un cartilage;
(r) stimuler les cellules ex vivo suivi par l'introduction de ces cellules stimulées à un locus spécifique d'un tissu pour induire la morphogénèse d'un tissu;
(s) utiliser dans une méthode comprenant les étapes d'isolement des cellules progénitrices à partir de la moëlle osseuse, stimuler ces cellules ex vivo pour induire la prolifération et retourner ces cellules vers cette moëlle osseuse;
(t) utiliser dans une méthode comprenant les étapes d'isolement de cellules biocompatibles à partir d'une lignée de culture cellulaire, stimulant ces cellules ex vivo pour induire la prolifération et l'implantation de ces cellules;
(u) induire l'activité mitogène d'une population de cellules progénitrices in vivo (p.ex. en utilisant un morphogène adapté pour une administration systémique, une implantation ou une injection) ;
(v) augmenter une population choisie de cellules souches hémopoiétiques (p.ex. par extraction des cellules à partir du sang par perfusion pour obtenir des cellules stimulées appropriées pour être retournées et stimuler ces cellules ex vivo vers le sang);
(w) augmenter une population de cellules progénitrices à renouveler chez un mammifère;
(x) induire une morphogénèse d'un tissu non squelettique chez un mammifère;
(y) utilisation dans une méthode comprenant les étapes de fournir des cellules progénitrices disposées sur cette matrice acellulaire sur un locus d'un tissu non squelettique in vivo pour induire par conséquent une prolifération et une différenciation spécifique d'un tissu non squelettique à l'intérieur de ce locus;
(a') induire le remplacement d'un tissu non squelettique à un locus d'un tissu non squelettique chez un mammifère;
(aa) conserver ou restaurer l'expression phénotypique des cellules différenciées de mammifères (les cellules du foie), qui sont devenues p.ex. (ou risquent de devenir) sénescentes ou quiescentes);
(ab) induire la prolifération de cellules progénitrices de mammifères (p.ex. ex vivo), à condition que ces cellules dans (b), (c), (f), (r), (s), (t), (u), (w), et (ab) sont des cellules d'un tissu non squelettique.

11. Composition ou utilisation selon l'une quelconque des revendications précédentes dans laquelle le morphogène comprend une protéine dimère qui induit une morphogénèse spécifique d'un tissu chez ce mammifère et comprend une paire de polypeptides pliés, dont la séquence d'acides aminés de chacun comprend une séquence partageant au moins 70%, p.ex. au moins 80%, d'homologie avec des résidus 38-139 de hOP1 (SEQ ID NO. 5).

12. Composition ou utilisation selon la revendication 11 dans laquelle cette séquence d'acides aminés possède une identité d'acides aminés supérieure à 60%, p.ex. supérieure à 65% avec des résidus 43-139 de hOP1 (SEQ ID NO. 5).

13. Composition ou utilisation selon la revendication 12 dans laquelle on choisit cette séquence d'acides aminés à partir du groupe comprenant les résidus 38-139 de hOP1 (SEQ ID NO. 5); mOP1 (SEQ ID NO. 6); hOP2 (SEQ ID NO. 7) ou mOP2 (SEQ ID NO. 8) ou elle est un variant biosynthétique ou naturel d'une séquence choisie dans ce groupe.

14. Composition ou utilisation selon l'une quelconque des revendications 11-13 dans laquelle on choisit cette séquence d'acides aminés à partir du groupe comprenant les séquences ID No. 9, 10, 11, 12, 13 et 14, ou un variant biosynthétique ou naturel de cette séquence.

15. Composition ou utilisation selon l'une quelconque des revendications 11-14 dans laquelle chaque séquence supplémentaire de ces polypeptides de morphogènes comprend une séquence d'acides aminés choisie à partir des résidus 1-37 du groupe comprenant les séquences ID No. 5, 6, 7 et 8, ou un variant biosynthétique ou naturel de celui-ci, telles que ces séquences comprennent une séquence choisie à partir des séquences de variants biosynthétiques ou se produisant naturellement ou de hOP1, mOP1, hOP2 matures de celui-ci.

16. Méthode de détection d'un dysfonctionnement d'un tissu non squelettique par contrôle des niveaux de morphogènes endogènes (p.ex. pour détecter les changements de stase dans les tissus ou les organes).

17. Méthode ex vivo pour:
(a) accroître une population de cellules progénitrices de mammifère; ou
(b) conserver l'expression phénotypique des cellules différenciées de mammifère (p.ex. les cellules risquant de devenir sénescentes ou quiescentes);
comprenant l'étape de mettre ces cellules ex vivo en contact avec un morphogène à une concentration et une période suffisante pour que ces cellules progénitrices soient stimulées pour proliférer et pour exprimer (ou ré-exprimer) leur phénotype, respectivement, où ces cellules sont des cellules d'un tissu non squelettique.
